# EUROPEAN PATENT SPECIFICATION

(11) **EP 1 147 413 B1**
(45) Date of publication and mention of the grant of the patent: **05.04.2006**
(21) Application number: 00903387.9
(22) Date of filing: 21.01.2000
(51) Int. Cl.: G01N 33/50, G01N 33/68, C07K 14/51, C07K 14/475, C07K 7/08, C07K 7/06, A01K 67/027, C12N 9/00, C12N 15/11

(54) **GROWTH DIFFERENTIATION FACTOR INHIBITORS AND USES THEREFOR**
INHIBITOREN FÜR WACHSTUMDIFFERENZIERUNGSFAKTOR UND IHRE ANWENDUNGEN
INHIBITEURS DE FACTEURS DE DIFFERENCIATION DE LA CROISSANCE ET LEURS UTILISATIONS

(30) Priority: 21.01.1999 US 116639 P; 10.06.1999 US 138363 P
(43) Date of publication of application: 24.10.2001
(73) Proprietor: Metamorphix, Inc., Baltimore, MD 21227 (US)
(72) Inventor: TOPOUZIS, Stavros, Seattle, WA 98119 (US); WRIGHT, Jill, F., Forest Hill, MD 21050 (US); RATOVITSKI, Tamara, Columbia, MD 21044 (US); LIANG, Li-Fang, Elkridge, MD 21075 (US); BRADY, James, L., Jr., Bethesda, MD 20814 (US); SINHA, Debasish, Gaithersburg, MD 20877 (US); YASWEN-CORKERY, Linda, Silver Spring, MD 20902 (US)
(74) Representative: Kirkham, Nicholas Andrew
(86) International application number: PCT/US2000/001552
(87) International publication number: WO 2000/043781

(56) References cited:
- EP-A- 1 100 887
- WO-A-94/21681
- WO-A-96/01845
- WO-A-96/28555
- WO-A-97/11162
- WO-A-97/17433
- WO-A-98/24925
- WO-A-98/33887
- WO-A-98/35019
- WO-A-98/48809
- WO-A-99/02667
- WO-A-99/06559
- WO-A-99/42573
- US-A- 5 574 143
- US-A- 5 616 466
- MCPHERRON, A.C. ET AL.: "Double muscling in cattle due to mutations in the myostatin gene" PROC. NATL. ACAD. SCI. USA, vol. 94, November 1997 (1997-11), pages 12457-12461, XP002085801

### SEQUENCE LISTING

<110> METAMORPHIX, INC.
<120> GROWTH DIFFERENTIATION FACTOR INHIBITORS AND USES THEREFOR
<130> MTN-024PC
<140>
   <141>
<150> 60/116,639
   <151> 1999-01-21
<150> 60/138,363
   <151> 1999-06-10
<160> 30
<170> PatentIn Ver. 2.0
<210> 1
   <211> 42
   <212> DNA
   <213> Artificial Sequence
<220>
   <223> Description of Artificial Sequence: Synthetic Sequence
<400> 1
   aatataaaca ctgatgagtc cgtgaggacg aaacatttgc ag 42
<210> 2
   <211> 42
   <212> DNA
   <213> Artificial Sequence
<220>
   <223> Description of Artificial Sequence: Synthetic Sequence
<400> 2
   gcacatgcat ctgatgagtc cgtgaggacg aaacacagcc cc 42
<210> 3
   <211> 42
   <212> DNA
   <213> Artificial Sequence
<220>
   <223> Description of Artificial Sequence: Synthetic Sequence
<400> 3
   gttgggcttt ctgatgagtc cgtgaggacg aaactacttt gt 42
<210> 4
   <211> 42
   <212> DNA
   <213> Artificial Sequence
<220>
   <223> Description of Artificial Sequence: Synthetic Sequence
<400> 4
   ttcacatcaa ctgatgagtc cgtgaggacg aaactctgcc aa 42
<210> 5
   <211> 20
   <212> DNA
   <213> Artificial Sequence
<220>
   <223> Description of Artificial Sequence: Synthetic Sequence
<400> 5
   acgtttttga cgttgagaca 20
<210> 6
   <211> 20
   <212> DNA
   <213> Artificial Sequence
<220>
   <223> Description of Artificial Sequence: Synthetic Sequence
<400> 6
   aaatataaat ggacaaatac 20
<210> 7
   <211> 20
   <212> DNA
   <213> Artificial Sequence
<220>
   <223> Description of Artificial Sequence: Synthetic Sequence
<400> 7
   ctcttgtcac tcgtttttct 20
<210> 8
   <211> 20
   <212> DNA
   <213> Artificial Sequence
<220>
   <223> Description of Artificial Sequence: Synthetic Sequence
<400> 8
   tacatgaacc tctgttttgt 20
<210> 9
   <211> 20
   <212> DNA
   <213> Artificial Sequence
<220>
   <223> Description of Artificial Sequence: Synthetic Sequence
<400> 9
   atgtttagga gtcatttgaa 20
<210> 10
   <211> 20
   <212> DNA
   <213> Artificial Sequence
<220>
   <223> Description of Artificial Sequence: Synthetic Sequence
<400> 10
   tctacaatat tctgttaaaa 20
<210> 11
   <211> 20
   <212> DNA
   <213> Artificial Sequence
<220>
   <223> Description of Artificial Sequence: Synthetic Sequence
<400> 11
   actagtcata ctacaggtct 20
<210> 12
   <211> 20
   <212> DNA
   <213> Artificial Sequence
<220>
   <223> Description of Artificial Sequence: Synthetic Sequence
<400> 12
   tactgctaat agtgcgatgt   20
<210> 13
   <211> 20
   <212> DNA
   <213> Artificial Sequence
<220>
   <223> Description of Artificial Sequence: Synthetic Sequence
<400> 13
   agactaaaag attacgttca 20
<210> 14
   <211> 20
   <212> DNA
   <213> Artificial Sequence
<220>
   <223> Description of Artificial Sequence: Synthetic Sequence
<400> 14
   atataaactc tgggcagctc 20
<210> 15
   <211> 20
   <212> DNA
   <213> Artificial Sequence
<220>
   <223> Description of Artificial Sequence: Synthetic Sequence
<400> 15
   tgaggatgtt gtcacaaaca 20
<210> 16
   <211> 20
   <212> DNA
   <213> Artificial Sequence
<220>
   <223> Description of Artificial Sequence: Synthetic Sequence
<400> 16
   tctgagtagt ttggatactt 20
<210> 17
   <211> 20
   <212> DNA
   <213> Artificial Sequence
<220>
   <223> Description of Artificial Sequence: Synthetic Sequence
<400> 17
   tctgccatgt tccatatgac 20
<210> 18
   <211> 19
   <212> DNA
   <213> Artificial Sequence
<220>
   <223> Description of Artificial Sequence: Synthetic Sequence
<400> 18
   agactttgaa ctgtacttg 19
<210> 19
   <211> 21
   <212> DNA
   <213> Artificial Sequence
<220>
   <223> Description of Artificial Sequence: Synthetic Sequence
<400> 19
   ggtccgtgac cataaaccgt c 21
<210> 20
   <211> 20
   <212> DNA
   <213> Artificial Sequence
<220>
   <223> Description of Artificial Sequence: Synthetic Sequence
<400> 20
   acttctgtca caacgtttta 20
<210> 21
   <211> 20
   <212> DNA
   <213> Artificial Sequence
<220>
   <223> Description of Artificial Sequence: Synthetic Sequence
<400> 21
   accgagtttg ttggacttag 20
<210> 22
   <211> 20
   <212> DNA
   <213> Artificial Sequence
<220>
   <223> Description of Artificial Sequence: Synthetic Sequence
<400> 22
   taactttatt ttcgaaatct 20
<210> 23
   <211> 20
   <212> DNA
   <213> Artificial Sequence
<220>
   <223> Description of Artificial Sequence: Synthetic Sequence
<400> 23
   actcttacca gtactagaac                                                    20
<210> 24
   <211> 20
   <212> DNA
   <213> Artificial Sequence
<220>
   <223> Description of Artificial Sequence: Synthetic Sequence
<400> 24
   gggtcctggt cctcttctac                                                    20
<210> 25
   <211> 23
   <212> PRT
   <213> Artificial Sequence
<220>
   <223> Description of Artificial Sequence: Synthetic Sequence
<400> 25
   Ala Asn Tyr Cys Ser Gly Glu Cys Glu Phe Val Phe Leu Gln Lys Tyr
     1               5                  10                  15
   Pro His Thr His Leu Val His
               20
<210> 26
   <211> 5
   <212> PRT
   <213> Artificial Sequence
<220>
   <223> Description of Artificial Sequence: Synthetic Sequence
<400> 26
   Asn Glu Asn Ser Glu
     1               5
<210> 27
   <211> 5
   <212> PRT
   <213> Artificial Sequence
<220>
   <223> Description of Artificial Sequence: Synthetic Sequence
<400> 27
   Asp Asp Ser Ser Asp
   1 5
<210> 28
   <211> 7
   <212> PRT
   <213> Artificial Sequence
<220>
   <223> Description of Artificial Sequence: Synthetic Sequence
<400> 28
   Gly Pro Val Asp Leu Asn Glu
   1 5
<210> 29
   <211> 13
   <212> PRT
   <213> Artificial Sequence
<220>
   <223> Description of Artificial Sequence: Synthetic Sequence
<400> 29
   Lys Ile Pro Ala Met Val Val Asp Arg Cys Gly Cys Ser
   1 5 10
<210> 30
   <211> 22
   <212> PRT
   <213> Artificial Sequence
<220>
   <223> Description of Artificial Sequence: Synthetic Sequence
<400> 30
   Leu Ser Lys Leu Arg Leu Glu Thr Ala Pro Asn Ile Ser Lys Asp Val
   1 5 10 15
   Ile Arg Gln Leu Leu Pro
   20

## Description

### Field of the Invention

The invention relates to inhibitors of GDF proteins, such as GDF-8 and GDF-11 proteins as well as methods of identifying these inhibitors and methods of using them.

### Background of the Invention

Growth and Differentiation Factor-8 (GDF-8), also known as Myostatin, is a member of the Transforming Growth Factor-beta (TGF-β) superfamily of structurally-related growth factors, all of which are endowed with physiologically important growth-regulatory and morphogenetic properties (D.M. Kingsley et al. (1994) *Genes Dev.,* 8, 133-46; P.A. Hoodless et al. (1998) *Curr. Topics Microbiol. Immunol.,* 228, 235-72). Members of the TGF-β superfamily signal through a heteromeric protein kinase receptor complex. The family also includes Bone Morphogenetic Proteins (BMPs), Activins, Inhibins, Mullerian Inhibiting Substance, Glial-Derived Neurotrophic Factor, and a still growing number of Growth and Differentiation Factors (GDFs). Myostatin itself is highly expressed specifically in the developing and adult skeletal muscle and to a much lesser extent in fat. Myostatin seems to be implicated in a number of physiological processes, the best established of which is its ability to regulate skeletal muscle mass, hence its name "Myostatin".

Myostatin "knock-out" mice generated by gene targeting develop normally, but have twice the normal skeletal muscle mass (A.C. McPherron et al. (1997) *Nature,* 387, 83-90). The identification of two breeds of double-muscled cattle, Belgian Blue and Piedmontese, and of the hypermuscular *Compt* mutant mouse, whose phenotypes are due to mutations of the Myostatin gene, suggests that Myostatin has the same developmental role in these two species (A.C. McPherron et al. (1997) *PNAS*, 94, 12457-61; R. Kambaduret al. (1997) *Genome Res.,* 7, 910-15; G. Szaboet al. (1998) *Mammalian Genome,* 9, 671-2). Sequence data from various species indicate the GDF-8 is highly conserved among vertebrates, suggesting that GDF-8 may have the same role across species (McPherron and Lee, (1997) PNAS, 94, 12457-61).

In agreement with the above, recent studies have shown that HIV-infection-associated muscle wasting in humans is accompanied by increases in Myostatin protein expression (N.F. Gonzalez-Cadavid et al. (1998) *PNAS,* 95, 14938-43). Overall, this evidence strongly supports a pivotal role of GDF-8 (or Myostatin) in the control of skeletal muscle mass.

### Summary of the Invention

The present invention is based, at least in part, on the identification of GDF-8 inhibitory activity in media obtained from cells expressing GDF-8. Accordingly, in one aspect, the invention features a method for identifying a GDF inhibitor, e.g., a GDF-8 inhibitor, such as a GDF polypeptide, which inhibits GDF, e.g., GDF-8, activity. In one embodiment, the inhibitor does not itself possess GDF, e.g., GDF-8 activity (e.g., the inhibitor is a GDF polypeptide which does not itself possess GDF activity). The method includes obtaining medium in which cells producing a GDF polypeptide have been cultured and testing the components of the medium for the ability to inhibit GDF activity, thereby identifying a GDF inhibitor. In one embodiment, the method includes performing chromatography on the medium before the medium is tested for the ability to inhibit GDF activity. In another embodiment, the method includes performing electrophoresis, e.g., preparative non-reducing or reducing SDS-PAGE, on fractions obtained from the chromatography and recovering the fractions, e.g., by electroelution.

In other preferred embodiments, the cells expressing a GDF polypeptide are cells, e.g., CHO cells, which are transfected with a plasmid containing an insert encoding a GDF polypeptide. In still other preferred embodiments, the cells produce a GDF polypeptide, e.g., GDF-8, endogenously. Such cells include, for example, the rhabdomyosarcoma line RD (ATCC, CCL-136) and QM7 muscle myoblast (ATCC, CRL-1962).

In another aspect, the invention features a method for identifying a GDF inhibitor, which includes preparing polypeptide fragments of a GDF polypeptide and testing the fragments for the ability to inhibit GDF activity, thereby identifying a GDF inhibitor. The polypeptide fragments can be prepared by digesting a GDF polypeptide, e.g., a native GDF polypeptide or a recombinantly produced GDF polypeptide, or they can be recombinantly or synthetically synthesized. For example, the GDF polypeptide can be digested using a protease, including but not limited to trypsin, thermolysin, chymotrypsin, pepsin, or any other known protease. The polypeptides can then be isolated before they are tested for the ability to inhibit GDF activity.

Prior to preparing the peptide fragments, the peptides also can be selected to elicit neutralization of endogenous GDF via an immune response. The testing of the polypeptide fragments can be performed by screening the polypeptide fragments for the ability to elicit an immune response resulting in the generation of GDF inhibitory antibodies.

GDF polypeptide inhibitors can be any length sufficient to inhibit GDF activity. Typically, the polypeptides range from 5-10, 10-25, 25-40 or 40 or more amino acids in length. In certain preferred embodiments, the GDF polypeptide fragments are at least 5, 10, 15, 20, 25, 30, 35, 40, or 45 amino acids in length.

In another aspect, the invention features a GDF inhibitor, e.g., an inhibitor which may or may not possess GDF activity, which has one or more of the following characteristics: it can be isolated from medium in which cells (e.g., CHO) stably transfected with an expression plasmid containing an insert encoding GDF-8 or GDF-11 have been isolated by column chromatography; it retains activity after heating at 100°C for up to 10 minutes; it retains or does not retain activity after reduction; and it retains activity after treatment with 6M Urea. In one embodiment, the invention features a GDF-8 inhibitor having a molecular weight of less than 70 kDa in a reducing and denaturing gel.

In another aspect, the invention features a GDF inhibitor, e.g., a GDF polypeptide, identified by the methods described herein.

In another aspect, the invention features a GDF inhibitor consisting of the pro-domain of a GDF polypeptide, or a portion thereof. In preferred embodiments, the pro-domain of a GDF polypeptide or a portion thereof is glycosylated.

In another aspect, the invention features a GDF inhibitor comprising a variant of a GDF polypeptide.

In another embodiment, the GDF polypeptide variant is a pro-domain variant. In yet another embodiment, the GDF polypeptide variant is a post-translational modification variant.

In another aspect, the invention features a GDF inhibitor comprising an isolated nucleic acid molecule which binds to and/or cleaves the RNA transcripts produced by genes encoding a GDF polypeptide. In certain embodiments, the nucleic acid is a ribozymc comprising the nucleotide sequence of any one of SEQ ID NOs: 1-4. In other embodiments, the nucleic acid is an antisense molecule comprising the nucleotide sequence of any one of SEQ ID NOs: 5-24.

In another aspect, the invention features an assay for measuring GDF activity, which can be used to identify GDF inhibitors. Suitable bioassays for testing inhibition og GDF activity include but are not limited to assays which detect the activity of muscle-specific enzymes, such as creatine kinase; assays which detect adipocyte differentiation, such as differentiation of 3T3-L1 pre-adipocytes; DNA replication assays; and transcription-based assays.

In yet another aspect, the invention features a method for testing GDF inhibitors in a cell system, using a protein secretion-based assay.

In a further aspect, the invention features transgenic animals in which expression of genes that encode the GDF inhibitors of the invention interfere with GDF polypeptide processing, GDF polypeptide secretion, and/or GDF polypeptide biological activity.

In other aspects, the invention features methods of using GDF inhibitors of the invention, for example, to treat a variety of diseases.

Other features and advantages of the invention will be apparent from the following detailed description, and from the claims.

### Brief Description of the Drawings

*Figure 1* shows an ion exchange chromatogram (HQ column) for fractions A and B which was measured at 230 nm.
*Figure 2* shows an ion exchange chromatogram (SP column) of fraction A measured at 215 run. The inset shows the gradient used in the elution where buffer A was 20 mM NaPhos pH 5.0 and buffer B was 20 mM NaPhos pH 5.0/2 M NaCl.
*Figure 3* shows an ion exchange chromatogram (SP column) of fraction B measured at 215 nm. The inset shows the gradient used in the elution where buffer A was 20 mM NaPhos pH 5.0 and buffer B was 20 mM NaPhos pH 5.0/2 M NaCl.
*Figure 4* shows a chromatogram of the reverse phase chromatography (C4 column) for fraction A which elutes between 21-23 minutes. The gradient used for the elution is also shown where buffer A was 0.1 % TFA and buffer B was 0.1 % TFA in 80% acetonitrile.
*Figure 5* shows a chromatogram of the reverse phase chromatography (C4 column) for fraction B which elutes at 27-29 minutes. The gradient used for the elution is also shown where buffer A was 0.1 % TFA and buffer B was 0.085% TFA in 80% acetonitrile.
*Figures 6A and B* are graphs showing that fraction A inhibits the effect of GDF-8 on cell proliferation. *Figure 6A* shows the effect of fraction A on DNA synthesis in G8 myoblasts as measured by BrdU incorporation. *Figure 6B* shows the effect of fraction A on DNA synthesis in CCL-64 mink lung epithelial cells as measured by [³H]-TdR incorporation.
*Figures 7A and B* are graphs showing the GDF-8-inhibitory activity of Fraction A monitored by transcription-based assays. Luciferase expression is derived from the reporter plasmid p(CAGA)₁₂-MLP (*Figures* 7A and 7B).
*Figure 8* is a graph showing the specificity of the inhibitory effect of Fraction A for GDF-8.
*Figure 9* is a graph showing the GDF-8-inhibitory activity of Fraction B monitored by transcription-based assays.
*Figure 10* is a graph showing the specificity of the inhibitory effect of Fraction B for GDF-8.
*Figure 11* is a depiction of an amino acid sequence alignment of murine, rat, human, baboon, bovine, porcine, ovine, chicken, turkey, and zebrafish GDF-8.
*Figure 12* is a schematic representation of various GDF-8 constructs. *Figure 12A* shows the construct for the wild-type, full-length GDF-8. GDF-8 is processed in cells generating the mature GDF-8 and the remainder pro-peptide. *Figure 12B* shows the uncleavable mutant with the replaced cleavage site. This mutant is secreted as a precursor molecule. *Figure 12C* shows the pro-domain of GDF-8, expressed independently.
*Figure 13* shows the nucleotide and amino acid sequence of mouse GDF-8. The mutations introduced to generate GDF-8 variants are indicated. The cleavage site is boxed. The beginning and the end of the pro-domain after the disposal of the signal sequence are marked by triangles. The predicted site ofN-linked glycosylation is underlined.
*Figure 14* is graph showing that the pro-region of GDF-8 can inhibit the activity of mature GDF-8.
*Figure 15* is a graph, showing the specificity of the inhibitory effect of pro-domain of GDF-8 for GDF-8.
*Figure 16* is a graph, showing dose-dependent inhibition of GDF-8 activity by the pro-domain.
*Figure 17* shows an alignment of the predicted amino acid sequence of human GDF-11 (top lines) and human GDF-8 (bottom lines). Vertical lines indicate identities. Dots represent gaps introduced in order to maximize the alignment. Numbers represent amino acid positions relative to the N-terminus. The putative proteolytic processing sites are shown by the open box. The conserved cysteine residues on the C-terminal region are shown by the shaded boxes.

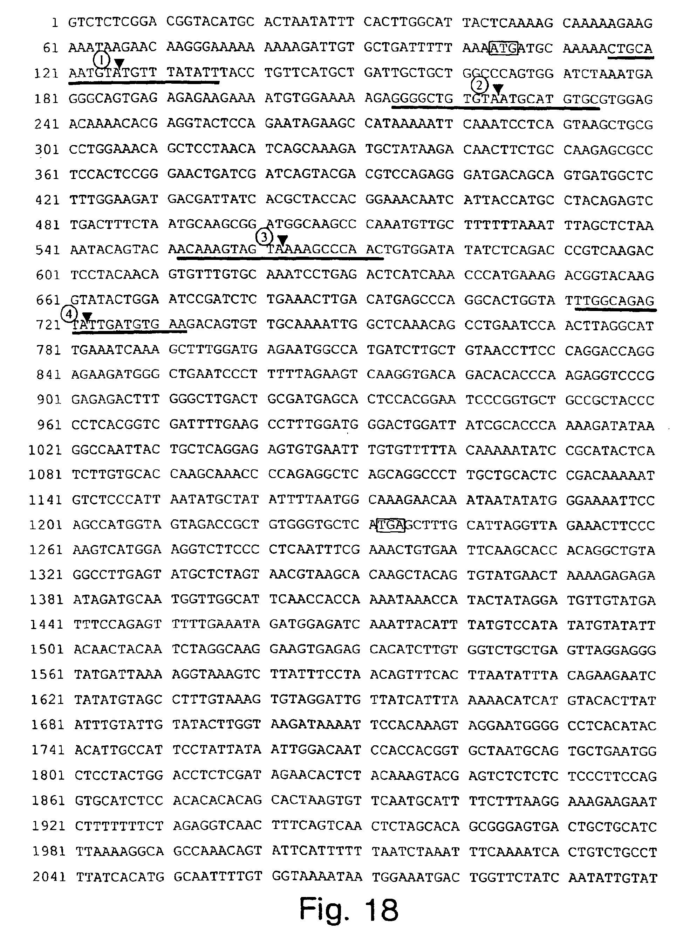

*Figure 18* shows ribozyme target sites in the mouse GDF-8 mRNA sequence. Inverted arrows indicate the four ribozyme cleavage sites that were chosen in the mouse GDF-8 mRNA sequence. Underlined nucleotides indicate regions that are complementary to sequences that flank the catalytic domain in each ribozyme. Translation initiation and termination codons for the GDF-8 protein are enclosed in boxes.

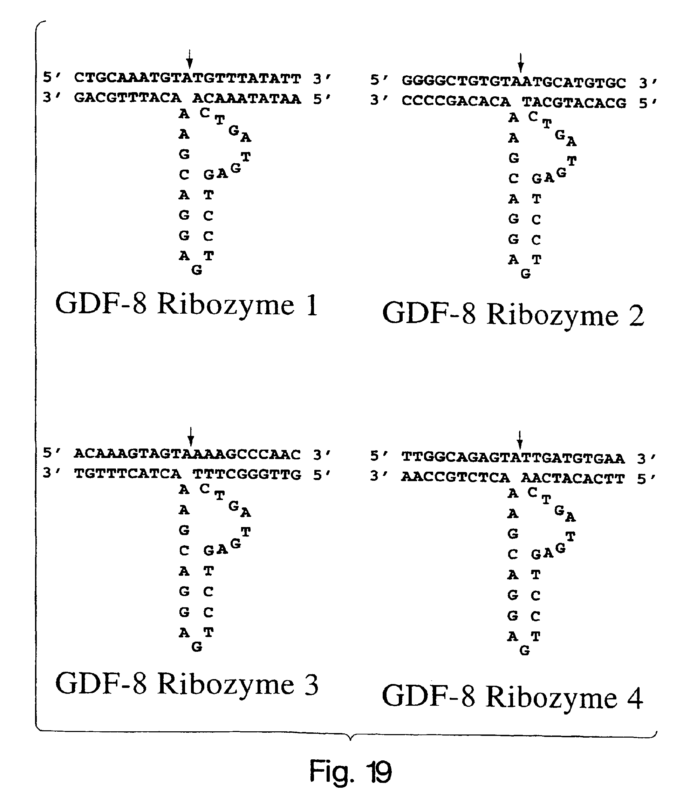

*Figure 19* shows the nucleotide sequences of four mouse GDF-8 ribozymes. The sequence of each ribozyme is shown underneath the mouse GDF-8 mRNA sequence to which it is complementary. Inverted arrows indicate target sites for ribozyme cleavage in the GDF-8 sequence.

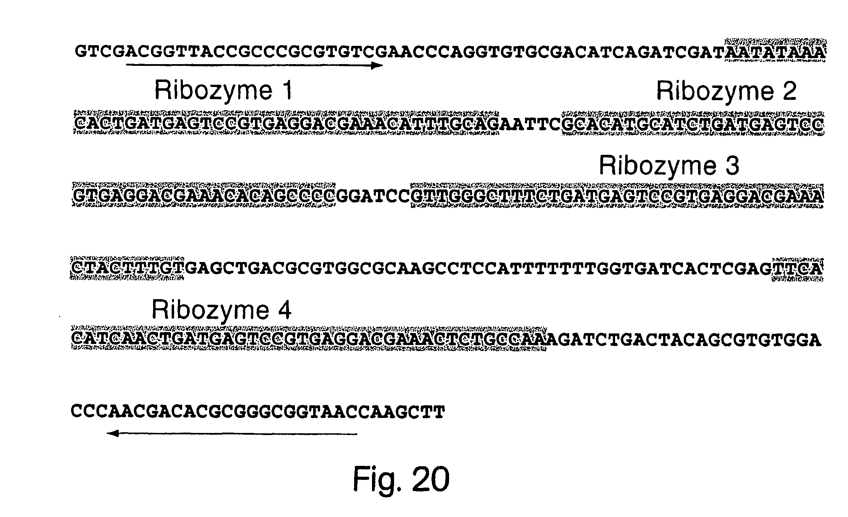

*Figure 20* shows the nucleotide sequence of the DNA cassette containing the four tandemly arrayed ribozymes shown in *Figure* 19 and the inverted repeats. The sequences of the four ribozymes (SEQ ID NOs:1-4) are highlighted and the inverted repeats are indicated by arrows underneath the sequence.

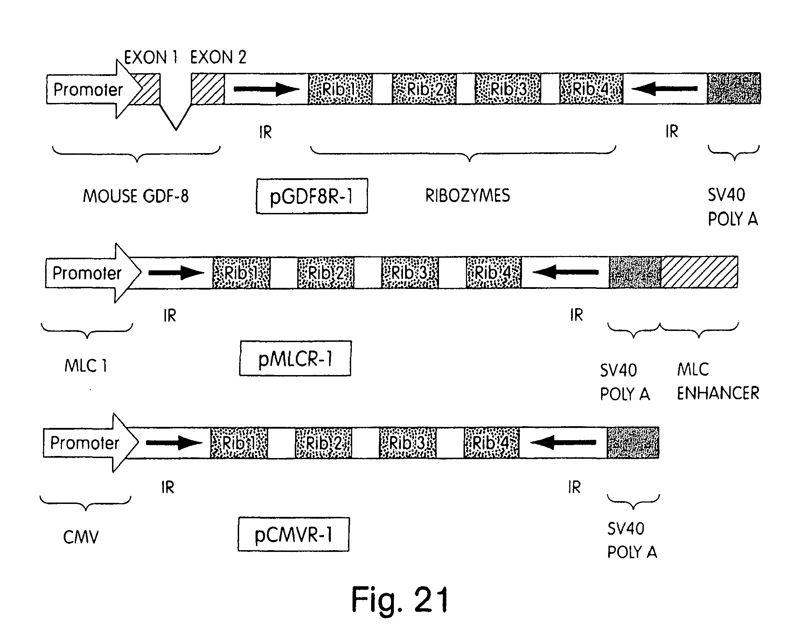

*Figure 21* shows selected ribozyme expression constructs. A ribozyme cassette with inverted repeats was ligated into three different plasmids. pGDF8R-1 contains 2.8 kb upstream of the mouse GDF-8 translation start site, exon1, intron 1, and part of exon 2 from the mouse GDF-8 gene ligated upstream of the ribozyme cassette. The polyadenylation signal from the SV40 large T antigen gene was ligated onto the other side of the ribozyme cassette. pMLCR-1 contains ~ 1500 bp upstream of the transcription start site for the rat myosin light chain 1 gene ligated upstream of the ribozyme cassette and a ~ 900 bp fragment containing the rat myosin light chain gene 3'enhancer ligated downstream of the ribozyme cassette. pCMVR-1 contains approximately 500 bp of the human cytomegalovirus major immediate early gene promoter ligated upstream of the ribozyme cassette. Rib 1, Rib 2, Rib 3, and Rib 4 denote the regions of DNA encoding the four different ribozymes; blocks with inverted arrows correspond to the inverted repeats.

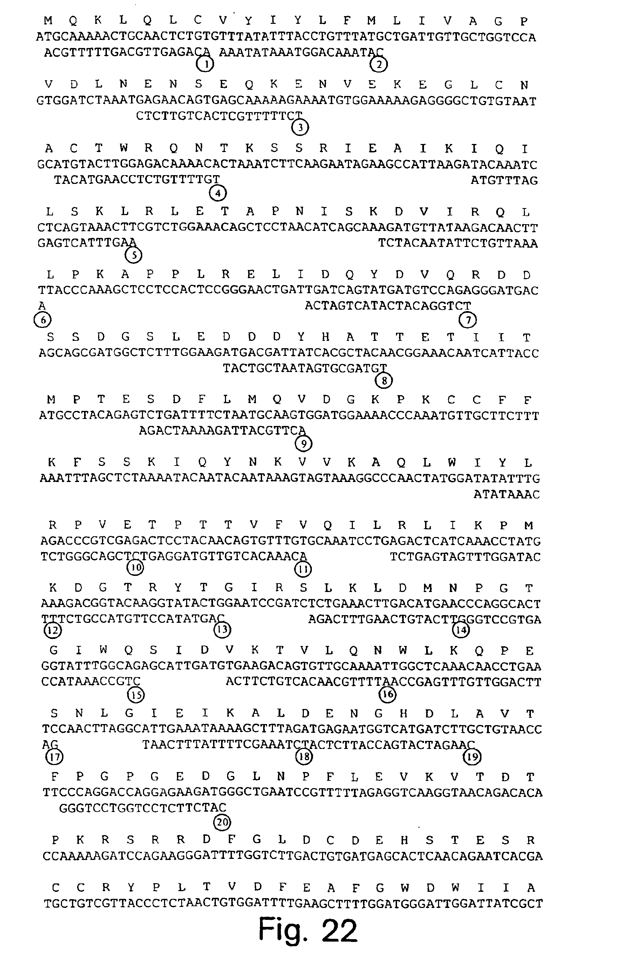

*Figure 22* shows the sequences of twenty oligonucleotides (SEQ ID NOs:5-24) complementary to the human GDF-8 cDNA sequence in the regions shown. Circled numbers beneath the oligonucleotide sequences indicate the 5' ends of the respective oligonucleotides.
*Figure 23* is a graph showing the results from an electrospray/ionization mass spectrometry analysis performed to further characterize Fraction B. The mass spec spectrum showed three peaks separated by 600 Da with the major component of molecular mass of 29472.0 Da.
*Figure 24* is a graph showing the effect of chemical modifications of the pro-domain of GDF-8 on its ability to inhibit the activity of mature GDF-8.
*Figure 25* is a graph showing the effect of the GDF-8 pro-domain purified from Fraction B on GDF-8 induction of a reporter plasmid in A204 cells. *Figure 25* shows the results obtained using reporter plasmid p(CAGA)₁₂-MLP.
*Figure 26* is a graph showing the effect of GDF-8 and TGF-β₁, on myogenic differentiation of C2C12 and chick primary myoblasts, as determined by a creatine kinase-based assay.
*Figure 27* is a graph showing the effects of GDF-8 on glucose uptake in 3T3-L1 adipocytes. The data indicate that GDF-8 inhibits glucose uptake in 3T3-L1 cells in response to insulin
*Figure 28* is a graph showing that *in vitro* deglycosylation of Fraction B results in the loss of its inhibitory activity.
*Figure* 29 is a graph showing the results from a transcription-based reporter activation assay performed to assess the biological activity of the GDF-8 complexes produced by QM-7 myoblasts.
*Figures 30A-B* are graphs showing the results from a transcription-based reporter activation assay performed to assess the biological activity of supernatants from m-calpain treated QM-7 cells (transfected with either the WT-GDF-8-F construct or a control).
*Figure 31* is a graph showing the effect of TGFβ and GDF-8 on the induction of a luciferase reporter plasmid in CCL-64 cells.
*Figure 32* is a graph showing the effect of TGFβ and GDF-8 on the induction of a luciferase reporter plasmid in R1B cells.
*Figure 33* is a graph showing the rescue of R1B cell responsiveness to TGFβ and GDF-8 by the re-introduction of the ALK-5 type I receptor into R1B cells.
*Figure 34* is a graph showing the effect of TGFβ and GDF-8 on the induction of a luciferase reporter plasmid in DR26 cells.
*Figure 35* is a graph showing the effect of the ActRIIB KR expression vector on the responsiveness of DR26 cells to GDF-8.

### Detailed Description

The present invention provides compositions and methods for inhibiting Growth Differentiation Factor, (GDF) proteins, as well as methods for identifying such inhibitors.

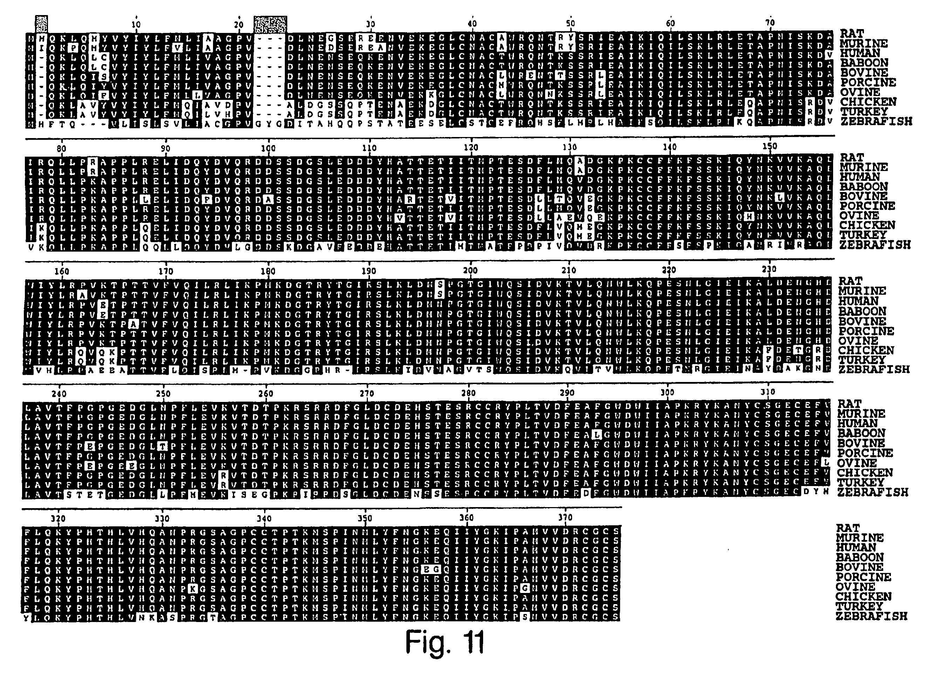

In particular, GDF inhibitors of the present invention can be identified using a variety of screening methods which test peptides from GDF proteins, such as GDF-8 and GDF-11, or medium from cells producing GDF proteins, for GDF inhibitory activity. In one screening method, fragments of GDF proteins (*i.e.*, peptides which comprise any portion of a GDF protein which is less than the whole, full length protein) are prepared and tested for GDF inhibitory activity. In a preferred embodiment, the fragments are derived from the Pro-region of the GDF protein, *e.g.*, from the N-terminus of the pro-region (pro-domain) of the protein. For example, the fragments can be derived from the region of the pro-domain that is upstream of Arg 99 in GDF-8 (see Figure 11). The peptides can be selected by, for example, the ability to elicit antibodies against GDF proteins which inhibit GDF activity. Alternatively, GDF inhibitors can be identified and isolated directly from media of cells producing GDF proteins, as described in detail below. For example, in the studies described herein, two chromatography fractions, Fractions A and B, were isolated from media of CHO cells expressing GDF-8.

As used herein, the terms "GDF polypeptide" and "GDF protein" include members of the Transforming Growth Factor-beta (TGF-β) superfamily of structurally-related growth factors, all of which are endowed with physiologically important growth-regulatory and morphogenetic properties. This family of related growth factors is described in, for example, D.M. Kingsley et al. (1994) *Genes Dev.,* 8, 133-46; P.A. Hoodless et al. (1998) *Curr. Topics Microbiol. Immunol.,* 228, 235-72.

Members of the TGF-β superfamily signal through a heteromeric protein kinase receptor complex.

Accordingly, the terms "GDF polypeptide" and "GDF protein, as used herein, refer to proteins within the TGF-β superfamily, including Bone Morphogenetic Proteins (BMPs), Activins, Inhibins, Mullerian Inhibiting Substance, Glial-Derived Neurotrophic Factor, and a still growing number of Growth and Differentiation Factors (GDFs), including GDF-8 (Myostatin) and GDF-11.

As used herein, the term "GDF inhibitor" includes any agent capable of inhibiting activity, expression, processing, and/or secretion of a GDF protein including but not limited to peptides, peptidomimetics, ribozymes, anti-sense oligonucleotides, or small molecules which specifically inhibit the action of GDF proteins. The GDF inhibitor may possess GDF activity or, preferably is a GDF inhibitor which does not possess GDF activity.

As used herein, the terms "GDF-8 inhibitor" and "GDF-11 inhibitor" include any agent capable of inhibiting GDF-8 or GDF-11 activity, expression, processing, and/or secretion including but not limited to peptides, peptidomimetics, ribozymes, anti-sense oligonucleotides, or small molecules which specifically inhibit the action of GDF-8 or GDF-11 while, preferably, leaving intact the activity of TGF-β or Activin or other members of the TGF-β superfamily. The GDF-8 or GDF-11 inhibitor may possess GDF-8 or GDF-11 activity or, preferably is a GDF-8 or GDF-11 inhibitor which does not possess GDF-8 or GDF-11 activity.

As used herein, the term "GDF-8 or GDF-11 activity" includes any activity mediated by GDF-8 or GDF-11. For example, GDF-8 is known to inhibit fibroblast differentiation to adipocytes, modulate the production of muscle-specific enzymes, e.g., creatine kinase, and modulate uptake glucose by cells, and stimulate myoblast cell proliferation. Accordingly, GDF-8 or GDF-1 1 inhibitors can be identified by, for example, testing GDF-8 or GDF-11 activity, as measured by the ability of GDF-8 or GDF-11 to interfere with the differentiation process of 3T3-L1 pre-adipocytes (fibroblasts) to adipocytes, the ability to modulate the activity of muscle-specific enzymes, e.g., creatine kinase, the ability to modulate glucose uptake by cells, or the ability to stimulate myoblast cell proliferation.

As used herein, the term "bioassay" includes any assay designed to identify a GDF inhibitor. The assay can be an *in vitro* or an *in vivo* assay suitable for identifying whether a GDF inhibitor can inhibit one or more of the biological functions of a GDF protein. Examples of suitable bioassays include DNA replication assays, transcription-based assays, creatine kinase assays, assays based on the differentiation of 3T3-L1 pre-adipocytes, assays based on glucose uptake in 3T3-L1 adipocytes, and immunological assays.

Various aspects of the present invention are described in further detail in the following subsections. To illustrate the invention, these subsections are directed to inhibtors of GDF-8 and GDF-11 (two highly homologous GDF proteins). However, the the invention (e.g., the following description and assays) can be applied to make and use inhibitors for any GDF protein and, therefore, should not be construed as limited to GDF-8 and GDF-11.

### I. PROTEIN INHIBITORS

### A. GDF-8 AND GDF-11 PEPTIDE INHIBITORS

### 1. Identification of GDF-8 or GDF-11 Inhibitors From Media in which Cells Expressing GDF-8 or GDF-11 Have been Cultured

In one embodiment, the invention provides a method which involves obtaining medium in which cells producing GDF-8 or GDF-11 have been cultured; and testing the medium for the ability to inhibit GDF-8 or GDF-11 activity, thereby identifying a GDF-8 or GDF-11 inhibitor.

The medium from which the GDF-8 or GDF-11 inhibitor is identified can contain cells which are transfected with a plasmid containing an insert encoding GDF-8 or GDF-11. Alternatively, the medium from which the GDF-8 or GDF-11 inhibitor is identified can contain cells which produce GDF-8 or GDF-11 endogenously, i.e., native GDF-8 or GDF-11 As used herein, the term "native protein" includes a protein recovered from a source occurring in nature.

The cell producing GDF-8 or GDF-11 can be any prokaryotic or eukaryotic cell. For example, the GDF-8 or GDF-11 protein can be expressed in bacterial cells such as *E. coli,* insect cells, yeast or mammalian cells (such as Chinese hamster ovary cells (CHO) or COS cells). Other suitable host cells are readily known to those skilled in the art.

The plasmid containing an insert encoding GDF-8 or GDF-11 can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride coprecipitation, DEAE-dextran-mediated transfection, lipofection, or electroporation. Suitable methods for transforming or transfecting host cells can be found in Sambrook, et al. *(Molecular Cloning: A Laboratory Manual. 2nd, ed., Cold Spring Harbor Laboratory,* Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding the GDF-8 or GDF-11 protein or can be introduced on a separate vector. Cells stably transfected with the introduced nucleic acid can be identified by drug selection (e.g., cells that have incorporated the selectable marker gene will survive, while the other cells die).

As used herein, the term "GDF-8" includes all known forms of GDF-8 including but not limited to human GDF-8, bovine GDF-8, chicken GDF-8, murine GDF-8, rat GDF-8, porcine GDF-8, ovine GDF-8, turkey GDF-8, baboon GDF-8, and fish GDF-8. These molecules are described in McPherron A. C. et al. (1997) *Proc. Natl. Acad. Sci.* 94:12457-12461. The amino acid sequences for these proteins are shown in *Figure 11.*

As used herein, the term "GDF-11" includes all known forms of GDF-11 including but not limited to human GDF-11, bovine GDF-11, chicken GDF-11, murine GDF-11, rat GDF-11, porcine GDF-11, ovine GDF-11, turkey GDF-11, baboon GDF-11, and fish GDF-11. These molecules are described in, for example, U.S. Patent 5,871,935 and in L.W. Gamer *et al.* (1999) *Developmental Biology,* 208, 222-232.

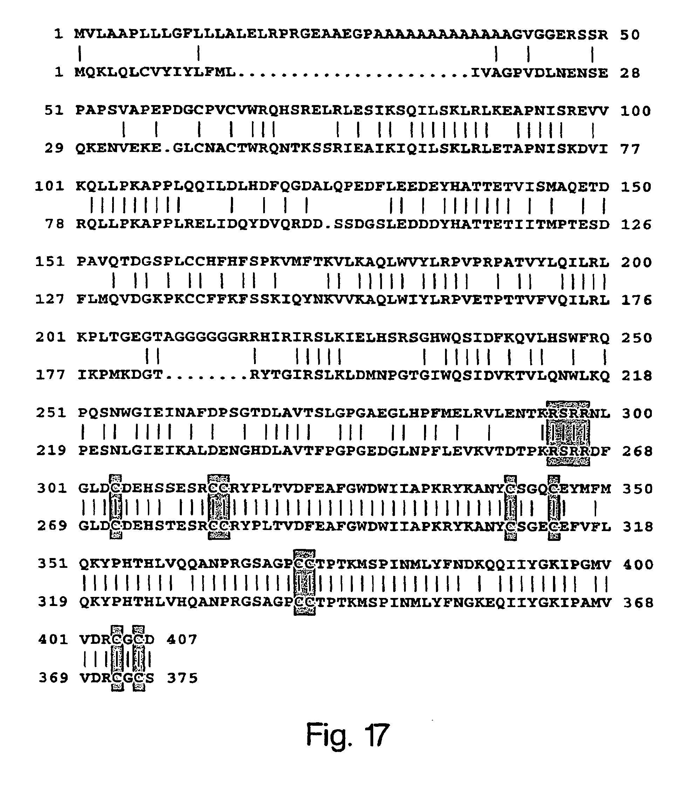

An alignment of the amino acid sequences of the GDF-8 and the GDF-11 proteins is shown in *Figure 17.*

GDF-8 or GDF-11 inhibitors can be identified and isolated from media of cells expressing GDF-8 or GDF-11 using techniques known in the art for purifying peptides or proteins including ion-exchange chromatography, reverse-phase chromatography, gel filtration chromatography, ultrafiltratior, electrophoresis, and immunoaffinity purification with antibodies specific for the GDF-8 or GDF-11 inhibitor, or a portion thereof. In one embodiment, the media obtained from cultures of cells which express GDF-8 or GDF-11 are subjected to high performance liquid chromatography (HPLC) as described in the Examples section.

The samples obtained can then be tested for GDF-8 or GDF-11 inhibitory activity as described below.

### 2. Identification of Peptides From GDF-8 or GDF-11 Which Inhibit GDF-8 or GDF-11 Activity

In another aspect of the invention, GDF-8 or GDF-11 inhibitors are identified by screening fragments of GDF-8 or GDF-11 for inhibitory activity. GDF-8 or GDF-11 fragments can be produced by a variety of art known techniques. For example, specific oligopeptides (approximately 10-25 amino acids-long) spanning the GDF-8 or GDF-1 1 sequence can be synthesized (e.g., chemically or recombinantly) and tested for their ability to inhibit GDF-8 or GDF-11, for example, using the assays described herein. The GDF-8 or GDF-11 peptide fragments can be synthesized using standard techniques such as those described in Bodansky, M. *Principles ofPeptide Synthesis,* Springer Verlag, Berlin (1993) and Grant, G.A (ed.). *Synthetic Peptides: A User's Guide,* W.H. Freeman and Company, New York (1992). Automated peptide synthesizers are commercially available (e.g., Advanced ChemTech Model 396; Milligen/ Biosearch 9600).

Alternatively, GDF-8 or GDF-11 fragments can be produced by digestion of native or recombinantly produced GDF-8 or GDF-11 by, for example, using a protease, e.g., trypsin, thermolysin, chymotrypsin, or pepsin. Computer analysis (using commercially available software, e.g. MacVector, Omega, PCGene, Molecular Simulation, Inc.) can be used to identify proteolytic cleavage sites.

GDF-8 or GDF-11 peptides of the invention are preferably isolated. As used herein, an "isolated" or "purified" protein or biologically active peptide thereof is substantially free of cellular material or other contaminating proteins from the cell or tissue source from which the GDF-8 or GDF-11 protein or peptide is derived, or substantially free from chemical precursors or other chemicals when chemically synthesized. The language "substantially free of cellular material" includes preparations of GDF-8 or GDF-11 protein or peptide thereof in which the protein or peptide thereof is separated from cellular components of the cells from which it is isolated or recombinantly produced. In one embodiment, the language "substantially free of cellular material" includes preparations of GDF-8 or GDF-11 protein or peptide thereof having less than about 30% (by dry weight) of non-GDF-8 or GDF-11 protein or peptide thereof (also referred to herein as a "contaminating protein"), more preferably less than about 20% of non-GDF-8 or GDF-11 protein or peptide thereof, still more preferably less than about 10% of non-GDF-8 or GDF-11 protein or peptide thereof, and most preferably less than about 5% non-GDF-8 or GDF-11 protein or peptide thereof. When the GDF-8 or GDF-11 protein or biologically active portion thereof is recombinantly produced, it is also preferably substantially free of culture medium, i.e., culture medium represents less than about 20%, more preferably less than about 10%, and most preferably less than about 5% of the volume of the protein preparation.

A two-step method can be used to produce and isolate such proteolytically cleaved GDF-8 or GDF-11 peptides. The first step involves enzymatic digestion of the GDF-8 or GDF-11 protein. GDF-8 or GDF-11 can be produced either as a dimer from CHO cell conditioned media, as a monomer in E.coli or yeast, or isolated from cells which naturally produce GDF-8 or GDF-11. Following purification of GDF-8 or GDF-11 monomers or dimers by, for example, HPLC chromatography, their enzymatic digestion is performed as described *infra.* The amino acids cleaved during the digestion depend on the specific protease used in the experiment as is known in the art. For example, if the protease of choice were trypsin, the cleavage sites would be amino acids arginine and lysine. The GDF-8 or GDF-11 protein can be digested using one or more of such proteases.

After the digestion, the second step involves the isolation of peptide fractions generated by the protein digestion. This can be accomplished by, for example, high resolution peptide separation as described *infra.* Once the fractions have been isolated, their GDF-8 or GDF-11 1 inhibitory activity can be tested for by an appropriate bioassay, as described below.

The proteolytic or synthetic GDF-8 or GDF-11 fragments can comprise as many amino acid residues as are necessary to inhibit, e.g., partially or completely, GDF-8 or GDF-11 function, and preferably comprise at least 5, 10, 15, 20,25, 30, 35, 40, 45, 50, 55, 60, 65, 70, 75, 80, 85, 90, 95, 100 or more amino acids in length.

In one embodiment, peptides are selected which do not contain a sufficient number of T cell epitopes to induce T cell mediated immune responses and/or which contain a sufficient number of B cell epitopes to elicit antibodies when administered to a mammal. Preferred GDF-8 or GDF-11 peptide inhibitors do not contain a sufficient number of T cell epitopes to induce T-cell mediated (e.g., cytokine) responses. However, B cell epitopes may be desirable and can be selected for by, for example, testing the peptide's ability to elicit an antibody response, as discussed below.

T cell epitopes within GDF-8 or GDF-11 fragments can be identified using a number of well known techniques. For example, T cell epitopes can be predicted using algorithms (see e.g., Rothbard, J. and Taylor, W.R. (1988) *EMBO J.* 7:93-100; Berzofsky, J.A. (1989) *Philos Trans R. Soc. Lond.* 323:535-544). Preferably, human T cell epitopes within a GDF-8 or GDF-11 protein can be predicted using known HLA class II binding specific amino acid residues. One algorithm for predicting peptides having T cell stimulating activity which has been used with success is reported in Rothbard, *1st Forum in Virology, Annals of the Pasteur Institute,* pp 518-526 (December, 1986), Rothbard and Taylor, (1988) *Embo J.* 7:93-100 and EP 0 304 279. These documents report defining a general T cell pattern (algorithm), its statistical significance and its correlation with known epitopes as well as its successful use in predicting previously unidentified T cell epitopes of various protein antigens and autoantigens. The general pattern for a T cell epitope as reported in the above-mentioned documents appears to contain a linear pattern composed of a charged amino acid residue or glycine followed by two hydrophobic residues. Other algorithms that have been used to predict T cell epitopes of previously undefined proteins include an algorithm reported by Margalit et al., (1987) *J*. *Immunol.,* 138:2213-2229, which is based on an amphipathic helix model.

Other methods for identifying T cell epitopes involve screening GDF-8 or GDF-11 inhibitory peptides of the invention for human T cell stimulating activity. This can be accomplished using one or more of several different assays. For example, *in vitro,* T cell stimulatory activity can be assayed by contacting a peptide of the invention with an antigen presenting cell which presents appropriate MHC molecules in a T cell culture. Presentation of a GDF-8 or GDF-11 inhibitory peptide of the invention in association with appropriate MHC molecules to T cells, in conjunction with the necessary co-stimulation can have the effect of transmitting a signal to the T cell that induces the production of increased levels of cytokines, particularly of interleukin-2 and interleukin-4. The culture supernatant can be obtained and assayed for interleukin-2 or other known cytokines. For example, any one of several conventional assays for interleukin-2 can be employed, such as the assay described in *Proc. Natl. Acad Sci USA,* 86:1333 (1989).

A kit for an assay for the production of interferon is also available from Genzyme Corporation (Cambridge, MA).

A common assay for T cell proliferation entails measuring tritiated thymidine incorporation. The proliferation of T cells can be measured *in vitro* by determining the amount of ³H-labeled thymidine incorporated into the replicating DNA of cultured cells. Therefore, the rate of DNA synthesis and, in turn, the rate of cell division can be quantified.

Other preferred fragments of GDF-8 or GDF-11 that are located on the surface of the protein, e.g., hydrophilic regions, as well as regions with high antigenicity or fragments with high surface probability scores can be identified using computer analysis programs well known to those of skill in the art (Hopp and Wood, (1983), Mol. Immunol., 20,483-9, Kyte and Doolittle, (1982), J. Mol. Biol., 157, 105-32, Corrigan and Huang, (1982), Comput. Programs Biomed, 3, 163-8).

Still other preferred fragments of GDF-8 or GDF-11 to be tested for GDF-8 or GDF-11 inhibitory activity include one or more B-cell epitopes. Such peptides can be identified by immunizing a mammal with the peptide, either alone or combined with or linked to an adjuvant (e.g., a hapten), and testing sera from the immunized animal for anti-GDF-8 or GDF-11 antibodies. Preferred peptides generate anti-GDF-8 or GDF-11 antibodies which inhibit GDF-8 or GDF-11 activity. For example, sera from immunized animals can be tested for GDF-8 or GDF-11 inhibitory activity using any of the GDF-8 or GDF-11 bioassays described herein.

A proteolytic or synthetic GDF-8 or GDF-11 fragment (alone or linked to a hapten) can be used to immunize a suitable subject, (e.g., rabbit, goat, mouse or other mammal or vertebrate). For example, the methods described in U.S. Patent Nos. 5,422,110; 5,837,268; 5,708,155; 5,723,129;and 5,849,531, can be used.

The immunogenic preparation can further include an adjuvant, such as Freund's complete or incomplete adjuvant, or similar immunostimulatory agent. Immunization of a suitable subject with an immunogenic proteolytic or synthetic GDF-8 or GDF-11 fragment preparation induces a polyclonal anti-GDF-8 or GDF-11 antibody response. The anti-GDF-8 or GDF-11 antibody titer in the immunized subject can be monitored over time by standard techniques, such as with an enzyme linked immunosorbent assay (ELISA) using immobilized GDF-8 or GDF-11. Subsequently, the sera from the immunized subjects can be tested for their GDF-8 or GDF-11 inhibitory activity using any of the bioassays described herein.

The antibody molecules directed against GDF-8 or GDF-11 can be isolated from the mammal (e.g., from the blood) and further purified by well known techniques, such as protein A chromatography to obtain the IgG fraction. At an appropriate time after immunization, e.g., when the anti-GDF-8 or GDF-11 antibody titers are highest, antibody-producing cells can be obtained from the subject and used to prepare e.g., monoclonal antibodies by standard techniques, such as the hybridoma technique originally described by Kohler and Milstein (1975) *Nature* 256:495-497) (see also, Brown et al. (1981) *J*. *Immunol.* 127:539-46; Brown et al. (1980) *J. Biol. Chem* 255:4980-83; Yeh et al. (1976) *Proc. Natl. Acad. Sci. USA* 76:2927-31: and Yeh et al. (1982) *Int. J Cancer* 29:269-75), the more recent human B cell hybridoma technique (Kozbor et al. (1983) *Immunol Today* 4:72), the EBV-hybridoma technique (Cole et al. (1985), *Monoclonal Antibodies and Cancer Therapy,* Alan R. Liss, Inc., pp. 77-96) or trioma techniques. The technology for producing monoclonal antibody hybridomas is well known (see generally R. H. Kenneth, in *Monoclonal Antibodies: A New Dimension In Biological Analyses.* Plenum Publishing Corp., New York, New York (1980); E. A. Lemer (1981) *Yale J. Biol. Med,* 54:387-402; M. L. Gefter et al. (1977) *Somatic Cell Genet.* 3:231-36). Briefly, an immortal cell line (typically a myeloma) is fused to lymphocytes (typically splenocytes) from a mammal immunized with a GDF-8 or GDF-11 immunogen as described above, and the culture supernatants of the resulting hybridoma cells are screened to identify a hybridoma producing a monoclonal antibody that binds GDF-8 or GDF-11.

Any of the many well known protocols used for fusing lymphocytes and immortalized cell lines can be applied for the purpose of generating an anti-GDF-8 or GDF-11 monoclonal antibody (see, e.g., G. Galfre et al. (1977) *Nature* 266:55052; Gefter et al. *Somatic Cell Genet.,* cited *supra;* Lerner, *Yale J. BioL Med,* cited *supra;* Kenneth, *Monoclonal Antibodies,* cited *supra).* Moreover, the ordinarily skilled worker will appreciate that there are many variations of such methods which also would be useful. Typically, the immortal cell line (e.g., a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, e.g., the P3-NSI/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind GDF-8 or GDF-11, e.g., using a standard ELISA assay. The antibodies can then be tested for GDF-8 or GDF-11 inhibitory activity using, for example, the assays described herein.

In another aspect of the invention, GDF-8 peptide inhibitors comprise all or a portion of the GDF-8 pro-domain. The pro-domain of GDF-8 or a portion thereof can be generated using various expression systems (e.g., CHO, baculovirus and the like). The expressed pro-domain of GDF-8 can be purified by, for example, using the method described in Bottinger et. al. (1996) *PNAS* 93:5877-5882, or any other art recognized method for purifying peptides. Alternatively, the pro-domain can be tagged with, for example, FLAG or 6-His, as described in the Examples below. In addition, the pro-domain of GDF-8 or a portion thereof can be generated by cleavage, e.g., chemical cleavage, of the native GDF-8. Moreover, the pro-domain of GDF-8 or a portion thereof can be chemically synthesized using art known techniques described herein.

In a specific embodiments, the GDF-8 inhibitor is a peptide, e.g., a pentacosapeptide, that includes (e.g. spans) the C-terminus of mature GDF-8. Preferably, the GDF-8 peptide is about 25 amino acids in length and comprises or consists essentially of a sequence selected from: ANYCSGECEFVFLQKYPHTHLVH (SEQ ID NO:25), KIPAMVVDRCGCS (SEQ ID NO:29), and/or LSKLRLETAPNISKDVIRQLLP (SEQ ID NO:30). In another embodiment, the GDF-8 inhibitor has all or a portion of the above-identified sequence and a length of about, 20-25, 25-30, 30-35, 35-40, or 40-45 amino acid residues. GDF-8 peptide inhibitors which are based on these peptides can then be tested for GDF-8 or GDF-11 inhibitory activity using the assays described herein.

In another embodiment, GDF-8 and GDF-11 inhibitors of the present invention are peptides, peptidomimetics, or small molecules which inhibit the release of the mature GDF-8 protein from the GDF-8/pro-domain complex, and/or which stabilize the GDF-8/pro-domain complex. Such GDF-8 and GDF-11 inhibitors include cysteine protease inhibitors, e.g., m-calpain inhibitors.

In yet another embodiment, GDF-8 and GDF-11 inhibitors of the present invention are soluble GDF-8 or GDF-11 receptors, *e.g.*, soluble fragments of GDF-8 or GDF-11 receptors which compete (with GDF-8 or GDF-11) for binding to GDF-8 or GDF-11 receptors. Such soluble GDF-8 or GDF-11 receptors are described herein.

### B. GDF-8 AND GDF-11 PROTEIN VARIANTS WHICH INHIBIT GDF-8 AND GDF-11 ACTIVITY

In another embodiment, the GDF-8 and GDF-11 inhibitors of the present invention are GDF-8 or GDF-11 protein variants which do not possess detectable GDF-8 activity. GDF-8 or GDF-1 1 variants of the invention may contain one or more conservative or non-conservative amino acid substitutions, deletions, insertions, or premature truncations of the amino acid sequence of wild type GDF-8 or GDF-11. Alternatively, the variants may contain one or more conservative or non-conservative amino acid substitutions, insertions or deletions in critical residues or critical regions for activity of the GDF-8 or GDF-11 protein. The resulting GDF-8 or GDF-11 protein variants interfere with, for example, GDF-8 or GDF-11 processing, secretion or/and biological activity, thereby serving as a GDF-8 or GDF-11 inhibitor.

A "conservative amino acid substitution" is one in which the amino acid residue is replaced with an amino acid residue having a similar side chain (e.g., charge, size etc.). A "non-conservative amino acid substitution" is one in which the amino acid residue is replaced with an amino acid residue having a side chain which is not similar (e.g., change, size etc.). Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

### 1. Cleavage Site Variants

In one embodiment, GDF-8 and GDF-11 inhibitors of the invention are dominant negative mutants of GDF-8 and GDF-11 proteins. Growth factors belonging to the TGF-β superfamily, such as GDF-8 and GDF-11, form homodimers and heterodimers by the dimerization of two precursor molecules. This interaction is necessary for the secretion of these factors. Activation of the ligand dimer requires cleavage of the carboxy-terminal mature peptide from the amino-terminal precursor remainder and occurs under correct physiological conditions. Thus, mutations in the conserved cleavage sequences, required for activation, can result in the synthesis of non-functional GDF-8 and GDF-11 molecules. Moreover, overexpression of these mutated precursor molecules can lead to heterodimer formation of the mutated GDF-8 and GDF-11 polypeptides with endogenous unmodified monomers in competitive fashion. When GDF-8 and GDF-11 mutants are expressed at high levels, most of the endogenous dimer will be titrated out by heterodimer formation, thus, completely blocking the secretion of the active growth factor, e.g., GDF-8 and/or GDF-11, in a specific manner. GDF-8 and GDF- 11 dominant negative mutants, e.g., the cleavage site mutant, can be prepared as described in the Examples below using the techniques described in, for example, Lopez *et al.* (1992) *Mol. Cell. Biol.,* 12, 1674-1679; Wittbrodt and Rosa, (1994) *Genes & Dev.,* 8, 1448-1462; Suzuki *et al.* (1997) *Dev. Biol.,* 189, 112-122; and Hawley *et al.* (1995) *Genes & Dev.,* 9, 2923-2935.

### 2. Cysteine Variants

In another embodiment, GDF-8 and GDF- 11 inhibitors of the present invention are GDF-8 and GDF-11 proteins containing point mutations at one or more critical cysteine residues involved in, for example, the intramolecular cysteine bridges which maintain the tertiary structure of TGF-β family members such as GDF-8 and GDF-11. For example, cysteine residues which are conserved among all members of the TGF-β superfamily, e.g., the cysteine residue at position 313 of the GDF-8 polypeptide, may be non-conservatively replaced with an amino acid residue which does not have a similar side chain (as described above). The cysteine mutants of the present invention can be prepared as described in the Examples section below using the techniques described in, for example, McPherron and Lee (1997) *Nature,* 387, 83-90 and Kambadur *et al.* (1997) *Genome Res.,* 7, 910-15.

### 3. Pro-domain Variants

In another embodiment, GDF-8 and GDF-11 inhibitors of the present invention are modified forms of the GDF-8 or GDF-11 polypeptides which include all or a portion of the pro-domain of the GDF-8 or GDF-11 polypeptide. Inhibitors comprising the pro-domain of GDF-8 and GDF-11 proteins, referred to herein as "Pro-GDF-8" and "Pro-GDF-11", respectively, can be prepared as described in the previous sub-section and in the Examples section below using the techniques described in, for example, Bottinger *et. al.* (1996) *PNAS,* 93, 5877-5882 and Gentry and Nash (1990) *Biochemistry* 29, 6851-6857. "Pro-GDF-8" and "Pro-GDF-11" inhibitors of the present invention can be used to inhibit GDF-8 or GDF-11 activity in the same species from which the pro-domain was derived or in a different species (*e.g.*, the mouse pro-GDF-8 can be used to inhibit human GDF-8 activity). In a preferred ambodiment, the inhibitor (e.g., Pro-GDF-8) comprises the N-terminus of the pro-domain of GDF-8 (*e.g.*, comprises a region of the pro-domain that is upstream of Arg 99 (see Figure 11)).

### 4. Post-Translational Modification Variants

In a further embodiment, GDF-8 and GDF-11 inhibitors of the present invention include GDF-8 or GDF-11 proteins or peptides which do not include post-translational modifications necessary for activity of the GDF-8 or GDF-11 protein or peptide (e.g., which include abberrant post-transational modifications). As used herein, the term "aberrant" includes a post-translational modification which deviates from the wild type post-translational modification of the GDF-8 or the GDF-11 polypeptides. Aberrant post-translational modifications include increased or decreased post-translational modifications, as well as post-translational modifications which do not follow the wild type pattern of GDF-8 or GDF-11 polypeptide post-translational modifications. As used herein, the term "post-translational modifications" includes any modification that the GDF-8 or the GDF-11 polypeptide undergoes after it has been translated (e.g., after peptide bond formation has occurred). Examples of post-translational modifications include glycosylation, acylation, limited proteolysis, phosphorylation, and isoprenylation. Post-translational modifications play an important role in protein processing, secretion and biological activity.

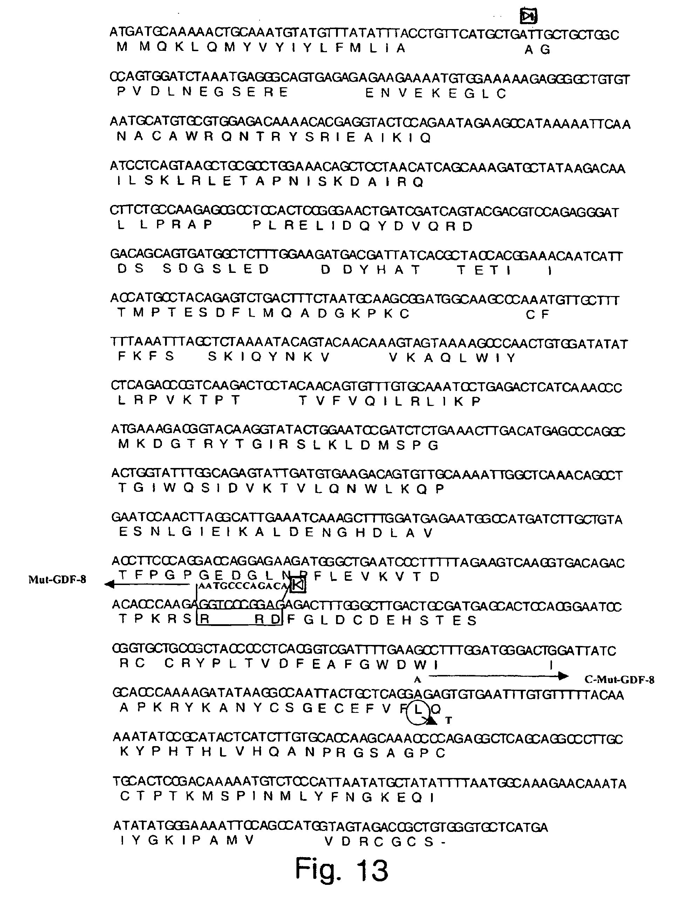

In a preferred embodiment, the GDF-8 and GDF-11 inhibitors of the present invention have aberrant glycosylation. For example, certain GDF-8 inhibitors of the present invention contain a mutation at the predicted N-linked glycosylation site within the GDF-8 pro-domain (see *Figure 13).*

### II. GDF-8 AND GDF-1 1 NUCLEIC ACID INHIBITORS

### A. RIBOZYMES

In still another embodiment, GDF-8 and GDF-11 inhibitors of the invention are ribozymes directed against GDF-8 or GDF-1 1 gene transcripts. Ribozymes are catalytic RNA molecules with ribonuclease activity which are capable of cleaving a single-stranded nucleic acid, such as an mRNA, to which they hybridize based on having a complementary region. Thus, ribozymes (e.g., hammerhead ribozymes described in Haselhoff and Gerlach (1988) *Nature* 334:585-591) of the invention can be used to catalytically cleave GDF-8 or GDF-11 mRNA transcripts, thereby inhibiting translation of GDF-8 or GDF-11 mRNA. A ribozyme having specificity for a GDF-8- or GDF-11-encoding nucleic acid can be designed based upon the nucleotide sequence of a GDF-8 or GDF-11 cDNA such as those described in McPherron A. C. et al. (1997) *Proc. Natl. Acad Sci.* 94:12457-12461 and U.S. Application Serial No. 08/706958.

For example, a derivative of a *Tetrahymena* L-19 IVS RNA can be constructed in which the nucleotide sequence of the active site is complementary to the nucleotide sequence to be cleaved in a GDF-8- or GDF-11-encoding mRNA (see, e.g., Cech et al. U.S. Patent No. 4,987,071; and Cech et al. U.S. Patent No. 5,116,742). Alternatively, GDF-8 or GDF-11 mRNA can be used to select a catalytic RNA having a specific ribonuclease activity from a pool of RNA molecules (see, e.g., Bartel, D. and Szostak, J.W. (1993) *Science* 261:1411-1418).

Alternatively, GDF-8 or GDF-11 inhibitor ribozymes can comprise a nucleotide sequence complementary to one or more of the regulatory regions of GDF-8 or GDF-11 genes (e.g., the GDF-8 or GDF-11 promoter and/or enhancers) to form triple helical structures that prevent transcription of the GDF-8 or GDF-11 gene in target cells. (The techniques for this are described in, for example, Helene, C. (1991) *Anticancer Drug Des.* 6(6):569-84; Helene, C. et al. (1992) *Ann. N.Y. Acad. Sci.* 660:27-36; and Maher, L.J. (1992) *Bioassays* 14(12):807-15).

In a particular embodiment, GDF-8 and GDF-1 1 inhibitors of the invention are ribozymes comprising one of the four nucleotide sequences set forth in SEQ ID NOs:1-4. GDF-8 and GDF-11 inhibitors of the invention also include ribozymes having a nucleotide sequence at least 70%, 75%, 80%, 85%, 90%, 95%, 98%, or more identical to the nucleotide sequence set forth in SEQ ID NOs:1-4 which inhibit expression of GDF-8 or GDF-11. To determine the percent identity of two nucleic acid sequences, the sequences are aligned for optimal comparison purposes (e.g., gaps can be introduced in one or both of a first and a second nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence. The nucleotides at corresponding nucleotide positions are then compared. When a position in the first sequence is occupied by the same nucleotide as the corresponding position in the second sequence, then the molecules are identical at that position (as used herein nucleic acid "identity" is equivalent to nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. In another embodiment, the percent identity between two nucleotide sequences is determined using the algorithm of E. Meyers and W. Miller (CABIOS, 4: 11-17 (1989)) which has been incorporated into the ALIGN program (version 2.0), using a PAM120 weight residue table, a gap length penalty of 12 and a gap penalty of 4.

### B. GDF-8 AND GDF-11 ANTISENSE OLIGONUCLEOTIDES

In another aspect, the invention provides GDF-8 or GDF-11 inhibitors in the form of isolated antisense nucleic acid molecules. An "antisense" nucleic acid comprises a nucleotide sequence which is complementary to a "sense" nucleic acid encoding a protein, e.g., complementary to the coding strand of a double-stranded cDNA molecule or complementary to an mRNA sequence. Accordingly, an antisense nucleic acid can hydrogen bond to a sense nucleic acid. The antisense nucleic acids of the invention can be complementary to an entire GDF-8 or GDF-11 coding strand, or to a portion thereof. In one embodiment, the GDF-8 or GDF-11 antisense nucleic acid is antisense to the coding region of a nucleotide sequence encoding GDF-8 or GDF-11. The term "coding region" refers to the region of the nucleotide sequence comprising codons which are translated into amino acid residues. In another embodiment, the antisense nucleic acid is antisense to a noncoding region of a nucleotide sequence encoding GDF-8 or GDF-11. The term "noncoding region" refers to 5' and 3' sequences which flank the coding region that are not translated into amino acids (i.e., also referred to as 5' and 3' untranslated regions).

Based on the known nucleotide sequences encoding GDF-8 and GDF-11, antisense nucleic acids of the invention can be designed according to the rules of Watson and Crick base pairing. The antisense nucleic acid molecule can be complementary to the entire coding region of GDF-8 or GDF-11 mRNA, but typically is an oligonucleotide which is antisense to only a portion of the coding or noncoding region of GDF-8 or GDF-11 mRNA. For example, the antisense oligonucleotide can be complementary to the region surrounding the translation start site of GDF-8 or GDF-11 mRNA. Suitable antisense oligonucleotides are, for example, about 5, 10,15, 20, 25, 30, 35, 40, 45 or 50 nucleotides in length. Such antisense GDF-8 or GDF-11 inhibitors of the invention can be constructed using chemical synthesis and enzymatic ligation reactions in accordance with procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense GDF-8 or GDF-11 inhibitors of the invention are typically administered to a subject or generated *in situ* such that they hybridize with or bind to cellular mRNA and/or genomic DNA encoding a GDF-8 or GDF-11 polypeptide to thereby inhibit expression of the polypeptide, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention include direct injection at a tissue site. Alternatively, antisense nucleic acid molecules can be modified to target selected cells and then administered systemically. For example, for systemic administration, antisense GDF-8 or GDF-11 molecules can be modified such that they specifically bind to receptors or antigens expressed on a selected cell surface, e.g., by linking the antisense nucleic acid molecules to peptides or antibodies which bind to cell surface receptors or antigens. The antisense GDF-8 or GDF-11 nucleic acid molecules can also be delivered to cells using art known vectors (e.g., expression vectors which are transcribed as an anti-sense mRNA directed against a GDF-8 or GDF-11 mRNA). To achieve sufficient intracellular concentrations of the antisense GDF-8 or GDF-11 molecules, vector constructs in which the antisense GDF-8 or GDF-11 nucleic acid molecule is placed under the control of a strong promoter, e.g., pol II or pol III promoter, are preferred.

In yet another embodiment, antisense GDF-8 or GDF-11 inhibitors of the invention include α-anomeric nucleic acid molecules. An α-anomeric nucleic acid molecule forms specific double-stranded hybrids with complementary RNA in which, contrary to the usual β-units, the strands run parallel to each other (Gaultier et al. (1987) *Nucleic Acids. Res.* 15:6625-6641). The antisense nucleic acid molecule can also comprise a 2'-o-methylribonucleotide (Inoue et al. (1987) *Nucleic Acids Res.* 15:6131-6148) or a chimeric RNA-DNA analogue (Inoue et al. (1987) *FEBS Lett.* 215:327-330).

In a particularly preferred embodiment, GDF-8 and GDF-11 inhibitors of the invention are antisense oligonucleotides comprising one of the twenty nucleotide sequences set forth in SEQ ID NOs:5-24.

### III. GDF-8 OR GDF-11 ASSAYS TO TEST FOR INHIBITION

GDF-8 or GDF-11 inhibitors of the present invention can be identified using a variety of appropriate assays which test for inhibition of GDF-8 or GDF-11 activity. Preferably, the inhibition is specific, i.e., the GDF-8 inhibitor can specifically inhibit the GDF-8 protein without affecting the activity of other proteins and the GDF-11 inhibitor can specifically inhibit the GDF-11 protein without affecting the activity of other proteins. In certain embodiments, the GDF-8 inhibitor is also able to inhibit GDF-11 activity and the GDF-11 inhibitor is also able to inhibit GDF-8 activity.

As used herein, the term "assay" includes any assay designed to identify a GDF-8 or GDF-11 inhibitor. The assay can be an *in vitro* or an *in vivo* assay suitable for identifying whether the GDF-8 or GDF-11 inhibitor effects, e.g., downmodulates, one or more of the biological functions of GDF-8 or GDF-11. Examples of suitable assays include DNA replication assays, transcription-based assays, creatine kinase assays, assays based on the differentiation of 3T3-L1 pre-adipocytes, assays based on glucose uptake control in 3T3-L1 adipocytes, and immunological assays, all as described in subsection II below and in the following Examples.

### Creatine Kinase Bioassay for the Identification of GDF-8 or GDF-11 Inhibitors

GDF-8 and GDF-11 modulate the protein levels, and therefore the activity, of a muscle-specific enzyme, creatine kinase. This effect of GDF-8 or GDF-11 can be used to screen for potential GDF-8 or GDF-11 inhibitors. Specifically, a creatine kinase assay can be performed in a myoblast, e.g., the mouse skeletal myoblast cell line CIC12 or primary chick myoblasts isolated from Day 11 chick embryos. To test for the inhibitors' ability to modulate the activity of creatine kinase, cells are typically grown in 48-well trays in serum-containing medium that maintains them undifferentiated. When a 70% confluence has been reached, medium is switched to 1% serum, thus allowing differentiation and creatine kinase expression. At the time of the switch, the potential GDF-8 or GDF-11-inhibitory fraction is added to some wells, followed some time later by GDF-8 or GDF-11 itself. Cells are returned to the incubator for an additional two to three day period. In the end, cells are lysed and creatine kinase activity is measured in the lysates using a commercially available kit (available by Sigma, St Louis, MO).

### Assay Based on the Differentiation of 3T3-L1 Pre-adipocytes

GDF-8 and GDF-11 interfere with the differentiation process of 3T3-L1 pre-adipocytes (fibroblasts) to adipocytes. This effect of GDF-8 or GDF-11 can be used to screen for potential GDF-8 or GDF-11 inhibitors. Specifically, the bioassay of the invention can be performed in the following manner. 3T3-L1 pre-adipocytes are allowed to reach confluence, and subsequently differentiation can be achieved by successive replacements of their serum-containing DMEM media as follows: DMEM + serum + methylisobutylxanthine + dexamethasone + insulin for 2 days, DMEM + serum + insulin for 2 additional days, DMEM + serum for 3 additional days (Spiegelman *et al.,* (1993) J. *Biol. Chem.* 268, 6823-6826). GDF-8 and potential inhibitors can be added at the onset of differentiation and re-supplied at each additional medium change. The degree of adipocyte differentiation can be assessed by various ways, e.g., visually by estimation of the content of fat droplets in the pre-adipocytes or, by quantitation of the glycerol/triglyceride content of cell lysates, using the Triglyceride (Gro-Trinder) kit from Sigma (St Louis, MO) according to the manufacturer's instructions.

### Assay Based on Glucose Uptake Control in 3T3-L1 Adipocytes

GDF-8 and GDF-11 modulate glucose uptake control in adipocytes. This effect of GDF-8 or GDF-11 can be used to screen for potential GDF-8 or GDF-11 inhibitors. Specifically, 3T3-L1 pre-adipocytes can be induced to fully differentiate following the protocol described above. Subsequently, a glucose transport assay is performed. Briefly, following differentiation, media are changed to serum-free DMEM, and cells are treated with the GDF-8 or GDF-11 inhibitor and GDF-8 or GDF-11 for a variable period of time ranging from 2 hours to three days. Subsequently, cells are switched for four hours to low glucose, serum-free DMEM (without the factors and inhibitors). Then. cells are washed twice with Ringer/Krebs solution and insulin (0.01 to 1 µM) is added to them for 5 to 20 minutes. Insulin is washed twice and tritiated (radioactive) deoxy-glucose (NEN Dupont) is added at 1 µCi/ml final concentration in Ringer buffer. Glucose uptake is left to proceed for 5 to 10 minutes, at which time cells are washed with excess volume of Krebs, solubilized, and radioactive glucose uptake is determined by scintillation counting of the cell lysates.

### DNA Replication Assay

GDF-8 and GDF-11 inhibitors of the invention can be tested using a DNA replication assay. This bioassay is a cell-based, growth assay and can be performed in any cell type responsive to GDF-8 or GDF-11. In this type of assay DNA replication and, thus, proliferation is accurately measured by the incorporation of Bromo-deoxyUridine (BrdU) or tritiated thymidine ([³H]-TdR) label into the DNA. This bioassay can be perfomed using several cell lines including, but not limited to, G8 mouse skeletal myoblasts, C2C 12 mouse myoblasts and the mink lung epithelial cell line, CCL-64, and its mutant derivatives.

Briefly, cells are plated using the appropriate culture conditions. For example, G8 myoblasts are plated in 96-well culture plates coated with 0.1% gelatin (Sigma), and CCL-64 cells are plated in un-coated plates. Cells are plated in serum-free DMEM containing 0.1% w/v BSA at 10×10³ cells per well. The following day, media are removed and replaced with fresh media. The relevant inhibitory fraction is added, and cells are returned to the incubator for 60 minutes before growth factors (GDF-8, Activin, and the like) are applied to them. In another embodiment, the inhibitory fraction can be mixed with the growth factor for 30 minutes to 2 hours at room temperature, to allow for inhibitor-ligand interactions, and thereafter the mixture can be applied onto the cells. In G8 myoblasts, the appropriate label is added 24 hours after the test factors, and incorporation is allowed to proceed for an additional 24 hours. In CCL-64 cells, the appropriate label is added 12 hours after the test factors, and incorporation is allowed to proceed for an additional 4 hours. When BrdU is used as the label, cells are finally fixed and processed according to the manufacturer's instructions manual using a commercially available kit (Boehringer-Manheim). The incorporated BrdU is determined colorimetrically. When [³H]-thymidine (NEN-Dupont) is used as a label, cells are washed twice with 10% trichloroacetic acid and radioactivity is extracted with 0.3N NaOH and determined by scintillation counting.

### Transcription-Based Assay

In another embodiment, GDF-8 or GDF-11 inhibitors of the present invention can be identified using a transcription-based assay in any GDF-8- or GDF-11-responsive cell line including, but not limited to G8 myoblasts, C2C12 myoblasts. CCL-64 mink lung epithelial cells, and A204 human rhabdomyosarcoma cells.

Artificial reporter plasmids which respond to GDF-8 or GDF-11 in this type of assay can be used. For example, p3TP-Lux (described in, for example, Wrana *et al.* (1992) *Cell,* 71, 1003-14) and p(CAGA)₁₂-MLP (described in, for example, Dennler *et al.* (1998) *EMBO J.,* 17, 3091-3100) can be used in this assay. In both p3TP-Lux and p(CAGA)₁₂-MLP, luciferase gene transcription (and thus activity) is driven by artificial minimal promoters which respond to members of the TGF-β family. Therefore, luciferase activity in cell lysates correlates linearly with the degree of stimulation of the cells by the applied growth factors.

Briefly, cells are plated in 48-well plates in the appropriate media (e.g., DMEM for CCL-64 and McCoy's medium for A204) supplemented with 10% fetal bovine serum, antibiotics and L-Glutamine. Upon reaching 80% confluence, cells are transfected using FuGENE-6 (Boehringer-Manheim) to facilitate plasmid uptake, according to the manufacturer's instructions. Cells are transiently transfected with a cocktail of two plasmids; pSV-β-gal plasmid to monitor transfection efficiency, and either of the two luciferase reporter plasmids. After overnight incubation with the transfection reagents, cells are washed twice with the appropriate serum-free medium containing 0.1% BSA. The putative inhibitory fraction is then added and cells are returned to the incubator for 60 minutes. At the end of this period, the following factors can be added: human recombinant Myostatin/GDF-8, produced from GDF-8-expressing CHO cell conditioned medium, human TGF-β₁ (R&D Biosystems, Minneapolis) and concentrated conditioned medium from CHO cells expressing Activin βA.

In another embodiment, the putative inhibitor is mixed with GDF-8, GDF-11, or other ligand for 30 minutes to 2 hours at room temperature and the mixture is added onto the recipient cells. Cells are lysed after a 6 hour incubation and luciferase and β-galactosidase activity can be determined in the same sample using the Dual-Light Luciferase Assay kit from Tropix Inc. Activity is expressed in Relative Luciferase units (RLU), i.e. luciferase activity corrected for transfection efficiency that is given by the corresponding values of β-galactosidase activity measured in the same sample.

### Protein Secretion Based Assays

In another embodiment, GDF-8 or GDF-1 inhibitors of the present invention can be identified using a protein secretion based assay. Briefly, the constructs encoding the GDF-8 or GDF-11 inhibitors (e.g., the dominant negative mutants, cysteine mutants, pro-domain variants and post-translational modification variants) can be introduced or co-introduced in muscle cells lines such as QM7 and RD, with a wild-type GDF-8 or GDF-11 producing construct. Subsequently, the ability of the co-transfected cells to produce and secrete mature GDF-8 or GDF-11 can be tested by, for example, a western blot analysis. GDF-8 or GDF-11 inhibitors which inhibit production and/or secretion of mature GDF-8 or GDF-11 can then be selected.

### IV. USES OF GDF-8 OR GDF-11 INHIBITORS

In another aspect, the invention provides GDF-8 or GDF-11 inhibitors identified by the methods described herein, as well as compositions, e.g., pharmaceutical compositions, and methods of using the inhibitors. The GDF-8 or GDF-11 inhibitors identified by the methods described herein can be used to modulate GDF-8 or GDF-11 expression or activity for therapeutic purposes. In an exemplary embodiment, a cell is contacted with a GDF-8 or GDF-11 inhibitor that modulates one or more of the activities of GDF-8 or GDF-11 protein activity associated with the cell. The contacting can be performed *in vitro* (e.g., by culturing the cell with the GDF-8 or GDF-11 inhibitor), *in vivo* (e.g., by administering the GDF-8 or GDF-11 inhibitor to a subject), or *in ovo* (e.g., by injecting the GDF-8 or GDF-11 inhibitor into eggs). The ovo injection can be performed as described herein using the techniques of, for example, H. Kocamis *et al.* (1998) *Poult. Sci.* 77, 1913-1919; or P.A. *Johnston et al.,* (1997) *Poult. Sci.* 76, 165-178; C.E. Dean *et al.* (1993) *Growth Dev. Aging* 57, 59-72.

As such, the present invention further provides methods of treating an individual afflicted with a disease or disorder characterized by aberrant expression or activity of a GDF-8 or GDF-11 protein or nucleic acid molecule, e.g., a muscle-associated disorder. Inhibition of GDF-8 or GDF-11 activity is desirable in situations in which GDF-8 or GDF-11 is abnormally upregulated and/or in which decreased GDF-8 or GDF-11 activity is likely to have a beneficial effect. Examples of disorders which can be treated using the GDF-8 or GDF-11 inhibitors of the invention include muscle-associated disorders such as cancer, muscular dystrophy, spinal cord injury, traumatic injury, congestive obstructive pulmonary disease, AIDS or cachexia. In addition, the GDF-8 or GDF-11 inhibitors of the invention can be used to treat obesity and related disorders, or disorders related to abnormal proliferation of adipocytes.

In preferred embodiments, the GDF-8 or GDF-11 inhibitors of the invention are used to treat diabetes, obesity, and disorders related to obesity. For example, GDF-8 or GDF-11 inhibitors of the invention can be used to modulate glucose transport in a subject, e.g., by increasing the activity of the glucose transporter GLUT4 in the subject.

The GDF-8 or GDF-11 inhibitors of the invention can further be used to decrease GDF-8 or GDF-11 activity in a subject. As used herein, the term "subject" includes any animal which expresses GDF-8 or GDF-11, preferably a mammal. In a preferred embodiment, the subject is a vertebrate. In an even more preferred embodiment, the vertebrate is a chicken, a turkey, a pig, a cow, a mouse, a rat, a rabbit, a goat, a fish, or a human. For example, the GDF-8 or GDF-11 inhibitors of the invention can be used to increase muscle mass in a subject, e.g., a chicken.

The GDF-8 or GDF-11 inhibitors of the invention can be incorporated into pharmaceutical compositions suitable for administration. Such compositions typically comprise the GDF-8 or GDF-11 inhibitor and a pharmaceutically acceptable carrier. As used herein the language "pharmaceutically acceptable carrier" is intended to include ) any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the GDF-8 or GDF-11 inhibitor, use thereof in the compositions is contemplated. Supplementary active compounds can also be incorporated into the compositions.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the GDF-8 or GDF-11 inhibitor in the required amount in an appropriate solvent with one or a combination of ingredients enumerated above, as required, followed by filtered sterilization. Generally, dispersions are prepared by incorporating the GDF-8 or GDF-11 inhibitor into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the GDF-8 or GDF-11 inhibitor can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The GDF-8 or GDF-11 inhibitor can also be prepared in the form of suppositories (e.g., with conventional suppository bases such as cocoa butter and other glycerides) or retention enemas for rectal delivery.

In one embodiment, the GDF-8 or GDF-11 inhibitors are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. GDF-8 or GDF-11 inhibitors which exhibit large therapeutic indices are preferred. While GDF-8 or GDF-11 inhibitors that exhibit toxic side effects may be used, care should be taken to design a delivery system that targets such GDF-8 or GDF-11 inhibitors to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any GDF-8 or GDF-11 inhibitor used in the method of the invention, the therapeutically effective dose can be estimated initially from cell culture assays. A dose may be formulated in animal models to achieve a circulating plasma concentration range that includes the IC50 (i.e., the concentration of the test GDF-8 or GDF-11 inhibitor which achieves a half-maximal inhibition of symptoms) as determined in cell culture. Such information can be used to more accurately determine useful doses in humans. Levels in plasma may be measured, for example, by high performance liquid chromatography.

The pharmaceutical compositions can be included in a container, pack, or dispenser together with instructions for administration.

The GDF-8 and GDF-11 inhibitors of the present invention, e.g., ribozymes or antisense inhibitors, can further be inserted into vectors and used in gene therapy. Gene therapy vectors can be delivered to a subject by, for example, intravenous injection, local administration (see U.S. Patent 5,328,470) or by stereotactic injection (see e.g., Chen et al. (1994) *Proc. Natl. Acad. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

Vectors suitable for use in gene therapy are known in the art. For example, adenovirus-derived vectors can be used. The genome of an adenovirus can be manipulated such that it encodes and expresses a gene product of interest but is inactivated in terms of its ability to replicate in a normal lytic viral life cycle. See for example Berkner et al. (1988) *BioTechniques* 6:616; Rosenfeld et al. (1991) *Science* 252:431-434; and Rosenfeld et al. (1992) *Cell* 68:143-155. Suitable adenoviral vectors derived from the adenovirus strain Ad type 5 d1324 or other strains of adenovirus (e.g., Ad2, Ad3, Ad7 etc.) are well known to those skilled in the art. Recombinant adenoviruses can be advantageous in certain circumstances in that they are not capable of infecting nondividing cells. Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (e.g., retroviral DNA). Moreover, the carrying capacity of the adenoviral genome for foreign DNA is large (up to 8 kilobases) relative to other gene delivery vectors (Berkner et al. cited *supra;* Haj-Ahmand and Graham (1986) *J. Virol.* 57:267). Most replication-defective adenoviral vectors currently in use and therefore favored by the present invention are deleted for all or parts of the viral E1 and E3 genes but retain as much as 80 % of the adenoviral genetic material (see, e.g., Jones et al. (1979) *Cell* 16:683; Berkner et al., *supra;* and Graham et al. in Methods in Molecular Biology, E.J. Murray, Ed. (Humana, Clifton, NJ, 1991) vol. 7. pp. 109-127). Expression of the gene of interest comprised in the nucleic acid molecule can be under control of, for example, the E1 A promoter, the major late promoter (MLP) and associated leader sequences, the E3 promoter, or exogenously added promoter sequences.

Yet another viral vector system useful for delivery of the GDF-8 and GDF-11 inhibitors of the invention is the adeno-associated virus (AAV). Adeno-associated virus is a naturally occurring defective virus that requires another virus, such as an adenovirus or a herpes virus, as a helper virus for efficient replication and a productive life cycle. (For a review see Muzyczka et al. *Curr. Topics in Micro. and Immunol.* (1992) 158:97-129). Adeno-associated virusses exhibit a high frequency of stable integration (see for example Flotte et al. (1992) *Am. J Respir. Cell. Mol. Biol.* 7:349-356; Samulski et al. (1989) *J. Virol.* 63:3822-3828; and McLaughlin et al. (1989) *J Virol.* 62:1963-1973). Vectors containing as few as 300 base pairs of AAV can be packaged and can integrate. Space for exogenous DNA is limited to about 4.5 kb. An AAV vector such as that described in Tratschin et al. (1985) *Mol. Cell. Biol.* 5:3251-3260 can be used to introduce DNA into T cells. A variety of nucleic acids have been introduced into different cell types using AAV vectors (see for example Hermonat et al. (1984) *Proc. Natl. Acad. Sci. USA* 81:6466-6470; Tratschin et al. (1985) *Mol. Cell. Biol.* 4:2072-2081; Wondisford et al. (1988) *Mol. Endocrinol.* 2:32-39; Tratschin et al. (1984) *J. Virol.* 51:611-619; and Flotte et al. (1993) *J. Biol. Chem.* 268:3781-3790). Other viral vector systems that may be useful for delivery of the GDF-8 and GDF-11 inhibitors of the invention are derived from herpes virus, vaccinia virus, and several RNA viruses.

The GDF-8 and GDF-11 inhibitors of the present invention can further be used to generate transgenic animals in which the GDF-8 and GDF-11 inhibitors interfere with GDF-8 or GDF-11 processing, GDF-8 or GDF-11 secretion, and/or GDF-8 or GDF-11 biological activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, turkeys, amphibians, fish, and the like. A transgene is exogenous DNA which is integrated into the genome of a cell from which a transgenic animal develops and which remains in the genome of the mature animal, thereby directing the expression of an encoded gene product in one or more cell types or tissues of the transgenic animal.

A transgenic animal can be created by introducing GDF-8 or GDF-11 inhibitor-encoding nucleic acid or GDF-8- or GDF-11-encoding nucleic acid into the male pronucleus of a fertilized oocyte, e.g., by microinjection, retroviral infection, and allowing the oocyte to develop in a pseudopregnant female foster animal. Intronic sequences and polyadenylation signals can also be included in the transgene to increase the efficiency of expression of the transgene. A tissue-specific regulatory sequence(s) can be operably linked to the GDF-8 or GDF-11 transgene to direct expression of the GDF-8 or GDF-11 inhibitor to particular cells. Methods for generating transgenic animals via embryo manipulation and microinjection, particularly animals such as mice, have become conventional in the art and are described, for example, in U.S. Patent Nos. 4.736.866 and 4,870,009, both by Leder et al., U.S. Patent No. 4,873,191 by Wagner et al. and in Hogan, B., *Manipulating the Mouse Embryo,* (Cold Spring Harbor Laboratory Press, Cold Spring Harbor, N.Y., 1986). Similar methods are used for production of other transgenic animals. A transgenic founder animal can be identified based upon the presence of the GDF-8 or GDF-11 inhibitor transgene in its genome and/or expression of the GDF-8 or GDF- 11 inhibitor mRNA in tissues or cells of the animals. A transgenic founder animal can then be used to breed additional animals carrying the transgene. Moreover, transgenic animals carrying a transgene encoding the GDF-8 or GDF-11 inhibitor can further be bred to other transgenic animals carrying other transgenes.

Clones of GDF-8 or GDF-11 inhibitor transgenic animals can also be produced according, for examples, to the methods described in Wilmut, I. *et al.* (1997) *Nature* 385:810-813 and PCT International Publication Nos. WO 97/07668 and WO 97/07669. In brief, a cell, e.g., a somatic cell, from the transgenic animal can be isolated and induced to exit the growth cycle and enter Gₒ phase. The quiescent cell can then be fused, e.g.. through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell, e.g., the somatic cell, is isolated.

In preferred embodiments, the GDF-8 or GDF-11 inhibitor transgenic animals can be selected such that the GDF-8 or GDF-11 function is only partially inhibitted.

This invention is further illustrated by the following examples which should not be construed as limiting. The entire contents of all references, patents and published patent applications cited throughout this application, as well as the Sequence Listing and the Figures are incorporated herein by reference.

### EXAMPLES

### Materials and Methods

The materials and methods used in the following Examples are first described.

### 1. Non-reducing SDS-PAGE and electroblotting

The Novex NuPAGE protocol was used (Novex). The reducing agent from both sample and running buffers was omitted.

### 2. Gel elution

The BioRad mini gel elutor protocol, with the elution buffer (25 mM Tris, pH 8.3 and 6 M urea), was used.

### 3. Acetone precipitation

Removal of excess SDS is necessary in order to successfully perform tryptic digestion. Therefore, ice-cold acetone was added to the SDS-containing sample to obtain final v/v of 20%. The solution was kept below -20°C for 20 minutes. The SDS-free protein was then recovered by centrifugation at maximum speed (13,000x g) for 15 minutes at 4°C. The supernatant was then removed without disturbing the pellet. The solution was not discared until the presence of protein in the pellet has been confirmed by SDS-PAGE. A high percentage of recovery (>90% per run) is usually achieved, but in some cases, the optimal concentration of acetone required for this process can be protein dependent, and careful monitoring should be performed.

### 4. Solublization of SDS-free protein

The SDS-free protein sample was air-dried for a few minutes to evaporate most of the acetone (never letting the pellet dry out). To solublize the sample, either solid urea or a 10M urea solution was added to the wet pellet. Due to the large volume dilution in subsequent tryptic digestions, the amount of volume used for solublization was kept to a minimum (<5µl) in order to avoid losing protein in the sample.

### 5. Tryptic digestion

A sufficient amount of digestion buffer (100 mM ammonium bicarbonate, pH 7.8 to 8.0, no adjustment of pH is needed) was added to the sample (final concentration of urea should be kept below 0.1 M). After adding trypsin (at 1 µg/ml in 1 mM HCl) in a w/w ratio of 1 (enzyme) to 20 (substrate), digestion was initiated by incubating at 37°C. After overnight incubation (12-16 hours), the reaction was quenched by adding PMSF (stock solution prepared in ethanol) to a final concentration of 1 mM.

### 6. High resolution peptide separation

A PMSF-treated tryptic sample solution was divided into two equal parts before it was subjected to HPLC separation using the conditions described below. One part was treated with reducing agent (TCEP/ 50 mM final concentration), and the other part is kept untreated (water is added to adjust volume). The samples were incubated at 60°C for 30 minutes before acidifying with 10% TFA. In the case that an alternative sample treatment is used, the possibility of fragment loss due to aggregation should also be considered. For example, solid guanidine.HCl can be added to the PMSF-treated tryptic sample solution to a final concentration of 4 M. Subsequently, the protocol described above can be used.
Column: C 18 RP column (2.1 x 250mm).
Solvents: A: 0.1% TFA in water, B: 0.1% TFA in 80% acetonitrile.
Flow Rate: 0.2ml/min.
Gradient: O%B to 100%B in 60 to 90 minutes.
Detector: 215nm
Optimization of the gradient is necessary in order to obtain the maximum resolution regarding disulfide containing peaks.

### 7. Secondary digestion

Selection of proteases is highly sequence-dependent. When target sequences are not known, suitable proteases (which are less specific than trypsin) can be used in accordance with methods well known in the art, e.g., thermolysin, chymotrypsin, pepsin, or any other commercially available protease. Since the target substrates of these proteases are quite broad and non-specific, they often can cleave the tryptic fragment and generate smaller fragments. Proteases that are more specific than those mentioned can also be used to generate larger fragments. Secondary digestion is preferably performed using a procedure similar to the trypsin digestion described above, in paragraph 5 except for differences in buffer and in pH levels.

### 8. Deglycosylation of the inhibitor

To solublize the SDS-free sample (5 to 10 µg), a minimum amount of hydrogenated Triton X-100 and 50 mM of ammoniun bicarbonate buffer (pH 7.8 to 8.0) can be added in a small increment of 2 to 3µl at a time. The final volume should be less than 30 µl, and the detergent concentration should be below 0.5% (to prevent denaturation of the enzyme). One unit of peptide N-glycosidase F (PNGase F) is then added to the reaction mixture. The sample is incubated at 37°C for 16 hours before acidifying with 10% TFA. The deglycosylated protein is further purified by C4 RPHPLC, and any inhibitory activity is monitored by an appropriate bioassay. Deglycosylation can also be performed using an N-Glycosidase F Deglycosylation Kit (Boehringer-Mannheim).

### 9. Site-directed mutagenesis and vector construction

The mutated GDF-8 cDNA that encodes a protein with a replaced cleavage site (RSRR) was generated using the overlapping PCR technique. The pair of overlapping inside primers, incorporating the mutation of the cleavage site (RSSR to NAQT), was used to amplify 5' and 3' ends of mouse GDF-8 cDNAs in the first round of PCR. Then, PCR products were combined and used as a template with the outside primers to generate the full-length mutated GDF-8 cDNA, referred to as dominant-negative mutant (Mut-GDF-8). The PCR fragment was ligated into the PCR 2.1 vector (Invitrogen) and sequenced to confirm the incorporation of the mutation. The cDNA encoding Mut -GDF-8 was then inserted into the FLAG-CMV-5a vector (Sigma) to produce an in-frame fusion with the FLAG epitope at the 3' end. The FLAG epitope can be used for detection and purification of the unprocessed GDF-8 protein. This vector contains cytomegalovirus promoter sequences, which result in high level expression in eukaryotic cells.

The expression plasmid containing the pro-domain of GDF-8 was generated through the same method. The partial mouse cDNA, encoding the pro-domain of GDF-8 (residues 1-266), which is referred to herein as "Pro-GDF-8", was generated by PCR-based mutagenesis. The Asp-267 codon (GAC) was changed to a STOP codon (TGA). The resulting cDNA was ligated into the PCR 2.1 vector, sequenced, and inserted into FLAG-CMV-5a as described herein. The wild-type (WT) mouse GDF-8 cDNA was also subcloned in the same vector to generate the full-length precursor protein. The resulting construct is referred to as WTGDF-8.

### 10. Transfection, polyacrylamide gel electrophoresis, and immunoblotting

The expression constructs were introduced into QM-7 quail myoblast cells by transient transfection using FuGene reagent (Boehringer Mannheim). Cells were plated on 60 mm dishes 24 hours before transfection and were 70-80% confluent at the time of transfection. The efficiency of transfection, monitored by a reporter construct containing β-galactosidase, was about 70-80%. Transfected cells were maintained in Opti-MEM (Gibco-Life Sciences), and conditioned media were collected 48 hours after transfection and concentrated in Centricon columns (Amicon) by about 50-fold. Expression of the various forms of recombinant GDF-8 proteins in QM-7 cells was confirmed by SDS-PAGE and immunoblotting with the anti-FLAG M2 specific antibody (Sigma). Proteins reactive with the anti-FLAG antibody were detected by a chemiluminescent technique according to the manufacturer's instructions (ECL, Amersham).

### 11. Protein purification

Affinity chromatography using an affinity gel coupled to a specific monoclonal antibody directed against the FLAG epitope (Sigma) was used to purify the WT-GDF-8-FLAG, the MutGDF-8-FLAG, and the Pro-GDF-8-FLAG from conditioned media of QM-7 cells according to the manufacturer's instructions. The bound proteins were eluted in 0.1 M glycine (pH 3.5) and quantitated in silver-stained SDS-PAGE gels (Novex).

### 12. Metabolic labeling and immunoprecipitation

Transfected QM-7 cells were maintained in Opti-MEM for 48 hours and then labeled for 3 hours in cysteine-free DMEM containing 200 pCi/ml of [³⁵S] cysteine (Amersham). Cells were washed twice and incubated in Opti-MEM for indicated time periods. The conditioned media were then collected, and the cells were lysed in 500 µl of lysis buffer (10 mM Tris-HC 1 [pH 7.5], 150 mM NaCl, 5 mM EDTA, 1% SDS, 0.25% deoxycholate, 0.25% NP-40, protease inhibitors cocktail [Boehringer Mannheim]) and spun for 10 minutes. For immunoprecipitations, either 1 ml of conditioned medium or 250 µl of lysate (diluted 1:2 with 10 mM Tris-HCl [pH 7.5], 150 mM NaCl, 5 mM EDTA), were incubated overnight at 4°C with anti-FLAG M2 affmity gel (Sigma). The immunoprecipitates were resuspended in 2X Laemmli loading buffer containing 2% mercaptoethanol, heated for 10 minutes at 40°C and electrophoresed in 14% SDS-PAGE gels. Gels were permeated with Amplify (Amersham), dried, and exposed to Kodak film at -70°C.

### 13. Identification of GDF-8 -inhibitory activity/Transcription-based assay

This type of bioassay is transcription-based and was performed in two different cell lines. A204 human rhabdomyosarcoma cells were used to monitor the efficacy of the inhibition because they are highly responsive to Myostatin. To further characterize the specificity of the inhibition, both A204 cells and the well-characterized mink lung epithelial cell line CCL-64 were used. The advantages of CCL-64 over A204 are two-fold: first, in contrast to A204 cells, CCL-64 respond to TGF-β, and second, CCL-64 cells have been used for years as a model cell line to elucidate the molecular mechanisms of action of TGF-β superfamily members. The artificial reporter plasmid p(CAGA)₁₂-MLP (described in *Dennler et al.* (1998) *EMBO J.,* 17, 3091-3100) was used in this assay. Luciferase gene transcription and, thus, activity is driven by an artificial minimal promoter which is induced by the members of the TGF-β family. Therefore, luciferase activity in cell lysates correlates linearly with the degree of stimulation of the cells by the applied growth factors.

Both cell types were plated in 48-well plates in their respective media (DMEM for CCL64, McCoy's medium for A204) supplemented with 10% fetal bovine serum, antibiotics and L-Glutamine. Upon reaching 80% confluence, cells were transfected using FuGENE-6 to facilitate plasmid uptake (Boehringer-Mannheim) according to the manufacturer's instructions. Cells were transiently transfected with a cocktail of two plasmids, pSV-β-gal plasmid to monitor transfection efficiency and luciferase reporter plasmid. The reporter plasmid p(CAGA)₁₂-MLP is described in Dennler at al. (1998) *supra.* After overnight incubation with the transfection reagents, cells were washed twice with the appropriate serum-free medium containing 0.1 % BSA. The inhibitory fraction was then added and cells were returned to the incubator for 60 minutes. At the end of this period, the following factors were added to the cells: recombinant human Myostatin produced from Myostatin-expressing CHO cell conditioned medium, human TGF-β, and concentrated conditioned medium from CHO cells expressing activin, and βA. Cells were lysed after a 6 hour incubation and luciferase and β-galactosidase activities were determined in the same samples using the Dual-Light Luciferase Assay kit (Tropix, Inc.). Activity is expressed in Relative Luciferase units (RLU), i.e. luciferase activity corrected for transfection efficiency that is given by the corresponding values of β-galactosidase activity measured in the same sample.

To chemically reduce the inhibitor sample, it was diluted by adding an excess of 50 mM Na₂PO₄, pH 7.0, urea, and tris(2-carboxyethyl)phosphine hydrochloride (TCEP) were added to the solution at final concentrations of 6 M and 10 mM, respectively, and the solution was incubated at 37°C for 30 minutes. To quench the reduction, iodoacetamide was added to a final concentration of 10 mM, and the solution was incubated at room temperature for 30 minutes. The sample was then acidified with 10 % TFA and the buffer exchanged to 0.1 % TFA.

### EXAMPLE 1: Production and Purification of Fractions A and B

Fractions A and B were derived separately, each from an independent batch of CHO-conditioned medium, using identical purification protocols. The conditioned media were collected from CHO cells stably transfected with the expression plasmid G8P-1/0.1, which contains an insert encoding human GDF-8. CHO cells were cultured in alpha medium supplemented with 0.1 mM methotrexate and I mg/ml G418 (Geneticin, GIBCO-Life Sciences).

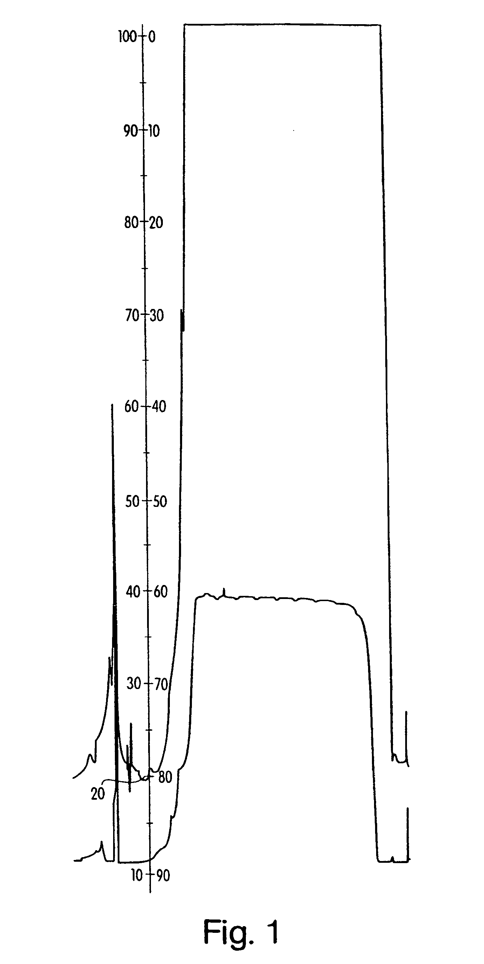

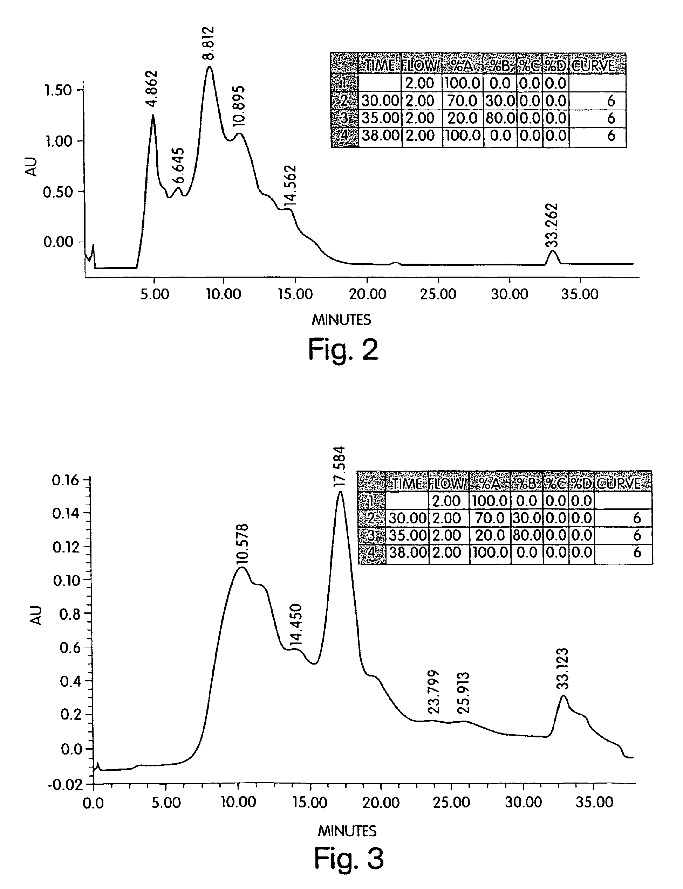

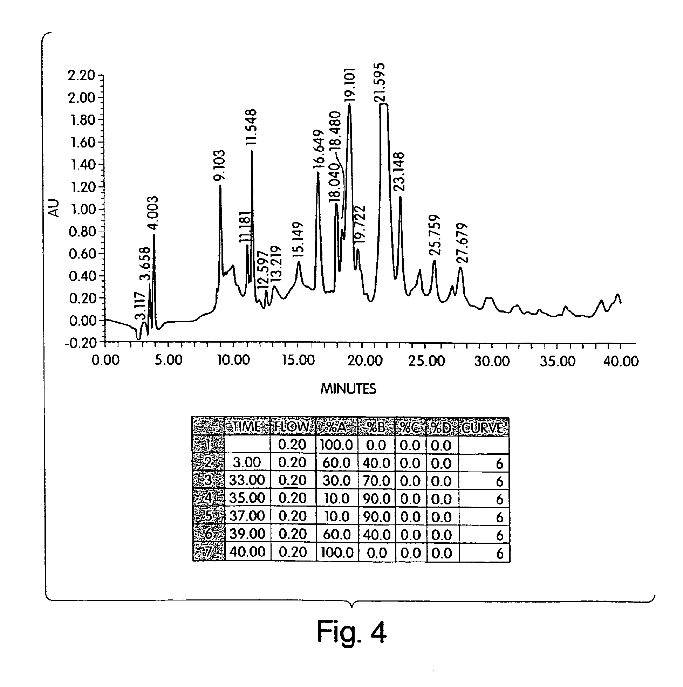

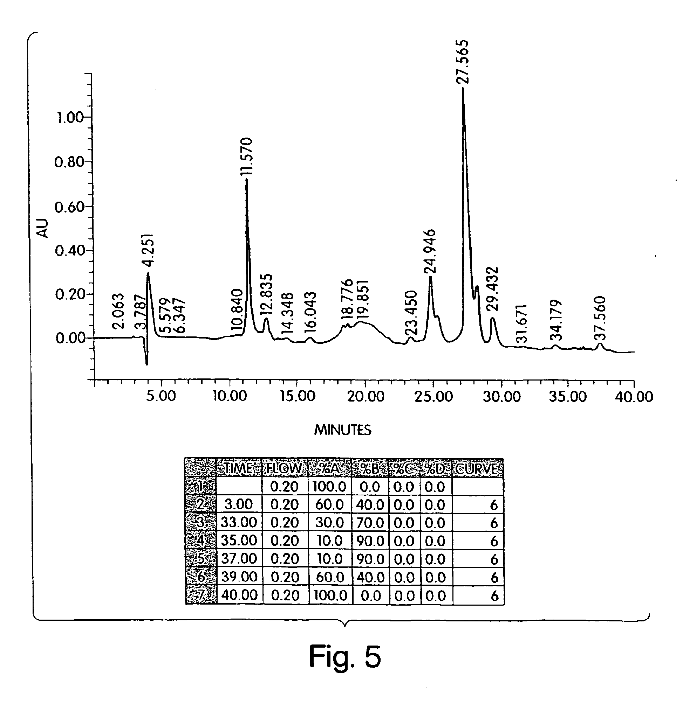

To obtain Fractions A and B, CHO-conditioned media were passed through three different HPLC columns. The first column was an ion exchange column (HQ from Pharmacia) where a fraction of the media components was associated with the flow-through. *Figure* 1 shows a representative chromatogram measured at 230 nm. The broad peak represents the HQ flow-through and the sharp peak represents the bound material. Subsequently, the pH of the HQ flow-through was adjusted to pH 5.0 with 6 N HCl, which allowed certain components of the HQ flow-through material to bind to an SP ion exchange column (Pharmacia). Using a shallow gradient, SP-bound material that was eluted between 6-9 minutes was collected to obtain Fraction A from one batch of CHO conditioned media and SP-bound material that was eluted between 14-19 minutes was collected to obtain Fraction B from another batch of CHO conditioned media. The gradients and the chromatograms measured at 215 nm for Fractions A and B are depicted in *Figures* 2 and 3, respectively. The SP material that eluted at the above times was desalted using 20 mM NaPhosphate, pH 5.0. Each SP material was then injected into a C4 column using a shallow acetonitrile gradient. The C4 fractions were collected and the acetonitrile was removed using a speed vac. The gradient and the chromatogram measured at 215 nm for the C4 runs that produced Fractions A (eluted between 21-23 minutes) and B (eluted at 27 minutes) are depicted in *Figures* 4 and 5, respectively. C4 fractions were stored in 0.1 % trifluoroacetic acid (TFA) and were tested for GDF-8-inhibitory activity as described below.

To denature samples, urea to a final concentration of 6 M was added, and the sample was incubated at 70°C for 10 minutes, followed by chilling on ice. To reduce Fractions A and B, the TFA in fractions A and B was diluted by adding an excess of 50 mM NaPhosphate, pH 7.0. Urea and tris(2-carboxyethyl)phosphine hydrochloride (TCEP) were added to the solution at a final concentration of 2 M and 10 mM, respectively. The solution was incubated at 37°C for 30 minutes. To quench the reduction, iodoacetamide was added to a final concentration of 10 mM and the solution was incubated at room temperature for 30 minutes. The sample was then acidified with 10 % TFA and the buffer exchanged to 0.1 % TFA. Samples from a separate, blank C4 run (no input protein) that eluted at 22.5 or 27 minutes were collected and treated in parallel with Fractions A and B, to be used as appropriate buffer controls in the bioassays.

### EXAMPLE 2: Identification of GDF-8-inhibitory Activity in Fractions A and B

Two independent types of bioassays were developed to test for the inhibitory potential of the C4 fractions obtained from original CHO-conditioned media.

### DNA replication assay

The first bioassay was a growth assay performed in G8 mouse skeletal myoblasts and in the mink lung epithelial cell line, CCL-64. In this type of assay DNA replication and thus proliferation is accurately measured by the incorporation of Bromo-deoxyUridine (BrdU) or tritiated thymidine label into the DNA.

Briefly, G8 myoblasts were plated in 96-well culture plates coated with 0.1% gelatin (Sigma), while CCL-64 were plated in uncoated plates. Both cell types were plated in serum-free DMEM containing 0.1% w/v BSA at 10x10³ cells per well. The following day, media were removed and replaced with fresh media. The relevant inhibitory fraction was added, and cells were returned to the incubator for 60 minutes before growth factors were applied to them. BrdU label at 1:1,000 final dilution (Boehringer-Manheim) was added to G8 myoblasts 24 hours after addition of the test factors, and incorporation was allowed to proceed for an additional 24 hours. G8 myoblasts were then fixed and processed according to the manufacturer's instructions manual using a commercially available kit (Boehringer-Manheim). The incorporated BrdU was determined colorimetrically. [³H]-thymidine (NEN-Dupont) was added to CCL-64 epithelial cells 12 hours after addition of test factors, for a total period of 4 hours. CCL-64 cells were washed twice with 10% trichloroacetic acid and radioactivity was extracted with 0.3N NaOH and determined by scintillation counting.

### Transcription-based Assay

The second type of bioassay was transcription-based and was performed in two different cell lines. A204 human rhabdomyosarcoma cells were used to monitor the efficacy of the inhibition, because they are particularly responsive to GDF-8. To further characterize the specificity of the inhibition, both A204 cells and the well-characterized mink lung epithelial cell line CCL-64 were used. The advantages of CCL-64 over A204 are two-fold: first, in contrast to A204 cells, CCL-64 respond to TGF-β, and second, CCL-64 cells have been used for years as a model cell line to elucidate the molecular mechanisms of action of TGF-β superfamily members. Two artificial reporter plasmids were used in this assay: p3TP-Lux (Wrana et al., (1992) *Cell*, 71, 1003-14) and p(CAGA)₁₂-MLP (Dennler et al., (1998) *EMBO J,* 17, 3091-3100). In both, luciferase gene transcription (and thus activity) is driven by artificial minimal promoters which respond to members of TGF-β family members. Therefore, luciferase activity in cell lysates correlates linearly with the degree of stimulation of the cells by the applied growth factors.

Briefly, both cell types were plated in 48-well plates in their respective media (DMEM for CCL-64, McCoy's medium for A204) supplemented with 10% fetal bovine serum, antibiotics and L-Glutamine. Upon reaching 80% confluence, cells were transfected using FuGENE-6 to facilitate plasmid uptake (Boehringer-Mannheim), according to the manufacturer's instructions. Cells were transiently transfected with a cocktail of two plasmids; pSV-β-gal plasmid (Promega) to monitor transfection efficiency, and either of the two luciferase reporter plasmids. The first reporter, p3TP-lux, is described in Wrana et al., 1992, *supra.* The second, p(CAGA)₁₂-MLP, is described in detail in Dennler at al., 1998, *supra.* After overnight incubation with the transfection reagents, cells were washed twice with the appropriate serum-free medium containing 0.1 % BSA. The inhibitory fraction was then added and cells were returned to the incubator for 60 minutes. At the end of this period, the following factors were added to the cells: human recombinant GDF-8-expressing CHO cell conditioned medium, human TGF-β₁ (R&D Biosystems, Minneapolis) and concentrated conditioned medium from CHO cells expressing Activin βA (Genetics Institute, Cambridge, MA). Medium from mock-transfected cells was devoid of activity.

Cells were lysed after a 6 hour incubation and luciferase and β-galactosidase activity were determined in the same sample using the Dual-Light Luciferase Assay kit from Tropix Inc. Activity is expressed in Relative Luciferase Units (RLU), i.e. luciferase activity corrected for transfection efficiency that is given by the corresponding values of β-galactosidase activity measured in the same sample.

### Inhibitory Effects of Fraction A (C4 column fraction eluted at 22.5 min)

### I. Effect on proliferation of G8 myoblasts and of CCL-64 mink lung epithelial cells

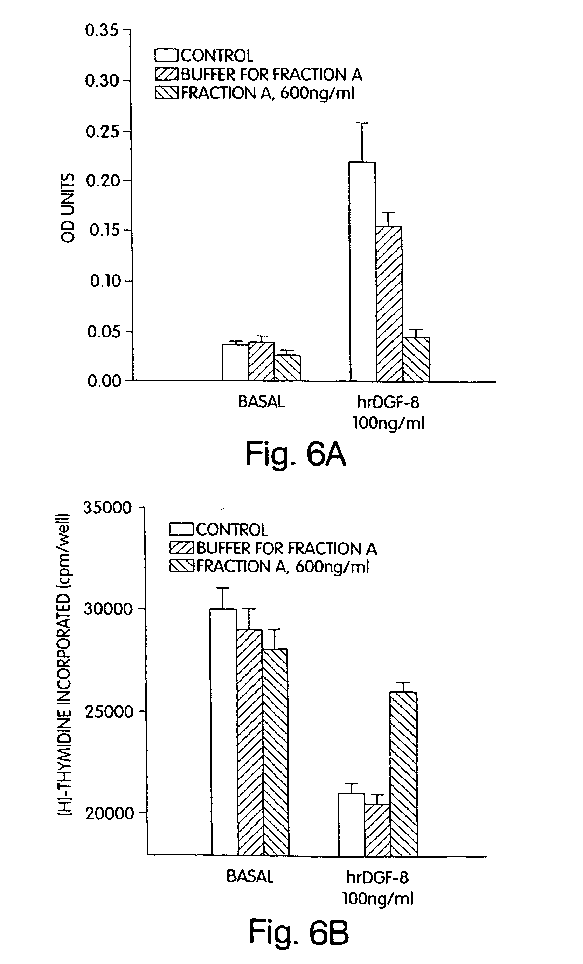

GDF-8 by itself at 100 ng/ml increased G8 myoblast proliferation 6-fold, as measured by increased incorporation of BrdU label in DNA *(Figure* 6A). The buffer vehicle used to resuspend fractions derived from the C4 column was devoid of activity on its own in this assay, and failed to substantially influence the effect of 100ng/ml of GDF-8 *(Figure* 6A).

Of the various fractions that were obtained from the C4 column, a bioactive peak that eluted at 22.5 minutes was identified, called Fraction A. Fraction A by itself had no effect on G8 myoblast proliferation. However, it was able to almost abolish the positive effect of GDF-8 on cell proliferation *(Figure* 6A). Similar GDF-8-inhibitory effects of Fraction A were observed when tritiated thymidine was used instead of BrdU as label, and when the effects of GDF-8 and Fraction A were tested in another cell type, CCL-64 cells *(Figure* 6B). In contrast to its effect on myogenic cell lines, GDF-8 inhibits cell proliferation in the CCL-64 epithelial cell line, an effect that is identical to that of TGF-β. This growth inhibition of GDF-8 was reversed when CCL-64 cells were pretreated for 60 minutes with an excess weight/weight Fraction A prior to addition of GDF-8 *(Figure* 6B). In sum, regardless of the cell line, the type (positive or negative) proliferative effect of GDF-8 and the method of measuring cell proliferation, Fraction A consistently displayed clear antagonistic activity versus GDF-8.

### II. Effects on transcription from minimal promoters/Sensitivity to denaturing and reduction/Specificity of Action

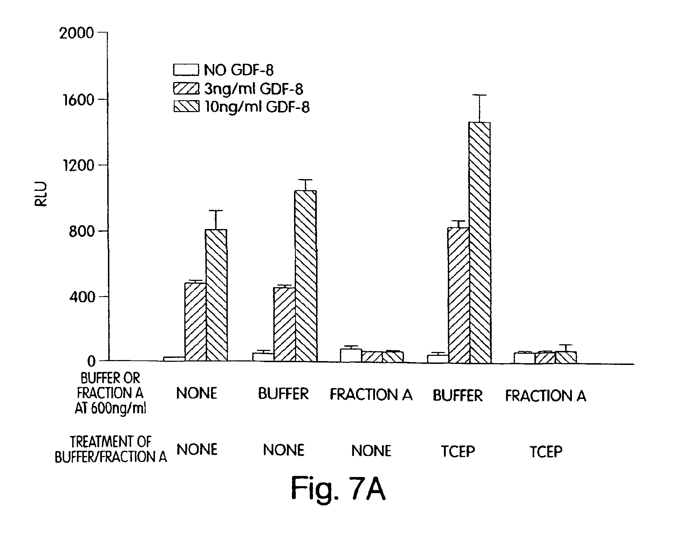

As shown seen in Figure 7A, 1-hour pre-incubation with Fraction A abolished the effect of 3 or 10 ng/ml of GDF-8 on p(CAGA)₁₂-MLP luciferase gene transcription in A204 rhabdomyosarcoma cells. This effect was also observed using the second, independent reporter plasmid p3TP-Lux in the same cells.

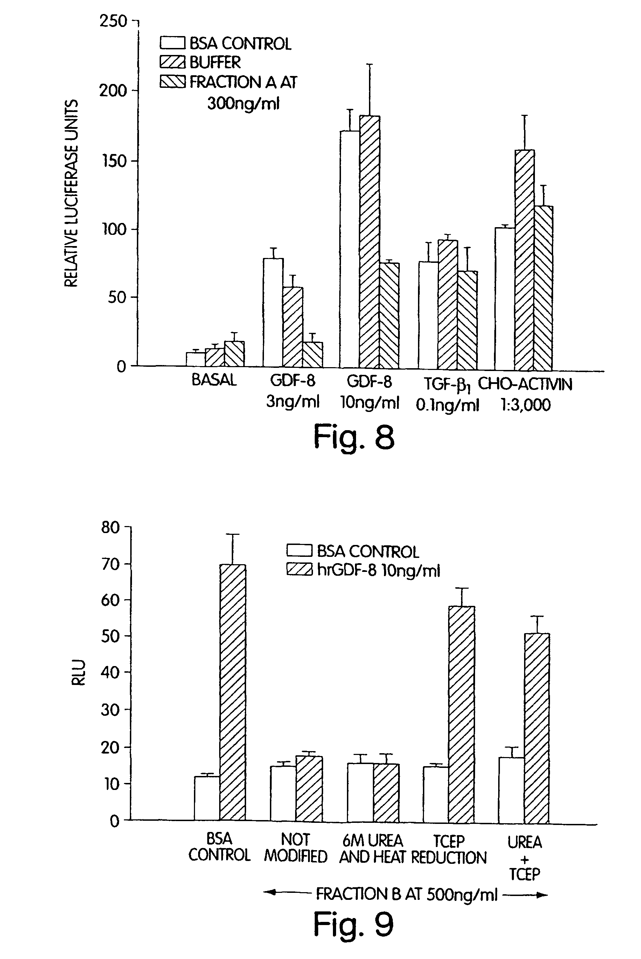

Reduction of Fraction A using two different reducing agents, DTT and TCEP/iodoacetamide did not affect the inhibitory potential of Fraction A, regardless of the reporter plasmid used, indicating that the active moiety within this fraction is not sensitive to strong reducing agents *(Figure* 7A). The fact that these conditions are indeed capable of effecting reduction was supported independently by showing, for example, that they can abolish the activity of another reduction-sensitive fraction, Fraction B (see Figure 9).

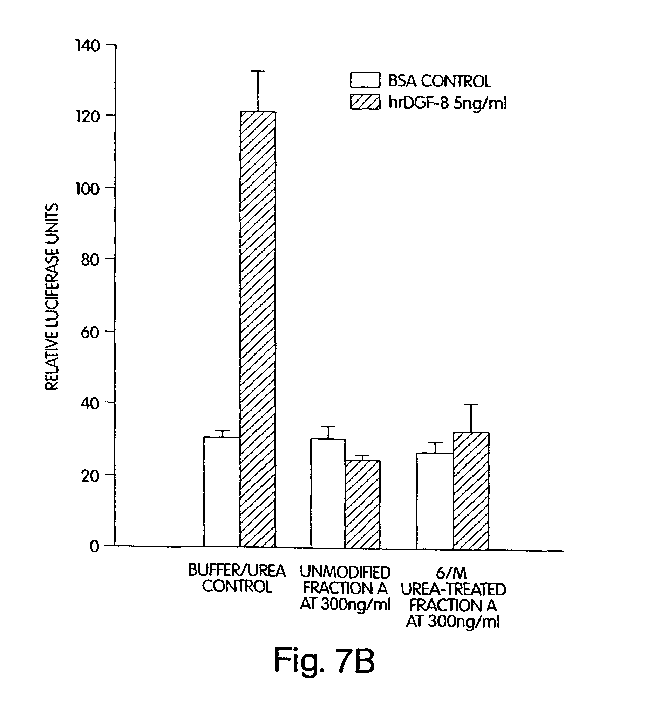

Denaturing of Fraction A by either heat (100°C for 10 minutes, then quick chilling on ice) or by 6M urea and heat did not affect its inhibitory activity either (*Figure* 7B)*.*

The issue of specificity was addressed in CCL-64 cells using the p(CAGA)₁₂₋MLP reporter. The effects of Fraction A on three different members of the TGF-β family: TGF-β itself, GDF-8, and Activin, were compared.

As shown in *Figure* 8, Fraction A (or its vehicle) did not modulate basal (background) luciferase activity by itself, nor did it appreciably alter the effect of TGF-β or Activin. In contrast, it still showed strong inhibitory activity against GDF-8. This piece of evidence indicates that Fraction A can clearly discriminate between structurally related factors that share a significant degree of homology (~40% at the amino acid level).

### Inhibitory Effects of fraction B (C4 column fraction eluted at 27 min)

### I. Effects of Fraction B on transcription from minimal promoters/Sensitivity to denaturing and reduction/Specificity of Action

A second, independently-derived inhibitory fraction was also identified from the processing of a separate batch of CHO-conditioned media. This novel inhibitor fraction had a retention time of 27 minutes in a C4 column. This fraction was tested in the transcription-based bioassay, under the same conditions as Fraction A. Similar to Fraction A, Fraction B was able to functionally inhibit the effect of GDF-8 on luciferase activity in A204 rhabdomyosarcoma cells (*Figure* 9).

Further analysis indicated that, similar to the activity in Fraction A, the inhibitory activity within Fraction B was insensitive to strong denaturing conditions (6M urea plus heat). In contrast to Fraction A however, TCEP reduction of the Fraction B sample abolished its inhibitory activity (*Figure* 9).

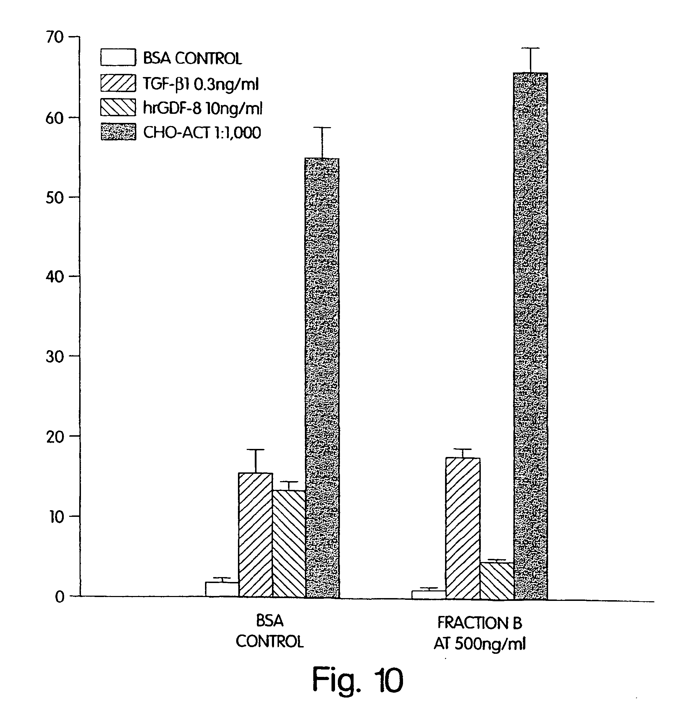

To test the specificity of this novel C4 fraction, an additional series of experiments was performed in CCL-64 cells using the p(CAGA)₁₂-MLP reporter. The results obtained indicate that Fraction B resembles Fraction A in its ability to specifically inhibit the action of GDF-8, while leaving intact the activity of TGF-β or Activin *(Figure* 10).

### EXAMPLE 3: Biochemical Characterization of fractions A and B

As part of an effort to better understand the composition of Fractions A and B, a silver stain was run with Fractions A and B under both reduced and non-reduced conditions. The methods used for reducing Fraction A are described above. Fraction A in solution comprises several components whether it is in a reduced or non-reduced state, ranging in weight from very low (co-migrating with the dye front) to 200 kDa. However, when the sample is reduced new bands appear at lower molecular weight and some of the higher molecular weight bands disappear.

Fraction B was reduced using the methodology and reducing agents supplied by Novex. The results reveal that there are two major species and several minor species in the non-reduced state of Fraction B. In the reduced state, a new band around 12 kDa is present giving a total of 3 major components with several minor components present as well.

### EXAMPLE 4: Identification and Characterization of the GDF-8 Inhibitor in Fractions A and B

A four-step strategy to identify and characterize the inhibitor in Fractions A and B can be performed as follows:

### I. Identification of the components of Fractions A and B that inhibit GDF-8 activity

Several distinct species are present in Fractions A and B. Therefore, it is necessary to further process these Fractions into their individual components in order to unambiguously identify the active moiety or moieties (e.g. the component(s) which inhibit GDF-8). This process can be done, as described in Example 5 below, by performing a preparative non-reducing SDS-PAGE, and by subsequently recovering the separated components by electroelution. Components possessing GDF-8 inhibitory activity can then be identified using bioassays described herein.

After successfully identifying one or more GDF-8 inhibitory components, the specificity of these components can be further studied, as described below in Example 5. For example, several molecules that possess structural and biological activity similar to GDF-8 included in this study, such as Activin, BMPs, and TGF-β can be used to test for GDF-8-specific inhibitory activity. The purified GDF-8 inhibitor can be tested for the ability to antagonize GDF-8 effects in a dose-dependent and GDF-8-specific fashion.

### II. Characterizing GDF-8 Inhibitors

As described in Example 5 below, one or more of a combination of N-terminal sequencing and mass spectrometry analyses can be used to characterize the inhibitor. In N-terminal sequencing analysis, at least 10 to 15 µg of either Fraction can be loaded onto non-reducing SDS-PAGE. Proteins are then separated and electroblotted onto a PVDF membrane. After staining with Coomassie blue, the protein band corresponding to the inhibitor is cut out for sequencing analysis. The number of residues observed at each sequencing cycle correspond directly with the number of different components in the sample, and the ratio of intensity among the various signals at each cycle is used to estimate the proportion of each different component.

The precise molecular weights of the inhibitor and its components can be determined by electrospray/ionization mass spectrometry (ESI/MS) in the absence and presence of a reducing agent. The mass spectrometry information, combined with the expected molecular weights calculated from the known primary sequences, can be used for two purposes: first, to either confirm or refine the composition of the inhibitor through N-terminal sequencing analysis, and second, to evaluate the integrity of the inhibitor regarding potential C-terminal truncation, internal cleavage, or post-translational modification, in which N-terminal sequencing analysis is unable to provide the answer.

### III. Characterizing Disulfide Patterns of GDF-8 Inhibitors

Experiments which characterize disulfide patterns are particularly relevant for the characterization of fractions, such as Fraction B, having inhibitory activity which is sensitive to reduction and which, therefore, contain disulfide bonds. Successful characterization of disulfide bonds is dependent on the consistency of both sample preparation and high resolution peptide mapping. The disulfide-containing fragments are identified by comparing HPLC chromatograms between Tris(2-carboxyethyl) phosphine (TCEP)-treated (reduced) and non TCEP-treated samples.

Excepting the early region (non-retentate) of the chromatogram, the disappearance of a peak in the chromatogram of the TCEP-treated sample suggests a disulfide-containing fragment. The corresponding fraction from a non TCEP-treated sample is analyzed both by N-terminal sequencing and by matrix-assisted laser desorption/ionization and time-of-flight mass spectrometry (MALDI-TOF/MS) analyses. In the case where a single disulfide bond is present, two amino acid residues are evidenced with comparable signal intensity, for each sequencing cycle.

The preliminary characterization of the inhibitory Fractions A and B, as well as their presence in the conditioned media of GDF-8-expressing CHO cells, is compatible with the notion that they are GDF-8-related (e.g. contain peptides of GDF-8 which inhibit GDF-8 activity). If this is supported by the sequencing efforts described above, then by comparison with the known primary sequence of GDF-8, the identity of disulfide-linked peptides, and more importantly, the disulfide position within the GDF-8 sequence is easily determined. The MALDI-TOF/MS analysis is used primarily for the purpose of confirmation.

In the case of either multiple sequences or multiple disulfide bonds being involved, a secondary digestion by a protease (the selection of the protease being completely sequence-dependent) is needed to generate analyzable fragments. Another run of peptide mapping can then be performed as described in the materials and methods section above.

### IV. Identification of Post-translational Modifications GDF-8 Inhibitors

One of the most common forms of mammalian protein modification is glycosylation, which in some instances can substantially affect the bioactivity of the protein. Therefore, it is important to investigate whether glycosylation of GDF-8 inhibitory peptides modulates the peptide's inhibitory activity.

To do so, the inhibitory species can be deglycosylated as described in the materials and methods section above and as illustrated in the following Example. The correlation between structure (glycosylation) and functional activity (inhibition) can be either confirmed or ruled out by the bioassays for GDF-8 inhibition described herein.

### EXAMPLE 5: Identification and Characterization of the GDF-8 Inhibitor in Fraction B

The individual component in Fraction B responsible for the inhibition of GDF-8 was determined by running a preparative non-reducing SDS-Page gel and separating the components by electroelution. It was determined that a peptide having a molecular weight of approximately 36 kDa is responsible for the inhibition of GDF-8. The specificity of the GDF-8 inhibition was also shown as described below.

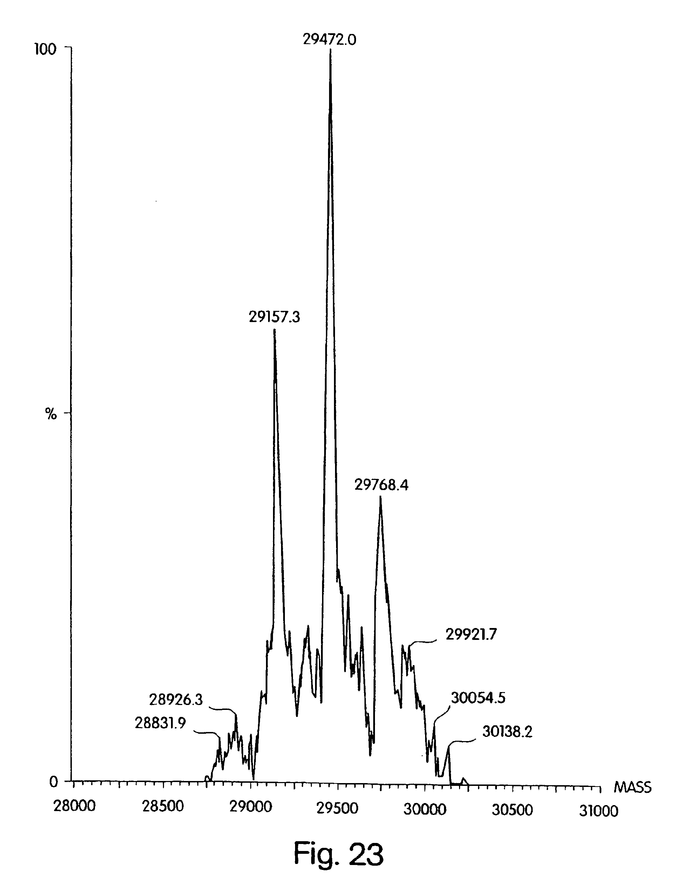

The first step in the characterization of the inhibitor was the determination of the precise molecular weight by electrospray/ionization mass spectrometry. The mass spec spectrum showed three peaks separated by 600 Da with the major component of molecular mass of 29,472.0 Da (M-Scan, Inc.) *(Figure* 23). The difference in the molecular weights from mass spec and the SDS-Page could have been due to the gel and the molecular weight marker.

The next step was to determine the amino acid sequence of the inhibitor by pulsed phase N-terminal sequencing with five cycles (determines the first five amino acids). Table I shows the major and minor amino acids detected with the major amino acids being greater than 90% present. The sequence (NENSE) (SEQ ID NO:26) corresponds to a sequence found in the pro-domain of GDF-8.

**TABLE I**

| Cycle No. | Major PTH-AA Detected | Minor PTH-AA Detected |
|---|---|---|
| 1 | Asn | --------- |
| 2 | Glu | Pro, Ala, Phe |
| 3 | Asn | Gln, Val, Lys |
| 4 | Ser | Asp,Lys |
| 5 | Glu | Leu |

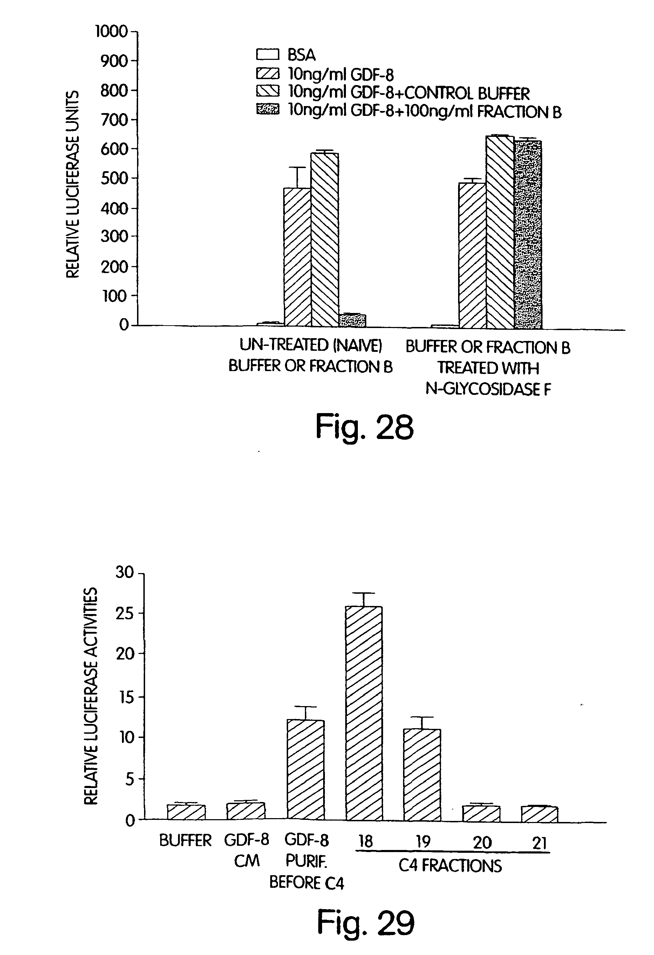

The next step was to determine whether the inhibitor from Fraction B (i.e., the pro-domain of GDF-8) referred to as "pro-GDF-8" is effected by post-translational modification. The most common form of modification in mammalian proteins is glycosylation. In the pro-domain of GDF-8 there is only one asparagine for glycosylation. To determine if glycosylation of the asparagine is needed to maintain the inhibitory activity the GDF-8 pro-domain, the pro-GDF-8 inhibitor from Fraction B was deglycosylated using an N-Glycosidase F Deglycosylation kit (Boehringer Mannheim). The enzyme, denaturing reagents and salts were removed by passing the deglycosylated protein through a C4 column. A transcription-based bioassay (using the (CAGA)₁₂-MLP construct) was used to determine the inhibitory activity of the glycosylated and deglycosylated protein. The results showed that the GDF-8 pro-domain must be glycosylated in order to inhibit GDF-8. (Figure 28)

Overall, the foregoing studies demonstrated that the GDF-8 peptide inhibitor isolate from Fraction B is the entire pro-domain of GDF-8 in glycosylated form, and that glycosylation (e.g., production of the peptide in cells that can glycosylate the peptide) is necessary for inhibitory activity.

### Characterization of the Fraction A Inhibitor Using a DNA Replication Assay

As shown in *Figure* 6A, GDF-8 itself at 100 ng/ml increases G8 myoblast proliferation 6-fold, as measured by increased incorporation of BrdU label in DNA. The buffer vehicle used to resuspend pro-GDF-8 was devoid of activity on its own in this assay, and failed to substantially influence the effect of 100 ng/ml of GDF-8. However, the pro-GDF-8 was able to almost abolish the positive effect of GDF-8 on cell proliferation.

Similar GDF-8-inhibitory effects of the pro-GDF-8 were observed when tritiated thymidine was used instead of BrdU as label, and the assay was performed in another cell type, CCL-64 cells *(Figure* 6B). In contrast to its effect on myogenic cell lines, GDF-8 inhibits cell proliferation in the CCL-64 epithelial cell line, an effect that is identical to that of TGF-β. This growth inhibition of GDF-8 was reversed when CCL-64 cells were pretreated for 60 minutes with a 6-fold excess weight/weight of pro-GDF-8 prior to addition of the mature GDF-8 protein *(Figure* 6B).

In conclusion, regardless of the cell line, the type (positive or negative) effect of GDF-8 on proliferation, and the label used to measure DNA replication, this modified growth assay accurately reflects the activity of GDF-8 and, can be used to determine the GDF-8-inhibitory potential of GDF-8 antagonists, such as pro-GDF-8.

### Characterization of the GDF-8 Inhibitor (Fraction B) Using a Transcription-based Assay

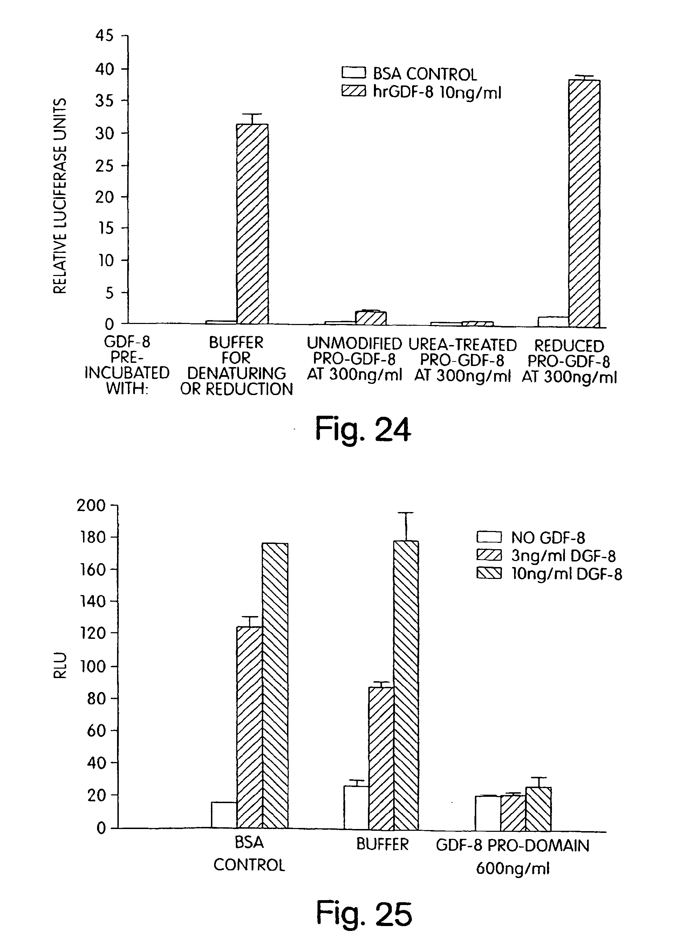

As shown in *Figure* 7A, reporter gene construct p(CAGA)₁₂-MLP respond in a dose-dependent fashion to GDF-8, with substantial induction detected as early as 6 hours after application of the growth factor. However, 1-hour pre-treatment of cells with the pro-domain of GDF-8 abolished the effect of 3 or 10 ng/ml of mature GDF-8 on p(CAGA)₁₂-MLP luciferase gene transcription in A204 rhabdomyosarcoma cells *(Figure* 25). This effect was also observed using the second, independent reporter plasmid p3TP-Lux in the same cells.

This type of transcription-based assay is more sensitive to the effect of GDF-8 (compare GDF-8 concentrations in *Figures* 7A and 7B to those in *Figures* 6A and 6B). This type of assay also determines specificity of inhibition, for example, in CCL-64 cells using the p(CAGA)₁₂-MLP reporter. Specifically, the effects of pro-GDF-8 on three different members of the TGF-β family: TGF-β itself, GDF-8 and Activin, were compared. As seen in *Figure* 8, pro-GDF-8 (or its vehicle) did not modulate basal (background) luciferase activity by itself, nor did it appreciably alter the effect of TGF-β or Activin. In contrast, it still showed strong inhibitory activity against GDF-8.

In conclusion, the above-described transcription based bioassay is specifically optimized to allow for sensitive monitoring of GDF-8 activity and for determining the inhibitory potential of putative GDF-8 antagonists, regardless of their mechanism of action (e.g., whether they act by binding and inactivating GDF-8 or by blocking the cognate receptors).

### Characterization of the GDF-8 Inhibitor Using a Creatine Kinase Assay

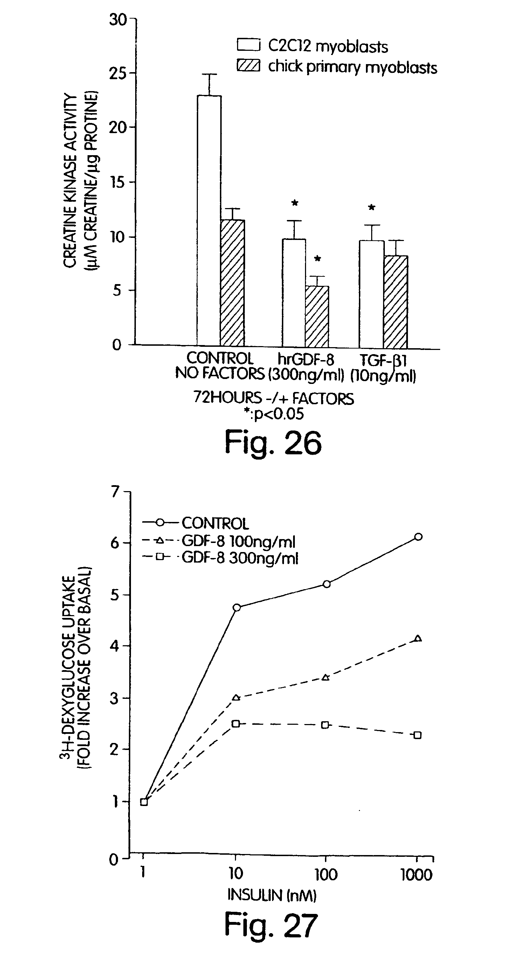

As shown in *Figure* 26, treatment of chick primary myoblasts or C2C12 myoblasts with GDF-8 for 72 hours at the onset of differentiation, reduced the activity of creatine kinase in cell lysates. This was also evident by comparison of cell morphology in the presence and absence of GDF-8. In the latter case, a lot fewer tubes had formed in the dish. TGF-β₁ had a similar effect to that of GDF-8 in C2C12 cells, but not in chick primary myoblasts (*Figure* 26). Therefore, the creatine kinase assay can be used to identify and characterize GDF-8 inhibitors, such as pro-GDF-8, by determining their ability to inhibit the reduction in creatine kinase activity by GDF-8.

### Characterization of the GDF-8 Inhibitor Usin an Assay Based on the Differentiation of 3T3-L1 Pre-adipocytes

Following a specific protocol, 3T3-L I pre-adipocytes can differentiate into adipocytes, displaying the full range of molecular markers and morphological characteristics typical of adipocytes in vivo. As the results of this experiment demonstrate, treatment of 3T3-L1 cells with GDF-8 at the onset of differentiation process resulted in a severe blockade of differentiation. This was assessed using two independent criteria. The effect of GDF-8 is evident by the near-absence of refractile cells that contain lipid droplets. At the mRNA level, the expression of the adipocyte-specific gene GLUT4 is blocked in GDF-8-treated cells. Two cytokines that have previously been implicated in the control of adipocyte metabolism, namely TNF-α and TGF-β₁, can mimick the effect of GDF-8. In addition, the degree of differentiation can be addressed by a variety of immunological, biochemical and molecular biological methods, as mentioned in the Materials and Methods section. Changes in adipocity in mouse and cattle that lack functional GDF-8 have been described. Therefore, this assay provides a physiologically important measure of GDF-8 activity *in vivo.* Accordingly, the effect of GDF-8 inhibitors on GDF-8 blockade of adipocyte differentiation can be used to evaluate such inhibitors.

### Characterization of the GDF-8 Inhibitor Using a Glucose uptake assay in 3T3-L1 Adipocytes

In differentiated adipocytes, insulin stimulates glucose transport through the Glut-4 transporter in a dose-dependent fashion *(Figure* 27). This effect can be blocked in a dose-dependent manner by treatment of the cells for 72 hours with GDF-8 (*Figure* 27). Importantly, this assay offers an *in vitro* correlate of GDF-8 activity of *in vivo* body fat metabolic functions.

### EXAMPLE 6: Generation of GDF-8 Inhibitors Derived From the GDF-8 Pro-domain

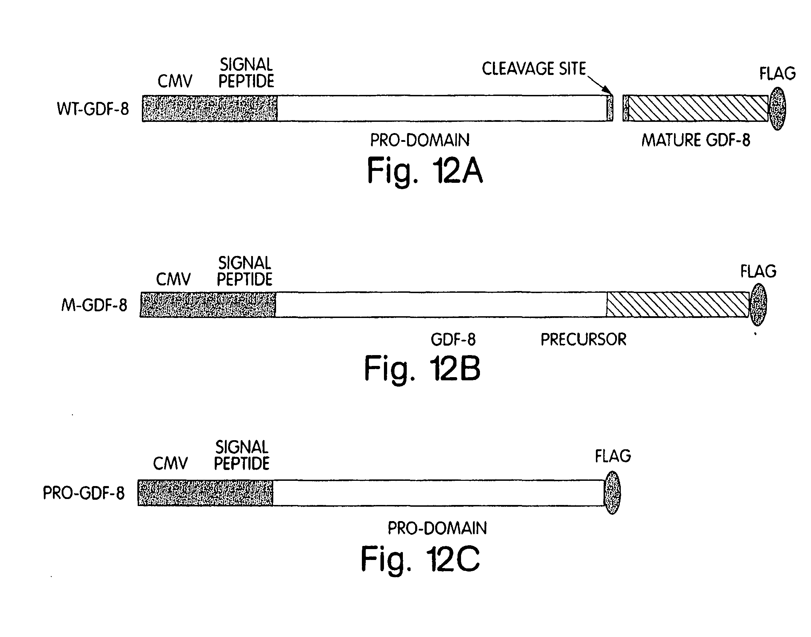

To produce GDF-8 inhibitors comprising all or a portion of the pro-domain of the GDF-8 protein, a partial mouse cDNA encoding the pro-domain (residues 1-266 shown in *Figure 13*) of GDF-8 (also referred to as "pro-GDF-8"), was generated by PCR-based mutagenesis. The PCR fragment was subcloned in the TA vector (Invitrogen), and sequenced to confirm the incorporation of the mutation. The cDNA encoding pro-GDF-8 was inserted in the FLAG-CMV-5a vector (Sigma) to produce an in-frame fusion with the FLAG epitope at the 3' end, used for detection and purification of the pro-GDF-8 protein (*Figure 12*C)*.* This vector contains cytomegalovirus promoter sequences, which result in high-level expression in eucaryotic cells. The construct expressing the wild type GDF-8 (WT-GDF-8) was generated using the same vector *(Figure 12*A)*.*

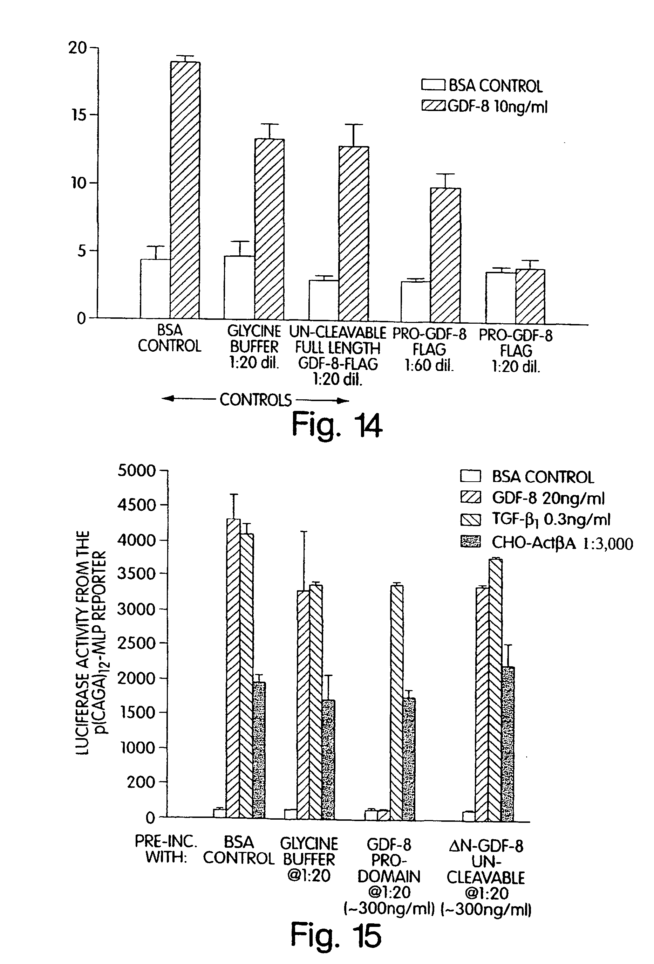

An un-cleavable full-length GDF-8 mutant was expressed using the same method, by replacing the predicted cleavage site at the boundaries between the pro-domain and the mature protein (*Figures* 13B *and* 14).

The resulting three expression constructs were introduced in QM-7 quail myoblast cells by transient transfection using FuGENE reagent (Boehringer Mannheim). The efficiency of transfection, monitored by a reporter construct containing β-gal, was about 70-80%. Affinity chromatography using an affinity gel coupled to a specific monoclonal antibody directed against the FLAG epitope (Sigma) was used to purify the recombinant proteins, that is the Mut-GDF-FLAG, and the pro-GDF-8-FLAG from conditioned medium of transfected QM-7 cells. The bound proteins were eluted in 0.1 glycine (pH 3.5), and quantitated by SDS-PAGE, followed by Silver Staining (Novex). The estimated concentration of the purified pro-GDF-8 was about 20 ng/ml.

The eluted proteins were also analyzed by immunoblot with the AntiFlag antibody. The Mut-GDF-8 and pro-GDF-8 proteins were properly recovered from the column, and the immunoreative proteins of the expected molecular weights were present in the recovered fractions, to be used in the transcription-based bioassay.

To test whether the pro-region of GDF-8 (pro-GDF-8) can interfere with the effects of mature GDF-8, column-purified pro-GDF-8 was pre-incubated with a specific amount of human recombinant GDF-8 for 30 minutes at room temperature. In parallel, comparable amounts of BSA, glycine buffer, or full-length uncleavable-GDF-8 (Mut-GDF-8) were added to separate tubes containing GDF-8, to be used as controls. Two different dilutions of pro-GDF-8 were chosen, to achieve 30-fold and 10-fold weight/weight excess compared to GDF-8. Following pre-incubation, the mixture was transferred onto A204 cells, transfected with either p(CAGA)₁₂-MLP or p3TP-Lux reporter plasmids mixed with pSV-β-gal plasmid. Six hours later cells were lysed and luciferase and β-galactosidase activity were determined in the lysates. The final concentrations of the buffers and column-purified proteins in each well were 10 ng/ml for GDF-8 and 300 ng/ml and 1,000 ng/ml for pro-GDF-8 and 1,000 ng/ml for the uncleavable full-length GDF-8.

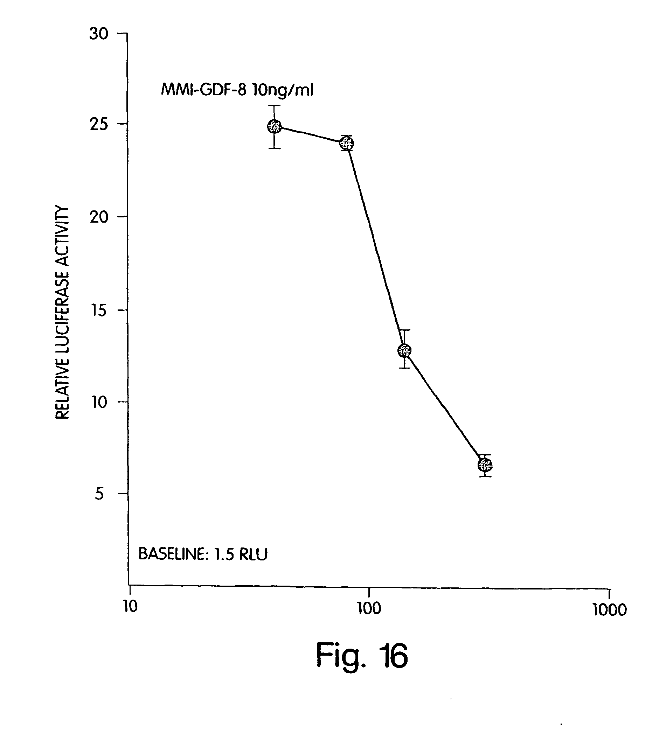

As seen in *Figure* 16 the column-purified pro domain of GDF-8 was able to totally suppress the induction of luciferase activity by GDF-8. This induction was not influenced when prior to its addition to the cells GDF-8 had been pre-incubated with either BSA, glycine buffer (used for column elution), or full length, uncleavable GDF-8 protein (Mut-GDF-8). This effect was dose-dependent *(Figure 16)* and specific for GDF-8, since pro-GDF-8 did not affect the induction of luciferase activity by TGF-β1 or by activin *(Figure 15).*

Similar to fractions A and B, the effect of denaturing and reduction on the inhibitory activity of pro-GDF-8 was examined. As seen in *Figure 24,* pro-GDF-8 retained its inhibitory activity after denaturing with 6M urea. However, reduction of pro-GDF-8 resulted in the complete loss of inhibition.

To study the effect of the pro-domain on secretion of wild-type GDF-8, the conditioned media from QM-7 cells co-transfected with Pro-GDF-8 and WT-F-GDF-8 expression plasmids were analyzed. Western blotting with anti-FLAG specific antibodies under reducing conditions detected major immunoreactive proteins migrating at 38 kDa in the conditioned media from cells transfected with Pro-GDF-8. This is consistent with the predicted size for the GDF-8 pro-domain. An additional polypeptide immunoreactive with the FLAG antibody, was also identified. This polypeptide has a molecular weight of approximately 25 kDa.

To determine the identity of the polypeptides represented by the 38 KDa and 25 kDa bands, the polypeptides were purified by affinity chromatography and the corresponding bands from the Coomassie-stained gel were subjected to N-terminal sequencing. The N-terminal sequence of the 25 kD polypeptide was DDSSD (SEQ ID NO:27), demonstrating that this 25 KDa polypeptide is the product of an alternative cleavage at a specific site (Arg 99). (The same cleavage site was also identified for both wild type and cleavage site mutant GDF-8 precursors).

Dimers of the pro-domain migrating at 80 kDa, as well as monomeric forms of the pro-domain were also present on the non-reducing gel.

Co-transfection of the Pro-GDF-8 construct with the WT-F-GDF-8 construct resulted in a significant decrease in the amount of secreted mature GDF-8.

Since two FLAG-immunoreactive proteins were detected in the conditioned media of QM-7 cells transfected with Pro-GDF-8, it was important to determine which of these polypeptides possessed the inhibitory activity over GDF-8. This was achieved by elution of the proteins from denaturing SDS-PAGE. Individual polypeptides were then tested for their ability to act as GDF-8 inhibitors in a reporter activation bioassay, as described above. The results demonstrate that only the higher molecular weight polypeptide (migrating at 38 kDa) representing the full-length pro-domain possessed inhibitory activity, whereas the polypeptide migrating at 25 kDa, a product of an alternative cleavage at Arg 99, was inactive. This indicates that the inhibitory (or GDF-8-binding) domain is located at the N-terminus of the pro-domain, upstream of Arg 99. Thus, GDF-8 inhibitors of small molecular size may be designed based on the sequence of the pro-domain upstream of Arg 99 (see *Figure 13).*

Overall, the foregoing studies demonstrate that the GDF-8 pro-domain is able to specifically inhibit the biological activity of GDF-8 *in vitro* in a transcription-based assay and that it affects the secretion of the mature GDF-8 in the conditioned media of cultured cells, when co-expressed with the wild type GDF-8 protein.

### EXAMPLE 7: Generation Of Transgenic Mice Overexpressing The GDF-8 Pro-Domain

To confirm the results described above in Example 6, *in vivo,* a DNA construct for muscle-specific expression of the GDF-8 pro-domain in mice was generated. Briefly, the partial mouse cDNA encoding the pro-domain (residues 1-266 shown in *Figure 13*) was fused with the FLAG epitope at the C-terminus, and inserted in the pMEX-NMCS2 expression vector downstream of the rat Myosin Light Chain 1 promoter. This vector was shown to facilitate fast fiber specific expression in skeletal muscle of transgenic mice (Neville, C. *et al.* (1996) *Dev. Genetics* 19: 157).

Transgenic mice were generated by standard pronuclei microinjections. Offspring were screened for transgene integration by tail PCR with specific primers to the FLAG epitope. Fourteen germ-line-integrated founders were established. The Northern blotting analysis of muscle tissues with GDF-8 specific probes identified 3 lines with high level of transgene expression in skeletal muscle. The expression of the transgene (the GDF-8 pro-domain) exceeded the levels of endogenous GDF-8 by 3-10 fold.

### EXAMPLE 8: GDF-8 Is Secreted Within A Latent Complex With Its Pro-Domain Which Is Activated By Acidification

Upon characterization of media from QM-7 cells transfected with WT GDF-8, an additional 38 kDa "double" band was detected. To determine the identity of this polypeptide the FLAG-tagged proteins were purified by affinity chromatography and the bands from the Coomassie-stained gel were subjected to N-terminal sequencing. The N-terminal sequence of the 38 kDa "double" band polypeptide was DDSSD (SEQ ID NO:27), indicating that it is the product of an alternative cleavage at a specific site (Arg 99) . The sequence analysis of this "double" band migrating at 38 kDa revealed also the polypeptide with an amino-terminal sequence GPVDLNE (SEQ ID NO:28), which is identical to that of the GDF-8 precursor protein. This band represents the pro-domain, lacking the signal peptide, which is co-purified with the FLAG-tagged GDF-8 precursor and mature fragment. This polypeptide, not recognized by an anti-FLAG M2 specific antibody, co-migrates with the independently expressed pro-domain. Since the FLAG epitope is located at the C-terminus of the WT-GDF-8 protein, the GDF-8 pro-domain could only bind to the anti-FLAG affinity gel if it is indeed associated with the mature GDF-8.

To further characterize the secreted GDF-8 complexes, cell media from QM-7 cells transfected with WT-GDF-8-F was size-fractionated using Microcon centrifugal filters (with a molecular weight cut-off of 50K). The retentate contained almost all GDF-8 related immunoreactive proteins, further supporting the presence of the high molecular weight complexes, which migrated at 60-120kDa under non-reduced conditions. Upon reduction, the mature GDF-8 fragments, migrating at 15kDa, as well as unprocessed forms of GDF-8 were detected. No mature GDF-8 dimer was found in the flow-through fraction. These data confirm that mature GDF-8 is present in the cell media within a high molecular weight complex.

To assess the biological activity of the GDF-8 complexes produced by QM-7 myoblasts, a transcription-based reporter activation assay was employed (see Figure 29). The crude cell media did not exhibit any GDF-8-like biological activity. In contrast, purified human mature GDF-8 efficiently induced luciferase activity from the reporter plasmid p(CAGA)₁₂-MLP (described above). Unlike crude cell media, the affinity purified GDF-8 complexes possessed activity. This limited activation occurred, most likely, from chemical acidification resulting from elution with glycine buffer (pH 3.5). Further purification of affinity purified complexes through an HPLC C4 column (upon acidification with TFA) resulted in the identification of a more active fraction eluting at approximately 18 minutes. This fraction (fraction #18) evoked the highest response in the transcription-based bioassay and is mainly composed of the mature GDF-8 migrating at 15 kDa on the reduced SDS-PAGE. Importantly, fraction # 18 contains far less amounts of unprocessed GDF-8, than the starting material (Figure 29, lane 1) or fraction #20 (Figure 29, lane 4).

Thus, it is possible that high molecular weight GDF-8-related proteins, which are secreted by QM-7 cells and co-purified (by affinity chromatography) with the mature GDF-8, can inhibit its biological activity. The aforementioned results suggest that the mature GDF-8 is secreted by QM-7 cells within a latent complex, containing the pro-domain. This is consistent with the ability of the purified pro-domain to inhibit the activity of GDF-8 in a transcription-based assay (described above). The underlying mechanism may be the association of the mature GDF-8 with its pro-domain, preventing its interaction with the signaling receptor.

### EXAMPLE 9: Activation Of The Latent GDF-8 Complex By Calpain

M-calpain is a ubiquitiously expressed calcium-dependent member of the calpain family of cysteine proteases. In these experiments, the ability of m-calpain to activate the GDF-8 complex was investigated. First, the ability of m-calpain to cleave GDF-8 proteins *in vitro* (*i.e.,* in the test tube) was studied. Briefly, QM-7 conditioned media containing WT-GDF-8-F or Pro-GDF-8-F proteins were treated with 0, 0.1, and 1 U/ml of calpain at 37 °C for an overnight period. The results demonstrated that m-calpain can specifically cleave the pro-domain of GDF-8, generating additional lower molecular weight species. Notably, the full-length precursor of wild type GDF-8 is also a substrate for m-calpain. Treatment with a higher concentration of calpain (1U/ml) resulted in a complete depletion of the full-length wild type GDF-8 protein. Only trace amounts of proteolytic products of this cleavage were detected. Importantly, the mature GDF-8 was not degraded by m-calpain. In the presence of QM-7 cells, m-calpain also cleaved the pro-domain, as evidenced by the appearance of lower molecular weight bands on the SDS-PAGE.

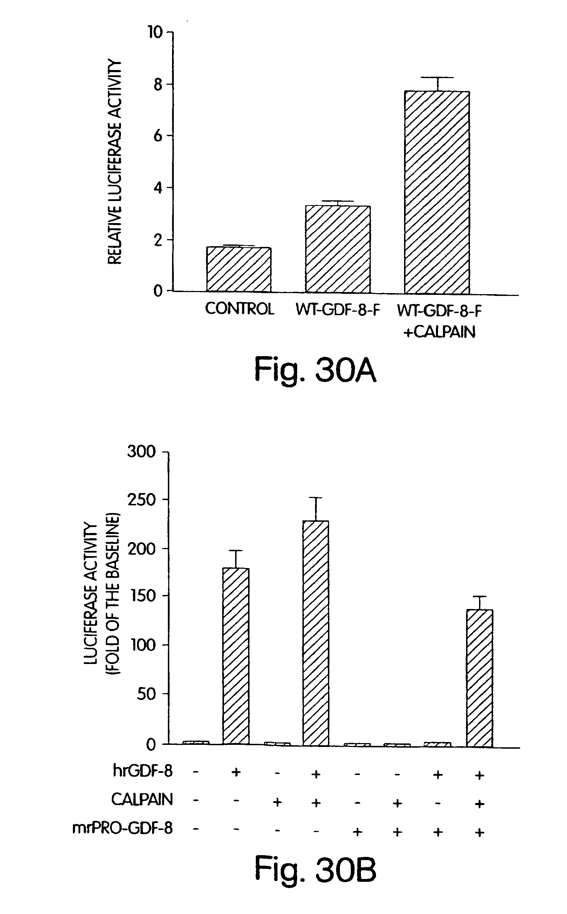

Since m-calpain was able to digest the GDF-8 pro-domain, it was hypothesized that m-calpain might activate the latent GDF-8 complex. To verify ths hypothesis, supernatants from QM-7 cells, transfected with WT-GDF-8-F construct or mock-transfected cells, were treated with 0 or 1U/ml of m-calpain for 2 hours at 37° C, followed by affinity purification of GDF-8 complexes on the anti-FLAG column (described above). The purified proteins were tested for activity in the transcription-based bioassay described herein. Luciferase activity was 2.5-fold higher after the incubation of GDF-8 complexes with m-calpain, compared to the untreated control (Figure 30A). In agreement with its inability to digest the mature factor, m-calpain did not inhibit the activity of mature human recombinant GDF-8 (hrGDF-8) (Figure 30B). However, pre-incubation of the pro-domain with m-calpain drastically reduced the pro-domain's ability to inhibit mature GDF-8 (Figure 30B). These results demonstrate that m-calpain can release active GDF-8 from the latent complex by cleaving the pro-domain.

The aforementioned data show that activation of GDF-8 and the release of the mature GDF-8 protein from the GDF-8/pro-domain complex targets for inhibiting GDF function. For example, the stability of the
pro-domain can be increased to, thereby, prevent its potential cleavage by cellular proteases. This will, in turn, lead to the stabilization of the GDF-8/pro-domain complex, thus, preventing the release of active GDF-8.

### EXAMPLE 10: Production And Characterization Of a GDF-8 Cleavage-site Mutant (Dominant-Negative Mutant)

A GDF-8 cleavage-site mutant (dominant-negative mutant) was generated and tested for GDF-8 inhibition as follows:

Briefly, a construct expressing an un-cleavable full-length GDF-8 mutant (Mut-GDF-8) was generated by replacing the predicted cleavage site at the boundaries between the pro-domain and the mature protein. After removal of the signal peptide, the predicted protein comprises the pro-domain followed by the C-terminal mature region of GDF-8 (*Figure 12*B)*.* Unlike wild-type GDF-8, the un-cleavable mutant can not be cleaved to generate the mature GDF-8 protein and is, therefore, not biologically active. The wild-type (WT) mouse GDF-8 cDNA was also subcloned in the same vector to generate the full-length precursor protein tagged with the FLAG epitope at the C-terminal. The resulting construct is referred to as WT-F-GDF-8 (*Figure 12*A)*.* The unmodified (not tagged) GDF-8 cDNA subcloned in the CMV-based expression vector is referred to as WT-GDF-8.

Both expression constructs were introduced into QM-7 quail myoblast cells by transient transfection, and GDF-8 proteins were immunoprecipitated from conditioned media with anti-FLAG affinity gel. The immunoprecipitates were further analyzed by SDS-PAGE, followed by detection with an anti-FLAG specific antibody as described in Methods. The wild-type (WT-F-GDF-8) was expressed and processed properly in QM-7 cells. Under reducing conditions two immunoreactive bands were detected, representing the full-length precursor (45-50kDa) and the mature GDF-8 (15kDa). The sizes of these proteins are consistent with ones previously reported for GDF-8. The remainder (pro-domain) is not detectable, as it lacks the FLAG epitope. No immunoreactive proteins were detected in conditioned media from cells transfected with the control empty vector. Transfection of the un-cleavable mutant MutGDF-8 resulted, as expected, in the generation of a major immunoreactive species, corresponding to precursor molecules, as well as some amounts of GDF-8 related proteins migrating at 38 kDa.

To test the ability of the cleavage site mutant to act as a dominant-negative inhibitor of the wild type GDF-8, QM-7 cells were co-transfected with different ratios of the Mut-GDF-8-F and WT-GDF-8-F constructs. The results demonstrated that the cleavage site mutant inhibits the secretion of the mature GDF-8 in a dose-dependent manner. To confirm that the cleavage site mutant also inhibits the secretion of non - tagged wild-type GDF-8, Mut-GDF-8-F and WT-GDF-8 constructs were co-introduced into QM-7 cells, and antibodies to GDF-8 were used to detect expressed proteins. The results were similar to those with the FLAG-tagged GDF-8.

The specificity of the inhibition was examined using recombinant BMP-2, a member of the TGF-β family sharing 41 % homology with GDF-8 within the mature region. The expression constructs for both BMP-2 and Mut-GDF-8-F were co-introduced into QM-7 cells, and secreted proteins were detected with an anti-BMP-2 antibody. The data demonstrated that the mutant GDF-8 did not affect the secretion and processing of mature BMP-2. Thus, inhibitory action of Mut-GDF-8 is selective with regard to other members of the TGF-β protein family.

To address the issue of stability of the dominant-negative mutant and in an attempt to reveal the mechanism of the inhibition, transfected QM-7 cells were grown in the presence of 200µCi/ml of [³⁵S] cysteine for 2 hours and chased at indicated time periods. The conditioned media were collected and cell lysates were prepared for further analysis. All FLAG-tagged proteins were immunoprecipitated with anti-FLAGM2 affinity gel and fractionated on SDS-PAGE. Data from this experiment demonstrates that the precursor form of WT-F-GDF-8 and the full-length Mut-GDF-8 proteins initially accumulate inside the cells. Three forms of GDF-8 were detected in the supernatants of the cells transfected with the WT-F-GDF-8: the precursor, the mature GDF-8 and the pro domain. No intracellular accumulation of the processed forms of GDF-8 was detected, both the C-terminal mature GDF-8 and the pro-domain were secreted immediately after cleavage and were detected in the supernatants as early as 3 hours after synthesis. Processed forms of GDF-8 were stable in the conditioned media for at least 24 hours. No additional accumulation of the processed forms of GDF-8 was observed after incubation of conditioned medium for 24 hours at 37° C. This indicates that the presence of the cells seem to be essential for processing. However, the possibility exists that some cleavage can occur outside of the cells upon availability of the specific protease. The unprocessed forms of WT-F-GDF-8 and the full-length Mut-GDF-8 were also efficiently secreted, but some intracellular accumulation was observed as well.

Upon co-transfection of the WT-F-GDF-8 together with the Mut-GDF-8, a pool of the mature C-terminal fragments, accumulating inside the cells was observed. The same phenomenon was observed when the Mut-GDF-8 was introduced into QM-7 stable cell lines constitutively expressing WT-F- GDF-8. Two of the positive clones were transiently transfected with the Mut-GDF-8 construct, labeled as above, and cell lysates were analyzed 24 hours later. As the data indicate, only in the cells co-transfected with the mutant GDF-8 there was a detectable cell-associated pool of the mature C-terminal fragment. These data confirm the hypothesized mechanism of the dominant-negative inhibition of secretion of GDF-8 by the un-cleavable mutant, i.e., formation of heterodimers (or other complexes) between the wild-type and mutant proteins which interfere with the normal secretion of this growth factor.

Pro-GDF-8 interacts and inhibits the activity of mature GDF-8 only when it is produced as such, and not when it is embedded in a larger protein, as is the case with the un-cleavable GDF-8 (Mut-GDF-8), which contains the GDF-8 pro-domain sequence. The studies described above show that pro-GDF-8 can act as an antagonist of endogenous mature GDF-8 either when the pro-GDF-8 is administered to a subject in protein form or when it is expressed from a suitable vector (i.e., gene therapy-type approach), since its target is the secreted mature, processed GDF-8. In contrast, the mode action of the Mut-GDF-8 is intracellular, during GDF-8 synthesis, resulting in the intracelluar formation of un-cleavable heterodimers. As a consequence, Mut-GDF-8 can inhibit GDF-8 function when it is expressed intracellularly.

### EXAMPLE 11: Production And Testing Of a GDF-8 Cysteine Mutant

A GDF-8 cysteine mutant was produced by introducing a point mutation into the GDF-8 cDNA using a PCR- based approach as described for the other GDF-8 mutants The mutated GDF-8 sequences were inserted in the FLAG-CMV-5a expression vector to produce an in-frame fusion with the FLAG epitope. The resulting construct (C-Mut-GDF-8) was introduced alone into QM7 cells, or cotransfected with WT-F-GDF-8 to test the effect of the mutant on the production and accumulation of the mature GDF-8.

Since immunoreactive proteins could not be detected in the conditioned media of cells transfected with C-Mut-GDF-8 by Western blotting, metabolic labeling of these cells was performed after transfection. As the experiments demonstrated, the C-Mut-GDF-8 protein was produced and properly processed in QM-7 cells, but it was secreted in much lower amounts than the wild-type GDF-8. Most of the full-length precursor form accumulated inside the cells, while both mature and unprocessed forms were secreted in the conditioned media. Thus, the mutation of one of the cysteines in the mature region of GDF-8 can still be processed and secreted. However, co-transfection experiments demonstrated that the cysteine mutant has the ability to inhibit secretion of the wild-type mature factor, when it is introduced into QM-7 cells together with the WT GDF-8.

### EXAMPLE 12: Analysis Of The Role Of N-Linked Glycosylation Of GDF-8 Variants

Glycosylation is one of the major forms of post-translational modifications of proteins. The carbohydrate structures of glycoproteins play important roles in protein processing, secretion and biological activity. The GDF-8 precursor contains one predicted site of N-linked glycosylation at Asn 72 within its pro-domain. To determine if this glycosylation site is used, the purified recombinant GDF-8 proteins were first treated with N-glycosydase F. This enzyme is able to release all common classes of N-glycans from the protein backbone. WT-F-GDF-8, Mut-GDF-8 and Pro-GDF-8 proteins were purified using anti FLAG affinity chromatography. The protein samples were denatured, incubated with N-glycosidase F in the test tube, and subjected to the SDS-PAGE in reducing conditions. As the results of this experiment demonstrated, the enzymatic removal *in vitro* of the carbohydrate structures results in the shift to a lower apparent molecular weight for precursor forms of the wild type and mutant GDF-8. The same shift was observed for the pro domain of GDF-8, expressed independently. Notably, there was no change in the mobility of the mature form of GDF-8, consistent with the absence of putative glycosylation sites within this region.

To examine the role of glycosylation in GDF-8 processing and secretion, the glycosylation inhibitor tunicamycin was used. This nucleoside antibiotic prevents the formation of the dolichol intermediate, necessary for oligosaccharide addition to the nascent polypeptide chain. The QM-7 quail myoblast cells were transiently transfected with the constructs expressing wild type GDF-8, or GDF-8 variants (the cleavage site mutant and the pro-domain). All three proteins were fused to a C-terminal FLAG epitope. Cells were treated with 2 µg/ml of tunicamycin 16 hours after transfection. Conditioned media and cell lysates were analyzed 24 hours later by Western blotting or immunoprecipitation, followed by detection of the proteins with anti-FLAG antibody. The results from this experiment demonstrate that tunicamycin appears to block the secretory exit of all three forms of GDF-8. An increase in the cell-associated non-glycosylated precursor form of GDF-8 and its pro-domain which migrate faster than the glycosylated species was observed. The data suggest that GDF-8 is a glycoprotein and its glycosylation is essential for normal processing and secretion. Treatment of Fraction B (containing the pro-domain) with N-glycosidase F eliminated the inhibitory activity of the pro-domain (Figure 36), thus providing further support that N-linked glycosylation is important for the inhibitory activity of the pro-domain.

### EXAMPLE 13: Effect Of The Human GDF-8 Pro-Domain From Fraction B And The Mouse GDF-8 Pro-Domain On Chicken Growth And Muscle Development

Since GDF-8 "knock-out" mice generated by gene targeting develop normally, but have twice the normal skeletal muscle mass, it was hypothesized that introduction of the GDF-8 pro-domain during development may result in an increase in skeletal muscle mass in treated animals. To test this hypothesis and the efficacy of *in ovo* administration of the GDF-8 pro-domain to enhance skeletal muscle development, purified GDF-8 pro-domain (human from Fraction B, described above, or mouse from Example 6) was injected into fertilized eggs from Cobb and Ross chickens. Fertilized eggs were injected on Day 0, 1, 2, 3, 11, 12, 13, 14, 15, 18, and 20 as described herein using the techniques described in, for example, H. Kocamis *et al.* (1998) *Poult. Sci.* 77, 1913-1919. Live, carcass, breast, and legs weights were obtained at the end of the 42-day grow-out period. Table II shows the percentage increase in live, breast, and leg weights for treated male and female birds (Cobb eggs, injection days Day 13 and Day 20) over control birds (uninjected).

**Table II**

| Cobb | Live Weight | Breast | Leg |
|---|---|---|---|
| Male (Day 13) | 11.8 | 18.0 | 18.6 |
| Female (Day 13) | 7.7 | 9.7 | 13.8 |
| | | | |
| Male (Day 20) | 3.4 | 13.8 | 7.4 |
| Female (Day 20) | 10.4 | 17.4 | 13.8 |

Table III shows the percentage increase in live, carcass, breast, and leg weights for treated male and female birds (Ross eggs, injection day Day 15) over control birds (buffer alone).

**Table III**

| Ross | Live Weight | Carcass | Breast | Leg |
|---|---|---|---|---|
| Male (Day 15) | 4.2 | 5.5 | 3.3 | 6.7 |
| Female (Day 15) | 15.8 | 18.0 | 20.8 | 9.2 |

These results demonstrate that *in ovo* administration of the GDF-8 pro-domain results in an increase in skeletal muscle mass for treated birds.

### EXAMPLE 14: Identification Of GDF-8 Receptors That Can Be Used As GDF-8 Inhibitors

### Binding of [¹²⁵-I]-GDF-8 to receptors expressed in COS-7 cells

To identify known (cloned) type II receptors that can bind GDF-8, expression constructs encoding three different serine-threonine receptors, or an empty vector (control) were introduced by transient transfection into COS-7 cells (50% confluent) using FuGENE 6 (Boehringer Mannheim). The type II receptors used were the TGF-β type II receptor, the Activin type IIB receptor (the B2 splice variant isoform), and the BMP type II receptor. Binding assays were performed 48 hours later, using [¹²⁵-I]-labeled GDF-8 as the ligand, followed by two types of analysis. First, an analysis was performed which involves cell lysis and quantitative determination of GDF-8 binding to the surface of the cells transfected by the various constructs, using scintillation counting. The second type of analysis was confirmatory, involving the visualization of GDF-8 binding to the surface of COS-1 cells and was performed as described in, for example, Lin *et al.*(1992) *Cell* 68, 775-85.

### Functional assays based on GDF-8-induced transcription from reporter constructs

The transcription-based bioassay was performed in two different cell lines, the A204 human rhabdomyosarcoma cell line and the mink lung epithelial cell line CCL-64. Two artificial reporter plasmids were used in this assay: p3TP-Lux and p(CAGA)₁₂₋MLP (described above). In both, luciferase gene transcription (and thus activity) is driven by artificial minimal promoters that respond to members of TGF-β family members. Therefore, luciferase activity in cell lysates correlates linearly with the degree of stimulation of the cells by the applied growth factors.

Both cell types were plated in 48-well plates in their respective media (DMEM for CCL-64, McCoy's medium for A204) supplemented with 10% fetal bovine serum, antibiotics and L-Glutamine. Upon reaching 80% confluence, cells were transfected using FuGENE-6 to facilitate plasmid uptake (Boehringer-Mannheim), according to the manufacturer's instructions. Cells were transiently transfected with a cocktail of the following plasmids; the receptor expression plasmids pCMV-ΔNβRII K-R (encoding the mutated, dominant-negative type II TGF-β receptor), pCMV-ΔNβRIIB2 K-R (encoding the mutated, dominant-negative type II Activin receptor) and the reporter plasmids pSV-β-gal (used to monitor transfection efficiency) and either of the two luciferase reporter plasmids. After overnight incubation with the transfection reagents, cells were washed twice with the appropriate serum-free medium containing 0.1 % BSA. Human recombinant GDF-8 was then added to the cells. Cells were lysed after a 6 hour incubation and Luciferase and β-galactosidase activity were determined in the same sample using the Dual-Light Luciferase Assay kit (Tropix Inc.) Activity is expressed in Relative Luciferase units (RLU), i.e., luciferase activity corrected for transfection efficiency that is given by the corresponding values of β-galactosidase activity measured in the same sample.

### Functional identification of the TGF-β receptor Alk-5 as the type I receptor for GDF-8.

The mink lung epithelial cell line CCL-64 is highly responsive to TGF-β. The cell line R1B, derived from CCL-64 by chemical mutagenesis, is unresponsive to TGF-β. It has been established that its unresponsiveness is due to lack of functional TβRI receptors. As a definitive proof, upon re-introduction of TβRI in this cell line, by transfection with a plasmid encoding this receptor, TGF-β responses can be fully restored (Wrana et al., Cell, (1992), 71, 1003-14).

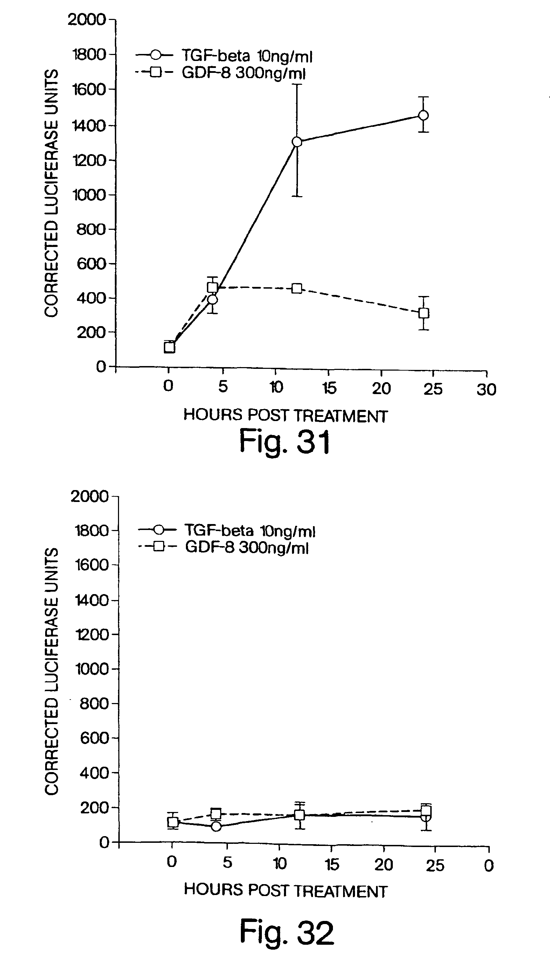

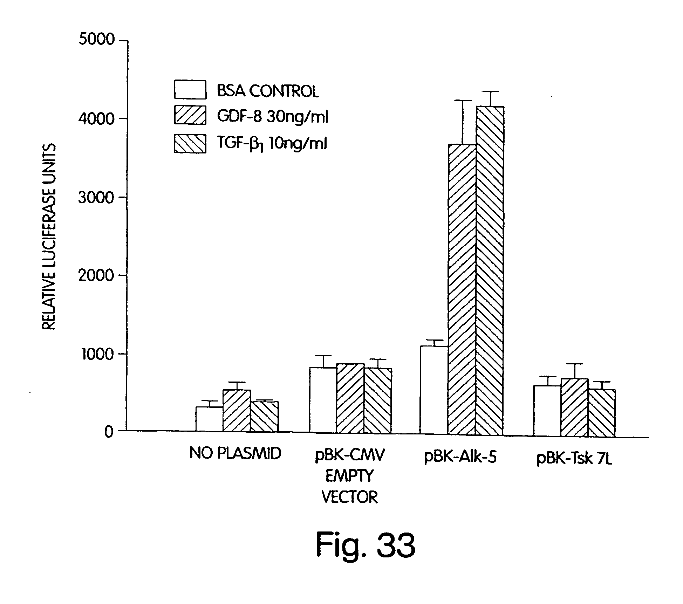

Similarly to TGF-β, GDF-8 also elicits strong responses in the wild-type cell line CCL-64 (Figure 31), but is inactive in the R1B mutant (Figure 31). However, GDF-8 responsiveness can be fully recovered when R1B cells are transfected with a plasmid encoding TβRI, thus resulting in full functional rescue (Figure 33). The foregoing data indicate that TβRI is a functional GDF-8 type I receptor.

### Identification of ActRIIB2 as a type II receptor for GDF-8.

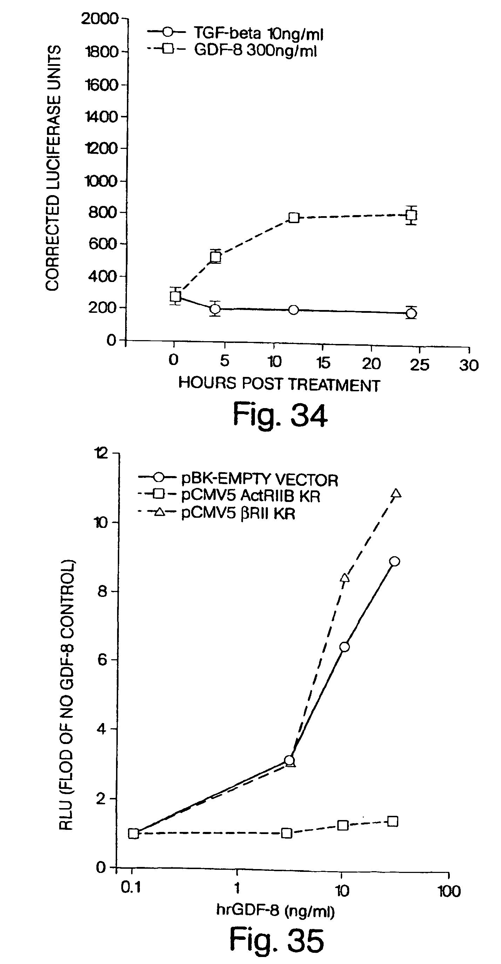

Another CCL-64-derived mutant cell line, DR26, lacks the TGF-β type II receptor and is therefore unresponsive to TGF-β. In contrast to the R1B cell line, DR26 cells fully retain their responsiveness to GDF-8, indicating that the TGF-β type II receptor is dispensable for GDF-8 activity, and some other type II receptor subunit must mediate GDF-8 responses (Figure 34).

To find out whether any of the already known (cloned) type II serine-threonine kinase receptor subunits can serve as a GDF-8 receptor, a variety of expression constructs encoding type II receptors were transfected into COS-7 cells. Cells were subsequently tested for [¹²⁵-I]-GDF-8 binding. In agreement with the functional analysis described in the preceding paragraph, no specific GDF-8 binding was detected to either TGF-β or BMP type II receptors. In contrast, the Activin type II receptor ActRIIB2 was identified as a GDF-8-binding receptor. This was confirmed by the increased binding of [¹²⁵-I]-GDF-8 to the surface of COS-7 cells transfected with ActRIIB, while cells transfected with a control vector displayed no specific binding.

The functional significance of the binding data was further demonstrated by using dominant-negative (ΔN) forms of ActRIIB2. Dominant-negative receptor isoforms were derived by point mutation of a specific amino acid residue in each receptor construct (K to R mutation) in the intracellular domain region of the receptors. These mutations do not interfere with the ability of the type II subunits to be properly expressed in the cell membrane, to bind ligand or to form heteromers with the respective type I subunits. However, these mutations eliminate the receptors' kinase activity and thus prevent their ability to relay functional signals after ligand binding (Wrana et al.(1992) Cell 71, 1003-14).

These dominant-negative receptor isoforms were introduced in CCL-64 mink lung epithelial cells or in A204 human rhabdomyosarcoma cells that are highly responsive to GDF-8. As expected from the binding experiment data and the functional data obtained in R1B cells, the ΔN form of the TGF-β type II receptor did not affect GDF-8 responses, thus, confirming that it is dispensable in this respect. In contrast, introduction of the ΔN form of ActRIIB2 in both cell backgrounds resulted in an inhibition of GDF-8 responses in DR26 cells (Figure 35) and A204 cells transfected with the reporter construct, presumably by out-competing endogenous ActRIIB2 receptors (i.e. by acting in a dominant-negative fashion).

The combination of the binding and functional data demonstrates that ActRIIB2 can serve as a functional GDF-8 receptor. In all, the above studies demonstrate that the heteromeric complex of TβRI and ActRIIB2 is a functional receptor combination that mediates GDF-8 responses. Of note, this particular combination of type 1-type II receptor units has not been described as a functional transducer of responses of any other member of the TGF-β growth factor family. It is likely that the TβRI-ActRIIB2 heteromer can be activated only by GDF-8 and by molecules highly homologous to GDF-8. The active (mature) region of GDF-11 bears more than 90% homology to that of GDF-8, suggesting that these two closely-related factors share similar binding and signaling properties. Therefore, the reagents described herein as well as their applications can be used for both GDF-8 and GDF-11 biological research and diagnostics.

### Methods for using GDF-8 receptors

The identification of GDF-8 receptors allows the construction of "receptor probes". The utility of such "receptor probes" is directly related to their ability to recognize and bind GDF-8, GDF-8-like molecules (e.g. GDF-11) and presumably other, structurally related or unrelated molecules or peptides, whether these are chemically synthesized or naturally occurring. These, in virtue of their interaction with a GDF-8 "receptor probe", are potential GDF-8 inhibitors.

Overall, the " receptor probes" can be utilized in a number of ways: (1) in the form of a soluble receptor as GDF-8 protein traps in vivo by directly binding and neutralizing GDF-8, (2) as modified ELISA systems for GDF-8 detection and quantitation in biological and other fluids, for use to diagnose muscle wasting in humans or (3) as a screening reagent to identify GDF-8 inhibitors/antagonists at the receptor level.

The generation of GDF-8 "receptor probes" may involve the generation of GDF-8 receptor fusion proteins (using techniques described in, for example, George *et al.* (1999) *Proc. Natl. Acad Sci. USA* 96: 12719-12724. In brief, the receptor fusion proteins are a heteromeric complex of two fusion proteins: a GDF-8 type I receptor extracellular domain fused to, e.g., an immunoglobulinG, (IgG₁) Fc fragment, and a GDF-8 type II receptor extracellular domain fused to, e.g., an immunoglobulinG₁ (IgG₁) Fc fragment (or any other fusion proteins that contain the extracellular domain of GDF-8 type I receptor fused to a protein domain X and a GDF-8 type II receptor extracelluar domain fused to a protein domain Y, whereby protein domain X and protein domain Y can interact to form heteromeric complex of the two fusion proteins). The construction of fusion receptors is not limited to using the Fc fragment; proteins or portions of proteins, both mammalian and bacterial that have the property of forming dimers or heterodimers can be utilized in this approach (e.g. basic helix-loop-helix domains). Methods for preparing such fusion receptor proteins are described in, for example, Davis *et al.* (1994) *Science* 266: 816-819 or US Patent No.5,844,099, the contents of which are incorporated herein by reference.

The following steps are necessary for developing the heterodimeric complex or "receptor probe".

### Creation of receptor/Fc hybrids

DNA encoding receptor/Fc fusion proteins is generated through multiple rounds of PCR. Based on similar experiments conducted with receptors for other TGF-β family members, DNA encoding the amino-terminal 160 amino acids of the receptor is amplified from a cDNA clone. The 3' PCR primer overlaps with the 5' sequence of the human IgG 1 sequence that is to be included in the fusion gene. Likewise, DNA encoding the Fc region of the human IgGI gene (Pro 100 - Lys 330) is amplified from human genomic DNA using a 5' primer that overlaps with the receptor sequence. The receptor and IgG 1 PCR products are then combined and used as overlapping templates to generate an amplification product that represents a fusion of the sequences encoding the receptor ligand binding domain and the IgG 1 Fc region. The final PCR product is ligated into the TA cloning vector (Invitrogen, San Diego, CA) and then subcloned into pCMV5 (Sigma) to produce a construct that can express the fusion protein in transfected eukaryotic cells. The constructs are tested in CHO cells and in NSO mouse myeloma cells.

Receptor/Fc hybrid proteins are purified from the conditioned media of transfected cells using a Protein A Sepharose column (Pharmacia, Piscataway, NJ). The fusion proteins are purified in the form of homodimers. Heterodimers are created by reducing the homodimers under conditions that favor the disruption of inter-chain disulfide bonds without affecting intra-chain disulfides. The two different types of monomers are mixed in equimolar amounts and oxidized to form a mixture of homo- and heterodimers. The homodimers can be separated then from the heterodimers by HPLC. Alternatively, one of the fusion proteins may be tagged with a string of carboxy-terminal histidine residues which allows purification of the recombinant proteins by nickel-chelate chromatography. The three types of dimeric proteins are then separated by elution of the bound proteins using increasing amounts of imidazole. Homodimers lacking the histidine tag are eluted at the lowest concentrations of imidazole; heterodimers with only one tagged protein are eluted at intermediate concentrations; and homodimers in which both subunits have histidine tags are eluted only at the highest concentrations of imidazole.

The receptor/Fc heterodimer preparations are analyzed by polyacrylamide gel electorphoresis and silver staining for the presence of contaminating proteins. If necessary, additional purification steps are performed. The purified heterodimers are also tested to ensure that they can bind GDF-8, as described below, and they are tested as antagonists in one of the bioassays described herein.

### Development of a "ligand trap" assay for quantitation of GDF-8 protein

The GDF-8 quantitation system is a modified sandwich ELISA that incorporates competitive and capture techniques and that uses heterodimers of receptor/Fc hybrid proteins as capture reagents in place of a capture antibody. The following steps comprise a general protocol that may be used with the GDF-8 "ligand trap" assay. ELISA plates are coated with receptor/Fc protein heterodimers and the unattached receptor/Fc proteins are washed off. The proteins are fixed and the plates are washed again. The protein samples are combined with GDF-8 standards at different dilutions, the anti-GDF-8 antibody is added, and the plate is incubated at 37°C. The protein/antibody mixtures are added to coated plates, the unattached ligands are washed off under stringent conditions, a secondary antibody coupled to horse radish peroxidase (HRP) is added, and the unbound secondary antibody is washed off. Subsequently, OPD substrate is added and the optical densities are measured using a microplate reader. Finally, the level of GDF-8 in a sample is determined using the absorbance value approach.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A method for identifying an inhibitor of a GDF protein, comprising:
(a) obtaining medium in which cells producing a GDF protein have been cultured;
(b) testing the medium for the ability to inhibit GDF activity, thereby identifying a GDF inhibitor.

2. The method in claim 1, further comprising performing chromatography and/or gel electrophoresis on said medium before said medium is tested for the ability to inhibit GDF activity.

3. A method for identifying an inhibitor of a GDF protein, comprising:
(a) preparing fragments of a wild type GDF protein;
(b) testing the fragments in vitro for the ability to inhibit GDF activity, thereby identifying a GDF inhibitor derived from the wild type GDF protein.

4. The method in claim 1 to 3, wherein said GDF protein is selected from human GDF-8 or GDF-11, bovine GDF-8 or GDF-11, chicken GDF-8 or GDF-11, murine GDF-8 or GDF-11, rat GDF-8 or GDF-11, porcine GDF-8 or GDF-11, ovine GDF-8 or GDF-11, turkey GDF-8 or GDF-11 and baboon GDF-8 or GDF-11.

5. The method in claim 1 to 4, wherein the GDF inhibitor comprises a GDF prodomain or a portion of the prodomain.

6. The method of claim 1 to 5, wherein the testing comprises a transcription-based assay for detecting the activity of creatine kinase.

7. An isolated polypeptide which inhibits GDF activity consisting of the prodomain of a GDF protein or a portion of the prodomain.

8. The polypeptide of claim 7:
(a) wherein the polypeptide does not inhibit TGF-β or activin activity; and/or
(b) wherein the polypeptide is synthetically produced; and/or
(c) wherein the polypeptide is derived from a native GDF polypeptide; and/or
(d) wherein the polypeptide is produced recombinantly; and/or
(e) wherein the polypeptide is glycosylated.

9. An isolated polypeptide as claimed in claim 7 consisting of the N-terminus of the GDF-8 pro-domain upstream of the arginine residue at position 99.

10. A variant of a GDF-8 polypeptide which inhibits GDF-8 activity, wherein the variant is a post-translational modification variant in which the asparagine residue at position 72 of the prodomain is mutated to prevent glycosylation of the pro-domain.

11. An isolated polypeptide as claimed in any one of claims 7 to 10 for use in therapy, wherein the therapy comprises inhibiting an activity of GDF in a cell.

12. An isolated GDF-8 polypeptide which inhibits an activity of GDF-8 in a cell consisting of an amino acid sequence selected from SEQ ID NO. 25, SEQ ID NO. 29 and SEQ ID NO. 30.

13. A non-human transgenic animal which expresses the polypeptide of any one of claims 7 to 10.

## Patentansprüche

1. Verfahren zur Identifizierung eines Inhibitors eines GDF-Proteins, bei dem man:
(a) Medium erhält, in dem Zellen, die ein GDF-Protein produzieren, kultiviert worden sind;
(b) das Mediums auf die Fähigkeit hin untersucht, GDF-Aktivität zu inhibieren, um **dadurch** einen GDF-Inhibitor zu identifizieren.

2. Verfahren nach Anspruch 1, wobei man ferner Chromatographie und/oder Gelelektrophorese an diesem Medium durchführt, bevor man dieses Medium auf die Fähigkeit hin untersucht, GDF-Aktivität zu inhibieren.

3. Verfahren zur Identifizierung eines Inhibitors eines GDF-Proteins, bei dem man:
(a) Fragmente eines Wildtyp-GDF-Proteins herstellt;
(b) die Fragmente in vitro auf die Fähigkeit hin untersucht, GDF-Aktivität zu inhibieren, um **dadurch** einen GDF-Inhibitor zu identifizierern, der sich von dem Wildtyp-GDF-Protein ableitet.

4. Verfahren nach Anspruch 1 bis 3, wobei man dieses GDF-Protein auswählt aus humanem GDF-8 oder GDF-11, bovinem GDF-8 oder GDF-11, GDF-8 oder GDF-11 vom Huhn, murinem GDF-8 oder GDF-11, GDF-8 oder GDF-11 von der Ratte, porzinem GDF-8 oder GDF-11, ovinem GDF-8 oder GDF-11, GDF-8 oder GDF-11 vom Truthahn und GDF-8 oder GDF-11 vom Pavian.

5. Verfahren nach Anspruch 1 bis 4, wobei der GDF-Inhibitor eine GDF-Prodomäne oder einen Teil der Prodomäne umfasst.

6. Verfahren nach Anspruch 1 bis 5, wobei das Untersuchen einen auf Transkription basierenden Assay zur Detektion der Aktivität von Creatinkinase umfasst.

7. Isoliertes Polypeptid, das GDF-Aktivität inhibiert, bestehend aus der Prodomäne eines GDF-Proteins oder einem Teil der Prodomäne.

8. Das Polypeptid von Anspruch 7:
(a) wobei das Polypeptid nicht TGF-β- oder Activin-Aktivität inhibiert; und/oder
(b) wobei das Polypeptid synthetisch hergestellt worden ist; und/oder
(c) wobei sich das Polypeptid von einem nativen GDF-Polypeptid ableitet; und/oder
(d) wobei das Polypeptid rekombinant hergestellt worden ist; und/oder
(e) wobei das Polypeptid glykosyliert ist.

9. Isoliertes Polypeptid nach Anspruch 7, bestehend aus dem N-Terminus der GDF-8-Prodomäne stromaufwärts des Argininrestes an Position 99.

10. Variante eines GDF-8-Polypeptids, das GDF-8-Aktivität inhibiert, wobei die Variante eine post-translationale Modifikationsvariante ist, in der der Asparaginrest an Position 72 der Prodomäne mutiert worden ist, um die Clykosylierung der Prodomäne zu verhindern.

11. Isoliertes Polypeptid nach einem der Ansprüche 7 bis 10 für den Einsatz in der Therapie, wobei die Therapie die Inhibierung einer Aktivität von GDF in einer Zelle umfasst.

12. Isoliertes GDF-8-Polypeptid, das eine Aktivität von GDF-8 in einer Zelle inhibiert, bestehend aus einer Aminosäuresequenz, die ausgewählt wird aus SEQ ID NO. 25, SEQ ID NO. 29 und SEQ ID NO. 30.

13. Nicht-humanes transgenes Tier, das das Polypeptid nach einem der Ansprüche 7 bis 10 exprimiert.

## Revendications

1. Procédé d'identification d'un inhibiteur d'une protéine GDF, comprenant :
(a) l'obtention d'un milieu dans lequel des cellules produisant une protéine GDF ont été mises en culture ;
(b) le test du milieu pour la capacité à inhiber l'activité de GDF, identifiant ainsi un inhibiteur de GDF.

2. Procédé selon la revendication 1, comprenant en outre la réalisation d'une chromatographie et/ou d'une électrophorèse sur gel sur ledit milieu avant que ledit milieu ne soit testé pour la capacité à inhiber l'activité de GDF.

3. Procédé d'identification d'un inhibiteur d'une protéine GDF, comprenant :
(a) la préparation de fragments d'une protéine GDF de type sauvage ;
(b) le test des fragments in vitro pour la capacité à inhiber l'activité de GDF, identifiant ainsi un inhibiteur de GDF dérivé de la protéine GDF de type sauvage.

4. Procédé selon l'une quelconque des revendications 1 à 3, dans lequel ladite protéine GDF est choisie parmi GDF-8 ou GDF-11 humaine, GDF-8 ou GDF-11 bovine, GDF-8 ou GDF-11 de poulet, GDF-8 ou GDF-11 murine, GDF-8 ou GDF-11 de rat, GDF-8 ou GDF-1 porcine, GDF-8 ou GDF-11 ovine, GDF-8 ou GDF-11 de dinde et GDF-8 ou GDF-11 de babouin.

5. Procédé selon l'une quelconque des revendications 1 à 4, dans lequel l'inhibiteur de GDF comprend un pro-domaine de GDF ou une portion du pro-domaine.

6. Procédé selon l'une quelconque des revendications 1 à 5, dans lequel le test comprend un dosage basé sur la transcription pour détecter l'activité de créatine kinase.

7. Polypeptide isolé qui inhibe l'activité de GDF constitué du pro-domaine d'une protéine GDF ou d'une portion du pro-domaine.

8. Polypeptide selon la revendication 7 :
(a) dans lequel le polypeptide n'inhibe pas l'activité de TGF-β ou de l'activine ; et/ou
(b) dans lequel le polypeptide est produit de manière synthétique ; et/ou
(c) dans lequel le polypeptide est dérivé d'un polypeptide GDF natif ; et/ou
(d) dans lequel le polypeptide est produit de manière recombinante ; et/ou
(e) dans lequel le polypeptide est glycosylé.

9. Polypeptide isolé selon la revendication 7 constitué de N-terminus du pro-domaine de GDF-8 en amont du résidu d'arginine à la position 99.

10. Variante d'un polypeptide GDF-8 qui inhibe l'activité de GDF-8, dans laquelle la variante est une variante de modification post-traductionnelle dans laquelle le résidu d'asparagine à la position 72 du pro-domaine subit une mutation pour empêcher la glycosylation du pro-domaine.

11. Polypeptide isolé selon l'une quelconque des revendications 7 à 10 pour une utilisation en thérapie, dans laquelle la thérapie comprend l'inhibition d'une activité de GDF dans une cellule.

12. Polypeptide GDF-8 isolé qui inhibe une activité de GDF-8 dans une cellule constituée d'une séquence d'acides aminés choisies parmi SEQ ID NO. 25, SEQ ID NO. 29 et SEQ ID NO. 30.

13. Animal transgénique non-humain qui exprime le polypeptide selon l'une quelconque des revendications 7 à 10.
